(19) Europäisches Patentamt
European Patent Office
Office européen des brevets

(11) EP 4 796 562 A1

(12) **EUROPEAN PATENT APPLICATION**
published in accordance with Art. 153(4) EPC

(43) Date of publication:
**26.08.2026 Bulletin 2026/35**

(21) Application number: **24878983.6**

(22) Date of filing: **15.10.2024**

(51) International Patent Classification (IPC):
***C07K 16/28*** *(2006.01)* ***C07K 19/00*** *(2006.01)*
***C12N 15/13*** *(2006.01)* ***C12N 15/62*** *(2006.01)*
***C12N 5/10*** *(2006.01)* ***C12N 15/85*** *(2006.01)*
***A61K 39/00*** *(2006.01)* ***A61P 35/00*** *(2006.01)*

(52) Cooperative Patent Classification (CPC):
**A61K 39/00; A61P 35/00; C07K 16/00; C07K 16/28; C07K 19/00; C12N 5/10; C12N 15/62; C12N 15/85**

(86) International application number:
**PCT/CN2024/124869**

(87) International publication number:
**WO 2025/082340 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.10.2023 CN 202311344635**

(71) Applicant: **Shanghai Simnova Biotechnology Co., Ltd.**
**Shanghai 201321 (CN)**

(72) Inventors:
- **WANG, Chao**
  **Shanghai 201318 (CN)**
- **GAO, Fanxiang**
  **Shanghai 201318 (CN)**
- **JIANG, Fuwei**
  **Shanghai 201318 (CN)**
- **YANG, Yu**
  **Shanghai 201318 (CN)**
- **YANG, Cuiqing**
  **Shanghai 201318 (CN)**
- **CAO, Zhuoxiao**
  **Shanghai 201318 (CN)**

(74) Representative: **Richly & Ritschel Patentanwälte PartG mbB**
**Sattlerweg 20**
**51429 Bergisch Gladbach (DE)**

Remarks:
The complete document including Reference Table(s) and the Sequence Listing(s) can be downloaded from the EPO website

(54) **CAR TARGETING DELTA-LIKE LIGAND 3 AND USE**

(57) The present disclosure relates to a delta-like ligand 3-targeting CAR-T cell and use, and specifically provides a nucleic acid encoding a chimeric antigen receptor targeting delta-like ligand 3, a corresponding chimeric antigen receptor, a vector, an immune effector cell, a preparation method and a product thereof, a pharmaceutical composition, pharmaceutical use, and a method for treating a tumor or cancer.

EP 4 796 562 A1

## Description

CROSS-REFERENCE TO RELATED APPLICATION

The present disclosure claims priority to the Chinese Patent Application No. 202311344635.9 filed on October 16, 2023, which is incorporated herein by reference in its entirety for all purposes.

## TECHNICAL FIELD

The present disclosure relates to the field of cell therapy, in particular to a T lymphocyte targeting DLL3 (delta-like ligand 3), a preparation method therefor, and use thereof.

## BACKGROUND

Delta-like ligand 3 (DLL3) is a member of the Notch ligand family. There are two types of spliceosomes. For the major spliceosome, the sequence homology of human and monkey extracellular segments is 91%, and the sequence homology of human and murine extracellular segments is 84%. DLL3 is distributed mainly in the Golgi apparatus and is partially localized at the cell membrane. Under physiological state, it mainly participates in the process of forming the somite in the embryonic development. DLL3-knockout mice show craniocerebral or neuronal developmental defects, and DLL3-mutant people show defects such as vertebral and rib hypoplasia. Unlike other ligands of the Notch family, which activate signaling pathways, DLL3 can interact with DLL1 or Notch1 in cis or trans manner to inhibit the activation of signaling pathways.

The morbidity and mortality of cancer are high each year worldwide, in which lung cancer ranks first. Lung cancer is mainly divided into two categories: non-small cell lung cancer (NSCLC) and small cell lung cancer (SCLC). DLL3 protein is a highly tumor-selective cell surface single-pass transmembrane protein, an inhibitory Notch pathway ligand. DLL3 is expressed in approximately 65% pulmonary large cell neuroendocrine carcinoma, 72% first-diagnosed SCLC, and 85% recurrent SCLC. DLL3 is not expressed in most normal tissue cells, and only a few normal cells (neuronal, islet and pituitary cells) have low abundance of DLL3 expression, which are expressed only in the cytoplasm. Small cell lung cancer has the characteristics of rapid growth, early distant metastasis, and high malignancy. Small cell lung cancer has very poor prognosis, and many previous chemotherapy schemes have not been successful. In small cell lung cancer, DLL3 shows a moderate-to-high expression in more than 80% patients, and is highly expressed in the cell membrane and cytoplasm of tumors. Clinical studies have shown that high expression of DLL3 in SCLC is negatively correlated with patient survival time, i.e., the higher the expression level of DLL3, the lower the patient survival time.

Neuroendocrine lung cancer accounts for 20% of the total lung cancer cases, with small cell lung cancer accounting for about 14%. Most patients have concurrent hematogenous metastasis, and only about 1/3 limited-stage patients have foci limited to the chest. The current standard first-line therapy for patients with extensive-stage small cell lung cancer includes radiotherapy, combined use of carboplatin + etoposide + atezolizumab, combined use of carboplatin + etoposide + durvalumab, and combined use of cisplatin + etoposide + durvalumab. Untreated patients are very sensitive to chemotherapy and radiotherapy, but most patients relapse within a short period of time due to a small number of cancer cells that remain and the insensitivity to tumor stem cell therapy. For the patients receiving the first-line therapy, the median progression-free survival is 2-3 months, the median overall survival is 8-13 months, while the 5-year survival is only less than 5%. For relapsed or refractory patients, there is currently a lack of effective treatment options. The recommended second-line therapy for patients having a relapse within 6 months includes topotecan and lurbinectedin, and other recommended therapy includes the antibody pembrolizumab and the like. For patients having a relapse after more than 6 months, it is recommended to maintain the original therapy. In view of the current state of treatment, there is a need to provide new effective therapies for patients.

The progress of cell immunotherapy has provided a promising approach to the treatment of a variety of tumors, one of which is to perform genetic engineering editing on immune cells, particularly T cells, to express chimeric antigen receptors (CARs) on the cell surface. A chimeric antigen receptor is a type of protein that typically involves grafting the specificity of a monoclonal antibody onto the effector functions of a T cell. The CAR is expressed in T cells, and the CAR-modified T cells (CAR-T cells) obtain some properties such as antigen-specific recognition, anti-tumor response, and proliferation, so that it can be used as a "live drug" to eradicate and target tumor cells. In principle, any antigen (e.g., cell surface molecule) can be targeted by these CAR-T cells.

## SUMMARY

The present application constructs a novel DLL3-targeting chimeric antigen receptor (CAR), the antigen-binding domain thereof being derived from an anti-DLL3 antibody with high specificity. The CAR can improve targeting and

treatment effects, and is of important significance in the field of treating DLL3-expressing cancers.

In a first aspect of the present application, provided is a chimeric antigen receptor (CAR) comprising an extracellular binding region that specifically recognizes a delta-like ligand 3 (DLL3) protein, a hinge region, a transmembrane region, an intracellular co-stimulatory domain, and an intracellular signaling domain. The extracellular binding region comprises an antibody or an antigen-binding fragment thereof that specifically recognizes delta-like ligand 3 (DLL3). The antibody or the antigen-binding fragment thereof comprises HCDR1, HCDR2, and HCDR3 contained in the heavy chain variable region (VH) set forth in SEQ ID NO: 1, 3, 5, 83-84, 87, or 90, and LCDR1, LCDR2, and LCDR3 contained in the light chain variable region (VL) set forth in SEQ ID NO: 2, 4, 6, 81-82, 85-86, or 89.

In a second aspect of the present application, provided is an isolated nucleic acid molecule encoding the chimeric antigen receptor according to the first aspect.

In a third aspect of the present application, provided is an expression vector comprising the isolated nucleic acid molecule according to the second aspect.

In a fourth aspect of the present application, provided is a cell comprising the chimeric antigen receptor according to the first aspect, or comprising the nucleic acid molecule according to the second aspect, or comprising the expression vector according to the third aspect.

In a fifth aspect of the present application, provided is a method for preparing a chimeric antigen receptor-modified immune cell, wherein the method comprises the step of delivering the nucleic acid molecule according to the second aspect or the expression vector according to the third aspect to an immune cell to be modified.

In a sixth aspect of the present application, provided is a pharmaceutical composition, wherein the pharmaceutical composition comprises the chimeric antigen receptor according to the first aspect, the nucleic acid molecule according to the second aspect, the expression vector according to the third aspect, the cell according to the fourth aspect, or a product prepared by the method according to the fifth aspect, and a pharmaceutically acceptable carrier.

In a seventh aspect of the present application, provided is the pharmaceutical composition according to the sixth aspect for use in preventing and/or treating a tumor or cancer.

In an eighth aspect of the present application, provided is use of the chimeric antigen receptor according to the first aspect, the nucleic acid molecule according to the second aspect, the expression vector according to the third aspect, the cell according to the fourth aspect, a product prepared by the method according to the fifth aspect, or the pharmaceutical composition according to the sixth aspect in preparing a composition for preventing and/or treating a tumor or cancer.

In a ninth aspect of the present application, provided is a method for preventing and/or treating a tumor or cancer, comprising administering to a patient in need thereof an effective amount of the chimeric antigen receptor according to the first aspect, the nucleic acid molecule according to the second aspect, the expression vector according to the third aspect, the cell according to the fourth aspect, a product prepared by the method according to the fifth aspect, or the pharmaceutical composition according to the sixth aspect.

## BRIEF DESCRIPTION OF THE DRAWINGS

FIG. 1 shows a schematic diagram of the plasmid structure of the CAR-T.

FIG. 2 shows the *in vitro* expansion results of DLL3 CAR-T.

FIG. 3 shows the CAR transfection efficiency of DLL3 CAR-T cells.

FIGs. 4A and 4B show the phenotypic measurements of DLL3 CAR-T cells; FIG. 4A shows the expression level of the T cell memory phenotype after 8 days of culture. FIG. 4B shows the expression level of the T cell depletion phenotypes TIM3 and PD-1 after 8 days of DLL3 CAR-T cell culture.

FIGs. 5A to 5C show the assessment results of the *in vitro* tumor killing function of DLL3 CAR-T cells, wherein FIG. 5A shows the killing effect on SHP-77 cells for 72 h; the effector-to-target ratios (E:T) are 1:2, 1:10, and 1:20, respectively; FIG. 5B shows the killing effect of the DLL3 CAR-T cells on NCI-H2171 cells for 72 h; the effector-to-target ratios (E:T) are 1:2, 1:10, and 1:20, respectively;

FIG. 5C shows the killing effect of the DLL3 CAR-T cells on 293T-Luc cells for 72 h; the effector-to-target ratios (E:T) are 1:2, 1:10, and 1:20, respectively.

FIG. 6 shows a schematic diagram of the plasmid structure of the CAR-T constructed from a DLL3 humanized sequence.

FIG. 7 shows the killing effect of the CAR-T cells constructed from a DLL3 humanized sequence on NCI-H2171 cells for 72 h; the effector-to-target ratios (E:T) are 1:2, 1:10, and 1:20, respectively.

FIGs. 8A to 8D show the T cell characterization results of the CAR-T constructed from a DLL3 humanized sequence after 15 days of *in vitro* expansion. FIG. 8A shows the results of *in vitro* expansion of the CAR-T. FIGs. 8B, 8C, and 8D separately show the CD4/CD8 ratio and the expression level of T cell depletion phenotype and T cell memory

phenotype after CAR-T *in vitro* expansion.

FIGs. 9A and 9B show the assessment results of the *in vitro* tumor killing function of the CAR-T cells constructed from a DLL3 humanized sequence, wherein FIG. 9A shows the killing effect on SHP-77 cells for 72 h; the effector-to-target ratios (E:T) are 1:2, 1:10, and 1:20, respectively; FIG. 9B shows the killing effect on NCI-H2171 cells for 72 h; the effector-to-target ratios (E:T) are 1:2, 1:10, and 1:20, respectively.

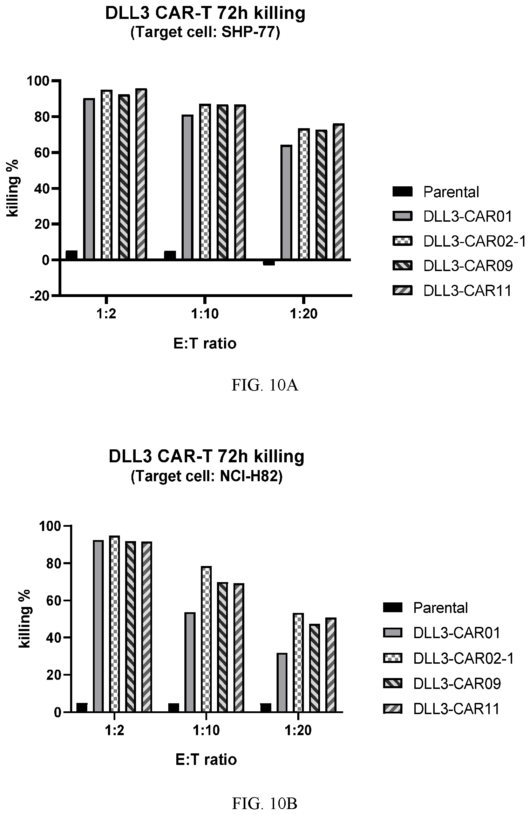

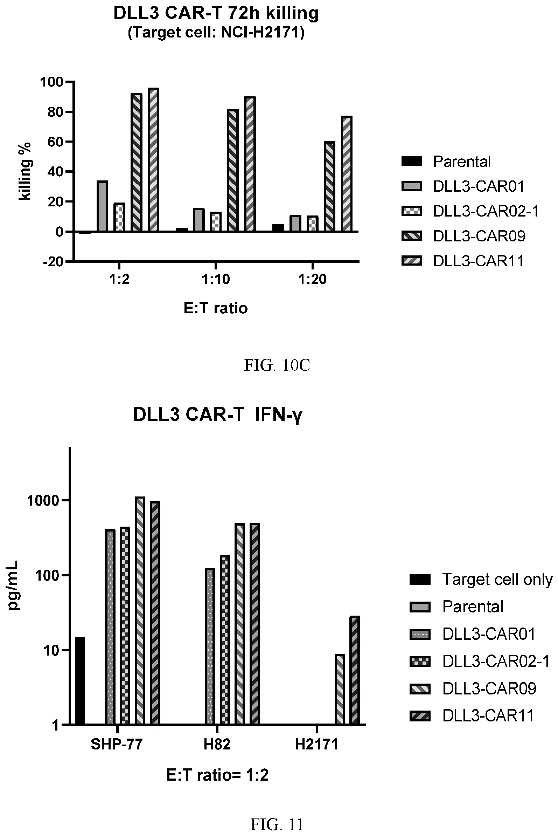

FIGs. 10A to 10C show the assessment results of the *in vitro* tumor killing function of the CAR-T cells constructed from a DLL3 humanized sequence, wherein FIG. 10A shows the killing effect of the DLL3 CAR-T cells on SHP-77 cells for 72 h; the effector-to-target ratios (E:T) are 1:2, 1:10, and 1:20, respectively; FIG. 10B shows the killing effect of the DLL3 CAR-T cells on NCI-H82 cells for 72 h; the effector-to-target ratios (E:T) are 1:2, 1:10, and 1:20, respectively; FIG. 10C shows the killing effect of the DLL3 CAR-T cells on NCI-H2171 cells for 72 h; the effector-to-target ratios (E:T) are 1:2, 1:10, and 1:20, respectively.

FIG. 11 shows the IFN-γ ELISA results of the killing culture supernatant of the CAR-T cells constructed from a DLL3 humanized sequence on SHP-77, NCI-H82, and NCI-H2171 cells for 72 h; the effector-to-target ratio (E:T) is 1:2.

FIG. 12 shows the *in vitro* expansion results of the CAR-T constructed from a DLL3 humanized sequence.

FIGs. 13A to 13C show the assay results of the phenotype of the DLL3 CAR-T cells, wherein FIGs. 13A, 13B, and 13C separately show the CD4 and CD8 cell typing, the expression level of depletion phenotypes TIM3 and LAG3, and the expression level of memory phenotype on day 11 of DLL3 CAR-T cell culture.

FIG. 14 shows the assessment results of the *in vitro* tumor killing function of the CAR-T cells constructed from a DLL3 humanized sequence on NCI-H2171 cells for 72 h; the effector-to-target ratios (E:T) are 1:2, 1:10, and 1:20, respectively.

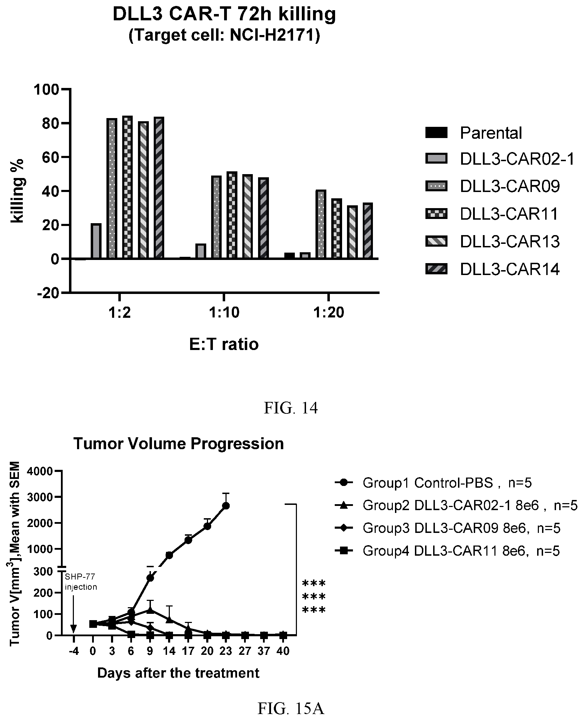

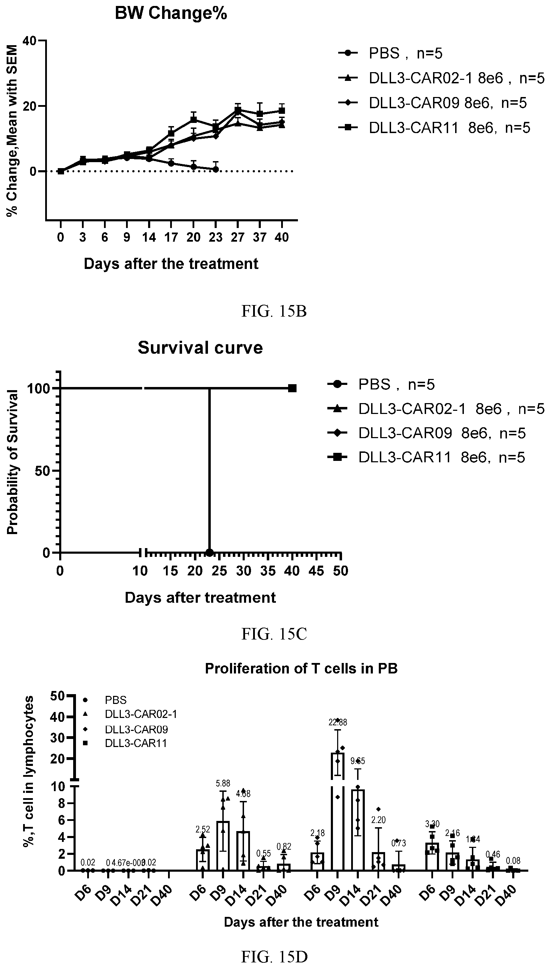

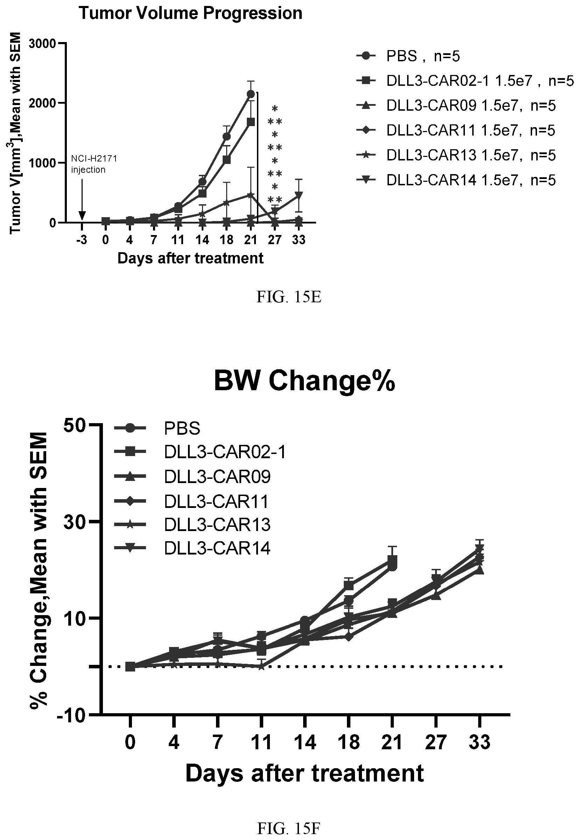

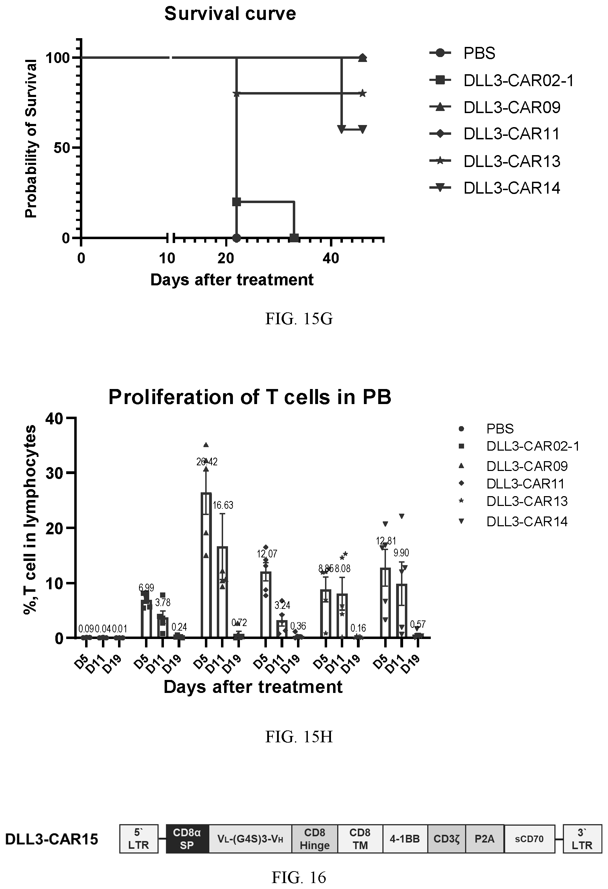

FIGs. 15A to 15H show assays of the anti-tumor efficacy of DLL3 CAR-T cells on a mouse subcutaneous tumor model, wherein FIG. 15A shows a tumor growth curve of a SHP-77 NPG mouse subcutaneous tumor model; FIG. 15B shows a mouse weight change curve of a SHP-77 NPG mouse subcutaneous tumor model; FIG. 15C shows a mouse survival curve of a SHP-77 NPG mouse subcutaneous tumor model; FIG. 15D shows the T cell assay results of peripheral blood from mice in a SHP-77 NPG mouse subcutaneous tumor model; FIG. 15E shows a tumor growth curve of a NCI-H2171 NPG mouse subcutaneous tumor model; FIG. 15F shows a mouse weight change curve of a NCI-H2171 NPG mouse subcutaneous tumor model; FIG. 15G shows a mouse survival curve of a NCI-H2171 NPG mouse subcutaneous tumor model; FIG. 15H shows the T cell assay results of peripheral blood from mice in a NCI-H2171 NPG mouse subcutaneous tumor model.

FIG. 16 shows a schematic diagram of the plasmid structure of a secretable CD70 armored DLL3 CAR-T.

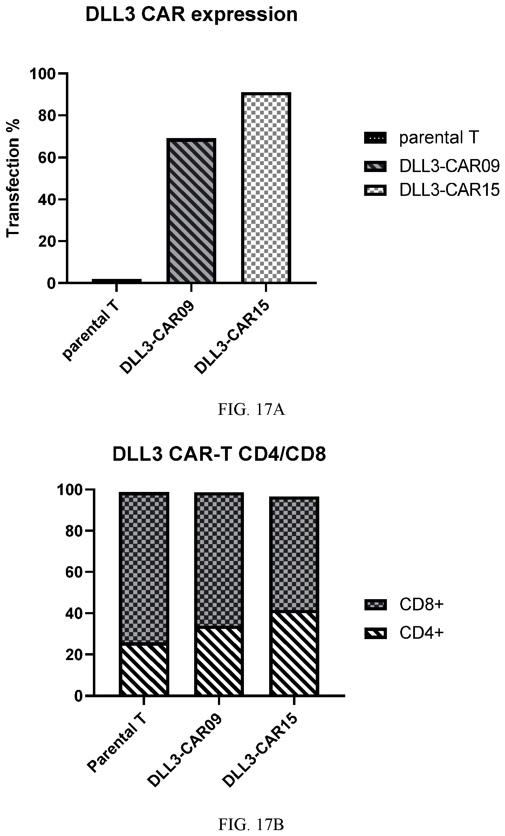

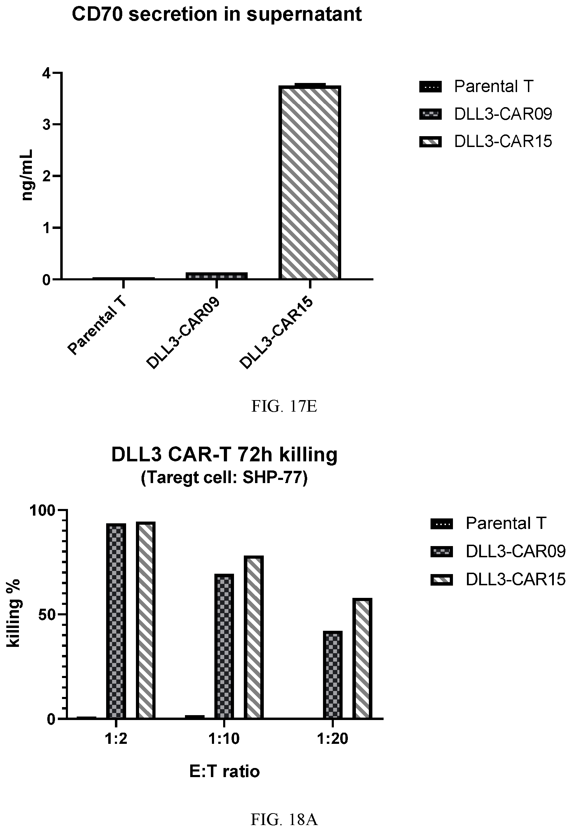

FIGs. 17A to 17E show T cell characterization results after transfection and one week of *in vitro* expansion of DLL3 CAR-T cells and secretable CD70 armored DLL3 CAR-T. FIGs. 17A to 17D separately show the expression levels of DLL3 CAR, the CD4/CD8 ratio, and the expression level of T cell depletion phenotype and T cell memory phenotype after CAR-T *in vitro* expansion. FIG. 17E shows the assay results of CD70 secretion from the secretable CD70 armored DLL3 CAR-T cells, and ELISA results of CD70 protein from a culture (3-day culture) supernatant that reaches a certain density of CAR-T (2E6/mL) cells during *in vitro* culture.

FIGs. 18A to 18C show *in vitro* killing assessment results of DLL3 CAR-T cells and secretable CD70 armored DLL3 CAR-T cells. FIG. 18A and FIG. 18B separately show the assessment results of the *in vitro* tumor killing function of DLL3 CAR-T cells and secretable CD70 armored DLL3 CAR-T cells on SHP-77 (FIG. 18A) and NCI-H2171 cells (FIG. 18B) for 72 h; the effector-to-target ratios (E:T) are 1:2, 1:10, and 1:20, respectively. FIG. 18C shows the assessment results of the continuous killing function of DLL3 CAR-T cells and secretable CD70 armored DLL3 CAR-T cells on NCI-H2171-luc cells.

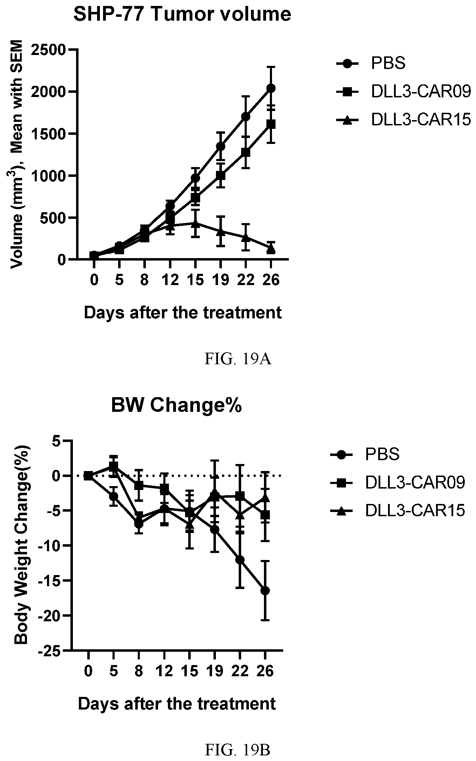

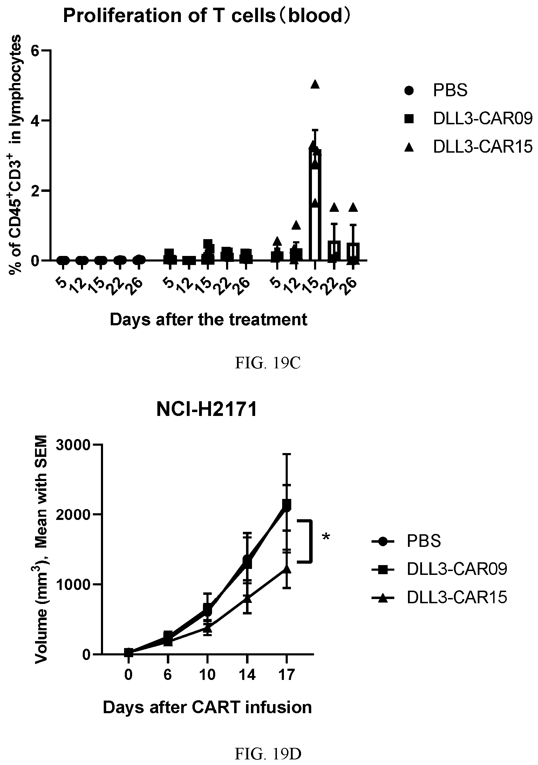

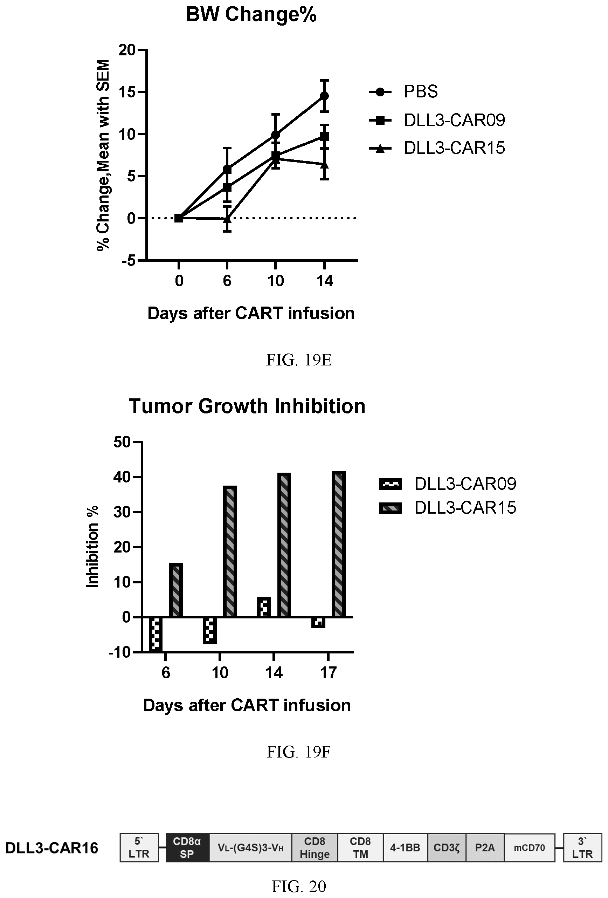

FIGs. 19A to 19F show assays of the anti-tumor efficacy of secretable CD70 armored DLL3 CAR-T cells on a mouse subcutaneous tumor model, wherein FIG. 19A shows a tumor growth curve of a SHP-77 NPG mouse subcutaneous tumor model; FIG. 19B shows a mouse weight change curve of a SHP-77 NPG mouse subcutaneous tumor model; FIG. 19C shows the T cell assay results of peripheral blood from mice in a SHP-77 NPG mouse subcutaneous tumor model; FIG. 19D shows a tumor growth curve of a NCI-H2171 NPG mouse subcutaneous tumor model; FIG. 19E shows a mouse weight change curve of a NCI-H2171 NPG mouse subcutaneous tumor model. FIG. 19F shows the tumor growth inhibition of each treatment group in the NCI-H2171 NPG mouse subcutaneous tumor model.

FIG. 20 shows a schematic diagram of the plasmid structure of a membrane-bound CD70 armored DLL3 CAR-T.

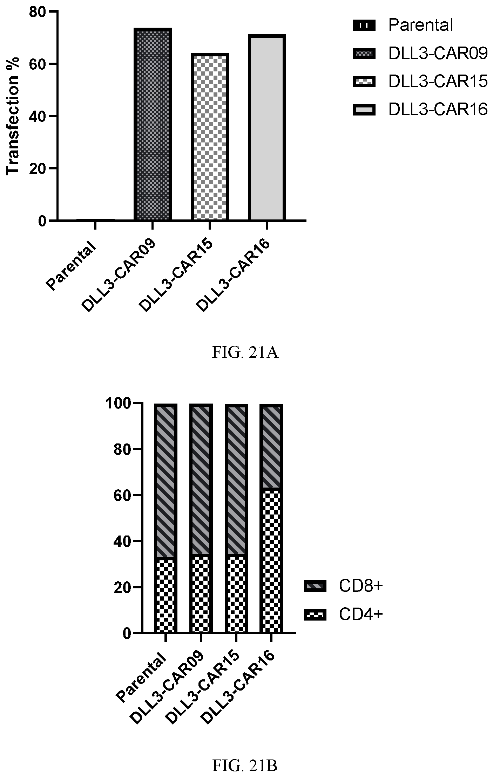

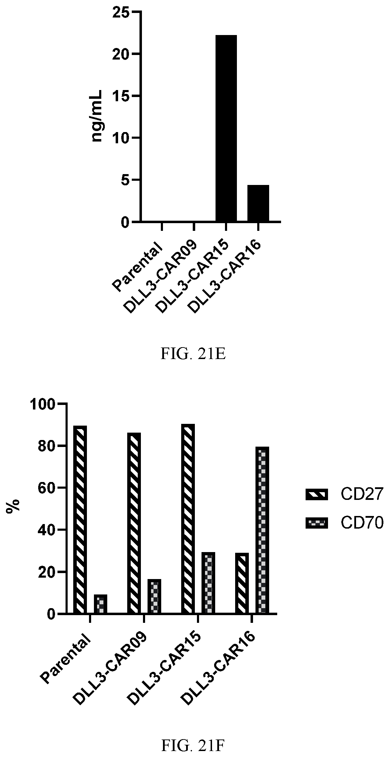

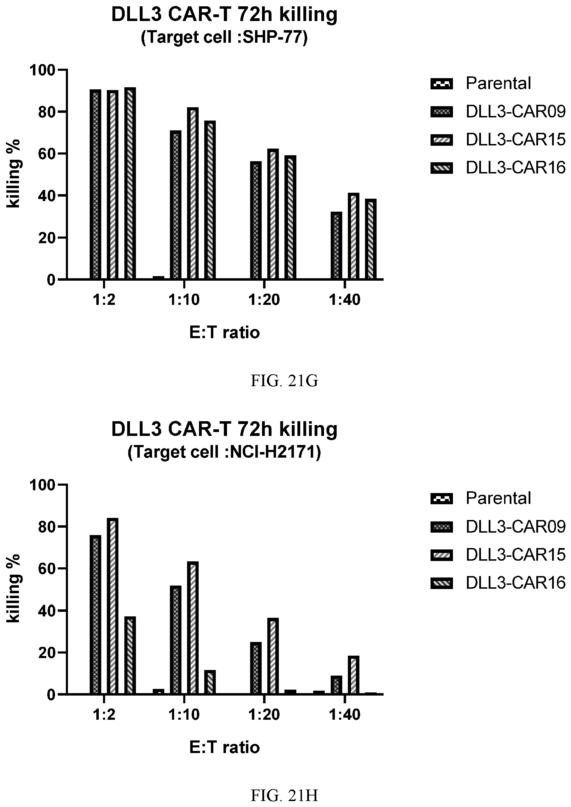

FIGs. 21A to 21H show T cell characterization results after transfection and one week of *in vitro* expansion of DLL3 CAR-T cells and secretable CD70 armored and membrane-bound CD70 armored DLL3 CAR-T. FIGs. 21A to 21D separately show the expression levels of DLL3 CAR, the CD4/CD8 ratio, and the expression level of T cell depletion phenotype and T cell memory phenotype after CAR-T *in vitro* expansion. FIG. 21E shows the assay results of CD70 secretion from DLL3 CAR-T cells, and ELISA results of CD70 protein from a culture (5-day culture) supernatant that reaches a certain density of CAR-T (2E6/mL) cells during *in vitro* culture. FIG. 21F shows the expression results of DLL3 CAR-T cell surface CD70 and CD27. FIG. 21G and FIG. 21H separately show the assessment results of the *in vitro* tumor killing function of non-armor, secretable CD70 armored and membrane-bound CD70 armored DLL3 CAR-

T cells on SHP-77 and NCI-H2171 cells for 72 h; the effector-to-target ratios (E:T) are 1:2, 1:10, 1:20, and 1:40, respectively.

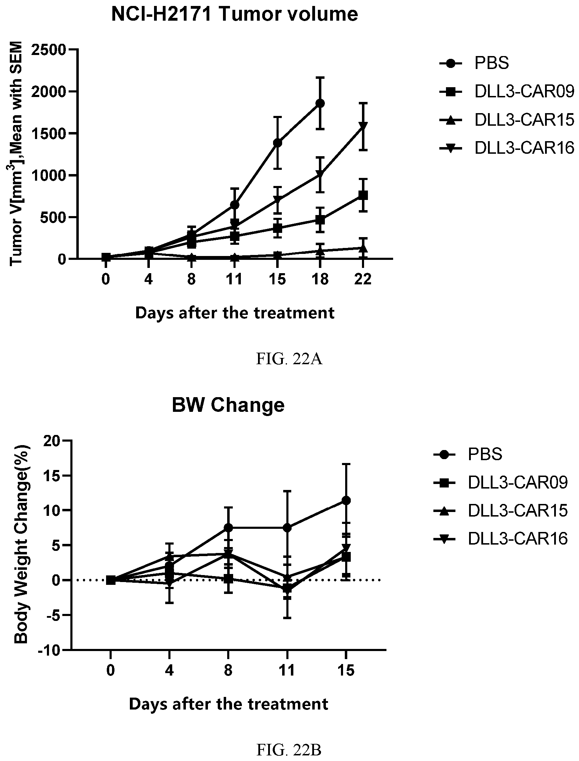

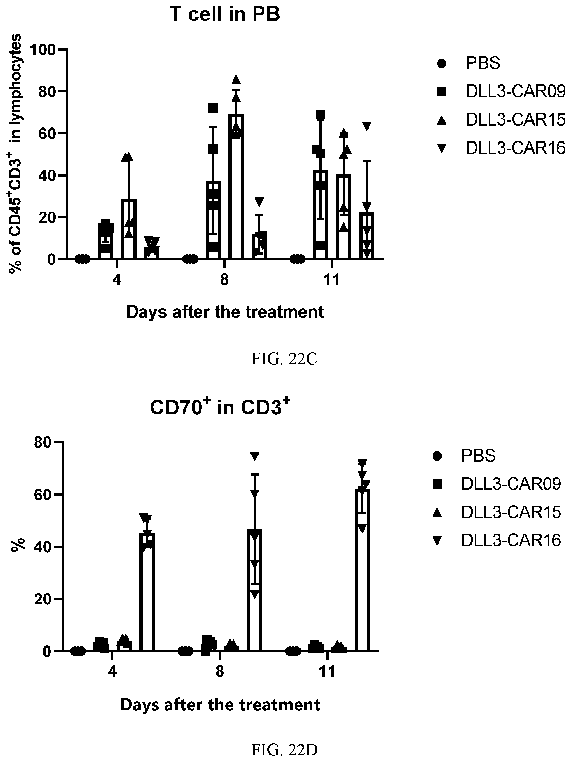

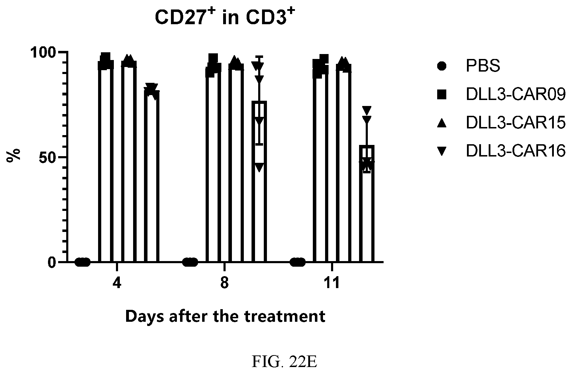

FIGs. 22A to 22E show assays of the anti-tumor efficacy of DLL3 CAR-T cells and secretable CD70 armored and membrane-bound CD70 armored DLL3 CAR-T cells on a mouse subcutaneous tumor model, wherein FIG. 22A shows a tumor growth curve of a NCI-H2171 NPG mouse subcutaneous tumor model; FIG. 22B shows a mouse weight change curve of a NCI-H2171 NPG mouse subcutaneous tumor model; FIG. 22C shows the T cell assay results of peripheral blood from mice in a NCI-H2171 NPG mouse subcutaneous tumor model; FIGs. 22D and 22E show the assay results of the expression levels of CD70 and CD27 on the mouse peripheral blood T cell surface.

## TERMINOLOGY AND DEFINITIONS

Unless otherwise defined herein, scientific and technical terms used in correlation with the present application shall have the meanings that are commonly understood by those skilled in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "include", "comprise", and "have" herein are used interchangeably and are intended to indicate the inclusion of a solution, implying that there may be elements other than those listed in the solution. Meanwhile, it should be understood that the descriptions "include", "comprise", and "have" used herein also provide the solution of "consist of ...".

The term "and/or" used herein includes the meanings of "and", "or", and "all or any other combination of elements linked by the term".

The term "delta-like ligand 3 (DLL3)" as used herein is a single transmembrane protein attached to the cell surface and is a member of the Notch ligand family. The human DLL3 gene is localized on chromosome 19q13 and has an open reading frame length of approximately 1800 bp. The human DLL3 protein consists of 619 amino acids, and its complete structure comprises one DSL domain, one intracellular domain, and six epidermal growth factor-like domains. The DSL gene sequence at the N-terminus of the extracellular domain is highly conserved in the ligand family, and the extracellular domain is an essential functional domain for binding to the Notch receptor. DLL3 has a short intracellular domain and its function remains unclear. Researches show that DLL3 is highly expressed in SCLC and other neuroendocrine tumors, but is rarely expressed in normal tissues. Activation of DLL3 may exert a pro-cancer or anti-cancer effect. DLL3 is widely expressed in human cancers, including small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, esophageal cancer, and the like.

The term "specifically bind to" herein refers to that an antigen-binding molecule (e.g., an antibody) specifically binds to an antigen and substantially identical antigens, generally with high affinity, but does not bind to unrelated antigens with high affinity. The affinity is generally reflected in an equilibrium dissociation constant (KD), where a relatively low KD indicates a relatively high affinity. In the case of antibodies, high affinity generally means having a KD of about $10^{-6}$ M or less, $10^{-7}$ M or less, about $10^{-8}$ M or less, or about $10^{-9}$ M or less. The KD is calculated as follows: KD = Kd/Ka, where Kd represents the dissociation rate and Ka represents the association rate. The equilibrium dissociation constant KD can be measured by methods well known in the art, such as surface plasmon resonance (e.g., Biacore) or equilibrium dialysis.

The term "antigen-binding molecule" herein is used in its broadest sense and refers to a molecule that specifically binds to an antigen. Illustratively, the antigen-binding molecule includes, but is not limited to, an antibody or an antibody mimetic. "Antibody mimetic" refers to an organic compound or a binding domain that is capable of specifically binding to an antigen, but is not structurally related to an antibody. Illustratively, the antibody mimetic includes, but is not limited to, affibody, affitin, affilin, a designed ankyrin repeat protein (DARPin), a nucleic acid aptamer, and a Kunitz domain peptide.

The term "antibody" herein is used in its broadest sense and refers to a polypeptide or a combination of polypeptides that comprises sufficient sequence from an immunoglobulin heavy chain variable region and/or sufficient sequence from an immunoglobulin light chain variable region to be capable of specifically binding to an antigen. "Antibody" herein encompasses various forms and various structures as long as they exhibit the desired antigen binding activity. The "antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer, I. P., Beste, G. & Skerra, A. (2003). Proteins, 53, 121-129.; Roque, A. C. A., Lowe, C. R. & Taipa, M. A. Antibodies and genetically engineered related molecules: production and purification. Biotechnol. Prog. 20, 639-654 (2004). Such scaffolds may also include non-antibody-derived scaffolds, such as scaffold proteins known in the art to be useful for grafting CDRs, including but not limited to, tenascin, fibronectin, peptide aptamers, and the like.

The term "antibody" includes intact antibodies and any antigen-binding fragments (i.e., "antigen-binding moiety") or single chains thereof. The "antibody" refers to a glycoprotein comprising at least two heavy (H) chains and two light (L)

chains that are linked together by disulfide bonds, or an antigen-binding moiety thereof. Each heavy chain consists of a heavy chain variable region (abbreviated herein as VH) and a heavy chain constant region. The heavy chain constant region consists of three domains: CH1, CH2, and CH3. Each light chain consists of a light chain variable region (abbreviated herein as VL) and a light chain constant region. The light chain constant region consists of one domain CL. The VH region and the VL region can be further subdivided into hypervariable regions called complementarity determining regions (CDRs), which are scattered among regions that are more conserved, termed framework regions (FRs). Each VH or VL consists of three CDRs and four FRs, which are arranged from the N-terminus to the C-terminus in the following order: FR1, CDR1, FR2, CDR2, FR3, CDR3, and FR4. The variable regions of the heavy and light chains comprise binding domains that can interact with antigens. The constant regions of the antibody may mediate the binding of the immunoglobulin to a host tissue or factor, and the host tissue or factor includes various cells (e.g., effector cells) of the immune system and the first component (C1q) of the classical complement system. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being μ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. The light chains can be divided into κ or λ chains according to the differences in the constant regions. Each of the five classes of Ig may have a κ chain or λ chain.

The term "CDR" herein may be numbered using the "IMGT numbering scheme", "Kabat numbering scheme", and "Chothia numbering scheme", where the "IMGT numbering scheme" generally refers to a numbering scheme based on the international ImMunoGeneTics information system (IMGT) initiated by Lefranc et al., see Lefranc, M. P., Pommié, C., Ruiz, M., Giudicelli, V., Foulquier, E., Truong, L., ... & Lefranc, G. (2003). IMGT unique numbering for immunoglobulin and T cell receptor variable domains and Ig superfamily V-like domains. Developmental & Comparative Immunology, 27(1), 55-77. The "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991). The "Chothia numbering scheme" herein generally refers to the immunoglobulin numbering scheme proposed by Chothia et al., which is a classical rule for identifying CDR region boundaries based on the position of structural loop regions (see, e.g., Chothia, C., & Lesk, A. M. (1987). Canonical structures for the hypervariable regions of immunoglobulins. Journal of molecular biology, 196(4), 901-917.).

The "antibody" herein also includes antibodies that do not comprise a light chain, e.g., heavy-chain antibodies (HCAbs) produced by *Camelus dromedarius*, *Camelus bactrianus*, *Lama glama*, *Lama guanicoe*, *Vicugna pacos*, and the like, as well as immunoglobulin new antigen receptors (Ig new antigen receptor, IgNAR) found in *Chondrichthyes*, e.g., shark.

The term "antibody" herein may be derived from any animal, including but not limited to human and non-human animals which may be selected from primates, mammals, rodents, and vertebrates, such as *Camelidae* species, *Lama glama*, *Lama guanicoe*, *Vicugna pacos*, sheep, rabbits, mice, rats, or *Chondrichthyes* (e.g., shark).

The term "heavy chain antibody" herein refers to an antibody lacking a light chain of a conventional antibody. The term specifically includes, but is not limited to, homodimeric antibodies comprising a VH antigen-binding domain and CH2 and CH3 constant domains in the absence of a CH1 domain.

As used herein, the term "chimeric antigen receptor (CAR)" refers to an artificial immune effector cell surface receptor that is engineered and expressed on an immune effector cell and specifically binds to an antigen, comprising, at least: (1) an extracellular antigen-binding domain, e.g., a variable heavy or light chain of an antibody, (2) a transmembrane domain that anchors the CAR on the immune effector cell, and (3) an intracellular signaling domain. The CAR is capable of redirecting T cells and other immune effector cells to a selected target, e.g., a cancer cell, in a non-MHC-restricted manner using the extracellular antigen-binding domain.

The term "signal peptide" herein refers to a fragment of a protein or polypeptide that is used to direct the protein or polypeptide into the secretory pathway, and transfer same to the cell membrane and/or cell surface. In some embodiments, the signal peptide is a CD8α signal peptide, and optionally, the CD8α signal peptide has at least 80% (e.g., at least 85%, at least 90%, at least 91%, at least 92%, at least 93%, at least 94%, at least 95%, at least 96%, at least 97%, at least 98%, at least 99%, or 100%) identity to the following sequence: MALPVTALLLPLALLLHAARP.

As used herein, the "transmembrane (TM) region" of a chimeric antigen receptor refers to a polypeptide structure that enables the chimeric antigen receptor to be expressed on the surface of an immune cell (e.g., a lymphocyte, an NK cell, or an NKT cell) and directs the cellular response of the immune cell against a target cell. The transmembrane domain may be natural or synthetic, and may be derived from any membrane-bound protein or transmembrane protein. The transmembrane domain can transduce a signal when the chimeric antigen receptor binds to a target antigen.

As used herein, the "hinge region" of a chimeric antigen receptor generally refers to any oligopeptide or polypeptide that serves to connect a transmembrane region and an antigen-binding region. In particular, the hinge region serves to provide greater flexibility and accessibility to the antigen-binding region. The hinge region may be derived

in whole or in part from a natural molecule, e.g., an extracellular domain derived in whole or in part from CD8, CD4, or CD28, or derived in whole or in part from an antibody constant region. Alternatively, the hinge region may be either a synthetic sequence corresponding to a naturally occurring hinge sequence, or a completely synthetic hinge sequence.

As used herein, the term "intracellular signaling domain" refers to a portion of a protein that transduces an effector function signal and directs a cell to exert a specified function. The intracellular signaling domain is responsible for the intracellular primary signaling after the antigen-binding domain binds to an antigen, resulting in the activation of the immune cell and immune response. In other words, the intracellular signaling domain is responsible for activating at least one of the normal effector functions of the immune cell in which the CAR is expressed. Exemplary intracellular signaling domains include CD3ζ.

As used herein, "co-stimulatory signaling binding domain" includes molecules on antigen-presenting cells (e.g., aAPC, dendritic cells, B cells, etc.) that specifically bind to homologous costimulatory molecules on T cells, thereby providing a signal that mediates T cell responses, including but not limited to proliferation, activation, differentiation, etc., in addition to the primary signal provided by, for example, binding of TCR/CD3 complex to peptide-loaded MHC molecule. Co-stimulatory signaling binding domains may include, but are not limited to, CD7, B7-1 (CD80), B7-2 (CD86), PD-L1, PD-L2, 4-1BBL, OX40L, inducible co-stimulatory ligand (ICOS-L), intercellular adhesion molecule (ICAM), CD30L, CD40, CD70, CD83, HLA-G, MICA, MICB, HVEM, lymphotoxin β receptor, 3/TR6, ILT3, ILT4, and agonists or antibodies that bind to Toll ligand receptors and ligands that specifically bind to B7-H3. Co-stimulatory signaling binding domains also include, among other things, antibodies that specifically bind to costimulatory molecules present on T cells, for example, but not limited to, CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds to CD83.

The term "immune effector cell" or "effector cell" as used herein refers to a cell involved in an immune response, e.g., promoting an immune effector response. Examples of the immune effector cells include T cells, e.g., α/β T cells and γ/delta T cells, B cells, natural killer (NK) cells, natural killer T (NKT) cells, mast cells, and myeloid-derived phagocytes.

The terms "T lymphocyte" and "T cell" herein are used interchangeably, and refer to the major type of white blood cells, which mature in the thymus and have multiple functions in the immune system, including recognizing specific foreign antigens *in vivo*, and activating and inactivating other immune cells in an MHC class I restricted manner. The T cell may be any T cell, for example, a cultured T cell such as a primary T cell, or a T cell from a cultured T cell line such as Jurkat or SupT1, or a T cell obtained from a mammal. The T cell may be a CD3+ cell. The T cell may be any type of T cell, and may be at any developmental stage, including but not limited to a CD4+/CD8+ double positive T cell, a CD4+ helper T cell (e.g., Th1 and Th2 cells), a CD8+ T cell (e.g., a cytotoxic T cell), a peripheral blood mononuclear cell (PBMC), a peripheral blood leukocyte (PBL), a tumor infiltrating lymphocyte (TIL), a memory T cell, a naive T cell, a regulatory T cell, a gamma delta T cells/γδT cell, and the like. Other types of helper T cells include, for example, Th3 (Treg), Th17, Th9, or Tfh cells. Other types of memory T cells include, for example, central memory T cells (Tcm cells), and effector memory T cells (Tem cells and TEMRA cells). The T cells may also refer to genetically engineered T cells, e.g., T cells modified to express a T cell receptor (TCR) or a chimeric antigen receptor (CAR). The T cells or T cell-like effector cells may also be differentiated from stem cells or progenitor cells. T cell-like derived effector cells may in some aspects have a T cell lineage, while also having one or more functional characteristics not present in primary T cells.

The term "CD70" herein is a type II transmembrane glycoprotein expressed in activated T cells, B cells, and mature dendritic cells (DCs). CD27 is a receptor for CD70 and needs to work in conjunction with it. The CD70/CD27 pathway plays an important role in human immune regulation, and the role in immune regulation is mainly shown in promoting the activation and proliferation of T cells, inducing the differentiation and formation of effector T cells and memory T cells and intervening regulatory T cells.

As used herein, "lentivirus" refers to a genus of the Retroviridae family. Lentiviruses are unique among retroviruses and are capable of infecting non-dividing cells. They can transmit a large amount of genetic information into the DNA of host cells, and are thus one of the most effective methods in gene delivery vectors. HIV, SIV, and FIV are examples of lentiviruses. Lentiviral-derived vectors provide a means to achieve significant levels of gene transfer *in vivo*.

As used herein, the term "vector" is a composition of matter that comprises an isolated nucleic acid and can be used to deliver the isolated nucleic acid to the interior of a cell. A variety of vectors are known in the art, including but not limited to, linear polynucleotides, polynucleotides associated with ionic or amphiphilic compounds, plasmids, and viruses. Thus, the term "vector" includes an autonomously replicating plasmid or virus. The term should also be construed to include non-plasmid and non-viral compounds that facilitate the transfer of nucleic acids into cells, e.g., polylysine compounds and liposomes. Examples of viral vectors include, but are not limited to, adenoviral vectors, adeno-associated viral vectors, retroviral vectors, and the like.

As used herein, the terms "percent (%) sequence consistency" and "percent (%) sequence identity" are interchangeable, and refer to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted

for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

As used herein, the term "at least 80% identity" is preferably 85%, 90%, 91%, 92%, 93%, 94%, 95%, 96%, 97%, 98%, 99%, or 100% identity.

As used herein, the term "mutation" includes insertion mutation, deletion mutation, and substitution mutation, and preferably, the substitution mutation is a conservative amino acid substitution. As used herein, the term "conservative amino acid" generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
Illustratively, the following six groups are examples of amino acids that are considered to be conservative replacements of each other:

1) alanine (A), serine (S), and threonine (T);
2) aspartic acid (D) and glutamic acid (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. In this context, the term "nucleic acid molecule" encompasses deoxyribonucleic acid (DNA), including, e.g., complementary DNA (cDNA) and genomic DNA; ribonucleic acid (RNA), in particular messenger RNA (mRNA); the synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone linkages or chemically modified residues. The nucleic acid molecule also encompasses DNA and RNA molecules suitable for use as vectors for direct expression of the antibodies of the present application *in vitro* and/or *in vivo*, e.g., in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule such that the mRNA can be injected into a subject to produce antibodies *in vivo* (see, e.g., Stadler, C. R., Bähr-Mahmud, H., Celik, L., Hebich, B., Roth, A. S., Roth, R. P., ... & Sahin, U. (2017). Elimination of large tumors in mice by mRNA-encoded bispecific antibodies. Nature medicine, 23(7), 815-817. or EP2101823B1).

The term "control sequence" herein refers to DNA sequences required for expression of an operably linked coding sequence in a particular host organism. Suitable control sequences for prokaryotes include, for example, promoters, optionally operator sequences and ribosome binding sites. Eukaryotic cells are known to utilize promoters, polyadenylation signals, and enhancers.

A nucleic acid is "operably linked" when it is placed into a functional relationship with another nucleic acid sequence. For example, DNA of a presequence or secretory leader sequence is operably linked to DNA of a polypeptide if it is expressed as a preprotein that participates in the secretion of the polypeptide; a promoter or enhancer is operably linked to a coding sequence if it affects the transcription of the sequence; alternatively, if a ribosome binding site is located so as to facilitate translation, it is operably linked to the coding sequence. Generally, "operably linked" means that the DNA sequences being linked are contiguous and, in the case of a secretory leader sequence, contiguous and in reading frame. However, enhancers need not be contiguous. The connection is achieved by a connection at a suitable restriction site. If

these sites are not present, synthetic oligonucleotide adaptors or linkers are used according to conventional practice.

The term "operably link" or "operably linked" refers to the juxtaposition of two or more biological sequences of interest in such a way that they are in a relationship permitting them to function in their intended manner, whether or not a spacer or linker is present. When used with respect to polypeptides, the term is intended to indicate that the polypeptide sequences are linked in such a way that the product of the linkage has the intended biological function. Illustratively, two or more polypeptide sequences may be linked using a self-cleaving peptide that separates the polypeptide sequences from each other upon expression, rather than being fused together, so that each can perform its respective function. The term may also apply to polynucleotides. For example, when a polynucleotide encoding a polypeptide is operably linked to a regulatory sequence (e.g., a promoter, enhancer, or silencer sequence), the term is intended to indicate that the polynucleotide sequence is linked in a way that allows for regulated expression of the polypeptide from the polynucleotide.

"Isolated" nucleic acid herein refers to a nucleic acid molecule that has been separated from components of its natural environment. The isolated nucleic acid includes a nucleic acid molecule contained in such a cell: the cell generally contains the nucleic acid molecule, but the nucleic acid molecule is present extrachromosomally or at a chromosomal location different from its natural chromosomal location.

The term "EC50" used herein refers to the half maximum effective concentration, which includes the antibody concentration that induces a halfway response between the baseline and maximum after a specified exposure time. EC50 essentially represents the antibody concentration at which 50% of the maximal effect is observed, and can be measured by methods known in the art.

The term "treatment" used herein refers to surgical or therapeutic treatment for the purpose of preventing or slowing (reducing) the progression of an undesired physiological or pathological change, e.g., cancer or tumor. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "subject" herein refers to an organism that receives the treatment for a particular disease or disorder described herein. Illustratively, the "subject" includes mammals, such as humans, primates (e.g., monkey), or non-primate mammals, that receive the treatment for a disease or disorder.

The term "effective amount" herein refers to an amount of a therapeutic agent that is effective to prevent or alleviate symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "cancer" herein refers to or describes a physiological condition in mammals that is typically characterized by unregulated cell growth. Included in this definition are benign and malignant cancers. The term "tumor" or "neoplasm" used herein refers to all neoplastic cell growth and proliferation, whether malignant or benign, and all precancerous and cancerous cells and tissues. The terms "cancer" and "tumor" are not mutually exclusive when referred to herein.

## DETAILED DESCRIPTION

The present application constructs a novel DLL3-targeting CAR and a related T cell product, the antigen-binding domain thereof being derived from an anti-DLL3 antibody with high specificity. The CAR and T cells are of important significance in the field of treating DLL3-expressing cancers.

In a first aspect of the present application, provided is a chimeric antigen receptor (CAR) comprising an extracellular binding region that specifically recognizes a delta-like ligand 3 (DLL3) protein, a hinge region, a transmembrane region, an intracellular co-stimulatory domain, and an intracellular signaling domain. The extracellular binding region comprises an antibody or an antigen-binding fragment thereof that specifically recognizes delta-like ligand 3 (DLL3). The antibody or the antigen-binding fragment thereof comprises HCDR1, HCDR2, and HCDR3 contained in the heavy chain variable region (VH) set forth in SEQ ID NO: 1, 3, 5, 83-84, 87, or 90, and LCDR1, LCDR2, and LCDR3 contained in the light chain variable region (VL) set forth in SEQ ID NO: 2, 4, 6, 81-82, 85-86, or 89.

In some specific embodiments, the antibody or the antigen-binding fragment thereof that specifically recognizes delta-like ligand 3 (DLL3) comprises the LCDR and HCDR below defined according to the Kabat numbering scheme:

(1) the LCDR1, the LCDR2, and the LCDR3 set forth in SEQ ID NOs: 10-12, and the HCDR1, the HCDR2, and the HCDR3 set forth in SEQ ID NOs: 7-9; or such 6 CDRs wherein one or more have 5, 4, 3, 2, or 1 amino acid substitution, deletion, or addition compared with the corresponding CDRs of the 6 CDRs described above;
(2) the LCDR1, the LCDR2, and the LCDR3 set forth in SEQ ID NOs: 10-12, and the HCDR1, the HCDR2, and the HCDR3 set forth in SEQ ID NOs: 7, 101, and 9; or such 6 CDRs wherein one or more have 5, 4, 3, 2, or 1 amino acid substitution, deletion, or addition compared with the corresponding CDRs of the 6 CDRs described above;
(3) the LCDR1, the LCDR2, and the LCDR3 set forth in SEQ ID NOs: 16-18, and the HCDR1, the HCDR2, and the HCDR3 set forth in SEQ ID NOs: 13-15; or such 6 CDRs wherein one or more have 5, 4, 3, 2, or 1 amino acid substitution, deletion, or addition compared with the corresponding CDRs of the 6 CDRs described above;
(4) the LCDR1, the LCDR2, and the LCDR3 set forth in SEQ ID NOs: 16, 17, and 88, and the HCDR1, the HCDR2, and the HCDR3 set forth in SEQ ID NOs: 13-15; or such 6 CDRs wherein one or more have 5, 4, 3, 2, or 1 amino acid substitution, deletion, or addition compared with the corresponding CDRs of the 6 CDRs described above;
(5) the LCDR1, the LCDR2, and the LCDR3 set forth in SEQ ID NOs: 22-24, and the HCDR1, the HCDR2, and the HCDR3 set forth in SEQ ID NOs: 19-21; or such 6 CDRs wherein one or more have 5, 4, 3, 2, or 1 amino acid substitution, deletion, or addition compared with the corresponding CDRs of the 6 CDRs described above;
(6) the LCDR1, the LCDR2, and the LCDR3 set forth in SEQ ID NOs: 22-24, and the HCDR1, the HCDR2, and the HCDR3 set forth in SEQ ID NOs: 19, 91, and 21; or such 6 CDRs wherein one or more have 5, 4, 3, 2, or 1 amino acid substitution, deletion, or addition compared with the corresponding CDRs of the 6 CDRs described above.

In some specific embodiments, the antibody or the antigen-binding fragment thereof that specifically recognizes delta-like ligand 3 (DLL3) comprises the LCDR and HCDR below defined according to the IMGT numbering scheme:

(7) the LCDR1, the LCDR2, and the LCDR3 set forth in SEQ ID NOs: 28-30, and the HCDR1, the HCDR2, and the HCDR3 set forth in SEQ ID NOs: 25-27; or such 6 CDRs wherein one or more have 5, 4, 3, 2, or 1 amino acid substitution, deletion, or addition compared with the corresponding CDRs of the 6 CDRs described above;
(8) the LCDR1, the LCDR2, and the LCDR3 set forth in SEQ ID NOs: 34-36, and the HCDR1, the HCDR2, and the HCDR3 set forth in SEQ ID NOs: 31-33; or such 6 CDRs wherein one or more have 5, 4, 3, 2, or 1 amino acid substitution, deletion, or addition compared with the corresponding CDRs of the 6 CDRs described above;
(9) the LCDR1, the LCDR2, and the LCDR3 set forth in SEQ ID NOs: 40-42, and the HCDR1, the HCDR2, and the HCDR3 set forth in SEQ ID NOs: 37-39; or such 6 CDRs wherein one or more have 5, 4, 3, 2, or 1 amino acid substitution, deletion, or addition compared with the corresponding CDRs of the 6 CDRs described above.

In some specific embodiments, the antibody or the antigen-binding fragment thereof that specifically recognizes delta-like ligand 3 (DLL3) comprises the LCDR and HCDR below defined according to the Chothia numbering scheme:

(10) the LCDR1, the LCDR2, and the LCDR3 set forth in SEQ ID NOs: 46-48, and the HCDR1, the HCDR2, and the HCDR3 set forth in SEQ ID NOs: 43-45; or such 6 CDRs wherein one or more have 5, 4, 3, 2, or 1 amino acid substitution, deletion, or addition compared with the corresponding CDRs of the 6 CDRs described above;
(11) the LCDR1, the LCDR2, and the LCDR3 set forth in SEQ ID NOs: 52-54, and the HCDR1, the HCDR2, and the HCDR3 set forth in SEQ ID NOs: 49-51; or such 6 CDRs wherein one or more have 5, 4, 3, 2, or 1 amino acid substitution, deletion, or addition compared with the corresponding CDRs of the 6 CDRs described above;
(12) the LCDR1, the LCDR2, and the LCDR3 set forth in SEQ ID NOs: 58-60, and the HCDR1, the HCDR2, and the HCDR3 set forth in SEQ ID NOs: 55-57; or such 6 CDRs wherein one or more have 5, 4, 3, 2, or 1 amino acid substitution, deletion, or addition compared with the corresponding CDRs of the 6 CDRs described above; or

in some specific embodiments, the antibody or the antigen-binding fragment thereof comprises a heavy chain variable region (VH) set forth in SEQ ID NO: 1, 3, 5, 83-84, 87, or 90, or a VH having 99%, 98%, 97%, 96%, 95%, 90%, 85%, or 80% identity to any one of the VHs; and a light chain variable region (VL) set forth in SEQ ID NO: 2, 4, 6, 81-82, 85-86, or 89, or a VL having 99%, 98%, 97%, 96%, 95%, 90%, 85%, or 80% identity to any one of the VLs.

In a preferred embodiment, the antibody or the antigen-binding fragment thereof comprises a VH set forth in SEQ ID NO: 1, and a VL set forth in SEQ ID NO: 2.

In a preferred embodiment, the antibody or the antigen-binding fragment thereof comprises a VH set forth in SEQ ID NO: 3, and a VL set forth in SEQ ID NO: 4.

In a preferred embodiment, the antibody or the antigen-binding fragment thereof comprises a VH set forth in SEQ ID NO: 5, and a VL set forth in SEQ ID NO: 6.

In a preferred embodiment, the antibody or the antigen-binding fragment thereof comprises a VH set forth in SEQ ID NO: 83 or 84, and a VL set forth in SEQ ID NO: 81.

In a preferred embodiment, the antibody or the antigen-binding fragment thereof comprises a VH set forth in SEQ

ID NO: 84, and a VL set forth in SEQ ID NO: 82.

In a preferred embodiment, the antibody or the antigen-binding fragment thereof comprises a VH set forth in SEQ ID NO: 87, and a VL set forth in SEQ ID NO: 85 or 86.

In a preferred embodiment, the antibody or the antigen-binding fragment thereof comprises a VH set forth in SEQ ID NO: 90, and a VL set forth in SEQ ID NO: 89.

In a preferred embodiment, the antibody or the antigen-binding fragment is in the form of an scFv having the structure of VH-linker-VL or VL-linker-VH.

In a preferred embodiment, the linker has the sequence set forth in SEQ ID NO: 70 or 71.

In some specific embodiments, the chimeric antigen receptor further comprises a signal peptide.

In a preferred embodiment, the signal peptide is a CD8 signal peptide.

In a preferred embodiment, the CD8 signal peptide has the amino acid sequence set forth in SEQ ID NO: 61.

In some specific embodiments, the hinge region is selected from a CD28 hinge region and a CD8α hinge region.

In a preferred embodiment, the hinge region comprises the amino acid sequence set forth in SEQ ID NO: 62 or 63.

In some specific embodiments, the transmembrane region is selected from CD4, CD8α, CD28, PD1, and/or 4-1BB transmembrane regions.

In a preferred embodiment, the transmembrane region is CD28 transmembrane region or CD8α transmembrane region.

In a preferred embodiment, the transmembrane region has the amino acid sequence set forth in SEQ ID NO: 64 or 65.

In some specific embodiments, the intracellular co-stimulatory signaling domain is selected from CD28 and 4-1BB intracellular co-stimulatory domains.

In a preferred embodiment, the intracellular co-stimulatory signaling domain has the amino acid sequence set forth in SEQ ID NO: 66 or 67.

In some specific embodiments, the intracellular signaling domain is a CD3ζ intracellular signaling domain.

In a preferred embodiment, the CD3ζ intracellular signaling domain has the sequence set forth in SEQ ID NO: 68.

In some specific embodiments, the chimeric antigen receptor further comprises a CD70 domain.

In a preferred embodiment, the CD70 domain has the sequence set forth in SEQ ID NO: 102 or 104.

In a preferred embodiment, the CD70 domain is linked to the intracellular signaling domain by P2A, the P2A having the sequence set forth in SEQ ID NO: 106.

In some specific embodiments, the chimeric antigen receptor comprises the amino acid sequence set forth in SEQ ID NO: 77, SEQ ID NO: 78, SEQ ID NO: 79, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 103, or SEQ ID NO: 105, or an amino acid sequence comprising at least 85%, at least 90%, at least 91%, at least 92%, at least 93%, at least 94%, at least 95%, at least 96%, at least 97%, at least 98%, at least 99%, or 100% sequence identity to any one of the amino acid sequences described above.

In a second aspect of the present application, provided is an isolated nucleic acid molecule encoding the chimeric antigen receptor according to the first aspect.

In some specific embodiments, the nucleic acid molecule is a DNA or an RNA, wherein the RNA is preferably an mRNA.

In a third aspect of the present application, provided is an expression vector comprising the isolated nucleic acid molecule according to the second aspect.

In a fourth aspect of the present application, provided is a cell comprising the chimeric antigen receptor according to the first aspect, or comprising the nucleic acid molecule according to the second aspect, or comprising the expression vector according to the third aspect.

In some specific embodiments, the cell is an immune cell; preferably, the immune cell is selected from T lymphocytes, NK cells, hematopoietic stem cells, pluripotent stem cells, and immune cells differentiated and cultured from embryonic stem cells.

In a fifth aspect of the present application, provided is a method for preparing a chimeric antigen receptor-modified immune cell, wherein the method comprises the step of delivering the nucleic acid molecule according to the second aspect or the expression vector according to the third aspect to an immune cell to be modified.

In a preferred embodiment, the means of delivery comprises viral transfection or delivery to the immune cells via cationic liposomes (e.g., LNP).

In a preferred embodiment, the method further comprises isolating and activating the immune cells prior to delivery.

In a preferred embodiment, the immune cell is selected from T lymphocytes, NK cells, hematopoietic stem cells, pluripotent stem cells, and immune cells differentiated and cultured from embryonic stem cells.

In a sixth aspect of the present application, provided is a pharmaceutical composition, wherein the pharmaceutical composition comprises the chimeric antigen receptor according to the first aspect, the nucleic acid molecule

according to the second aspect, the expression vector according to the third aspect, the cell according to the fourth aspect, or a product prepared by the method according to the fifth aspect, and a pharmaceutically acceptable carrier.

In a seventh aspect of the present application, provided is the pharmaceutical composition according to the sixth aspect for use in preventing and/or treating a tumor or cancer.

In specific embodiments, the tumor or cancer is selected from a solid tumor, a hematologic tumor, and a DLL3-expressing infiltrative cancer.

In a preferred embodiment, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

In an eighth aspect of the present application, provided is use of the chimeric antigen receptor according to the first aspect, the nucleic acid molecule according to the second aspect, the expression vector according to the third aspect, the cell according to the fourth aspect, a product prepared by the method according to the fifth aspect, or the pharmaceutical composition according to the sixth aspect in preparing a composition for preventing and/or treating a tumor or cancer.

In specific embodiments, the tumor or cancer is selected from a solid tumor, a hematologic tumor, and a DLL3-expressing infiltrative cancer.

In a preferred embodiment, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

In a ninth aspect of the present application, provided is a method for preventing and/or treating a tumor or cancer, comprising administering to a patient in need thereof an effective amount of the chimeric antigen receptor according to the first aspect, the nucleic acid molecule according to the second aspect, the expression vector according to the third aspect, the cell according to the fourth aspect, a product prepared by the method according to the fifth aspect, or the pharmaceutical composition according to the sixth aspect.

In specific embodiments, the tumor or cancer is selected from a solid tumor, a hematologic tumor, and a DLL3-expressing infiltrative cancer.

In a preferred embodiment, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

## Example

The present disclosure is further described below with reference to specific examples; the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples herein are illustrative only, and do not limit the scope of the present disclosure in any way. It will be appreciated by those skilled in the art that various modifications or substitutions may be made to the technical solutions of the present disclosure in form and details without departing from the spirit and scope of the present disclosure, and that these modifications and substitutions shall fall within the protection scope of the present disclosure.

### Example 1: Screening and Preparation of DLL3 Antibodies

Mice were immunized with DLL3 (27-492)-hFc protein (purchased from Acro, Cat. No. DL3-H5255), and a positive clone was screened by splenocyte fusion and hybridoma techniques. The variable region sequences of the antibody were obtained by sequencing. The CDR region sequences of the antibody were determined by the Kabat, Chothia, and IMGT numbering schemes, and prepared into scFv antibodies. The scFv antibodies finally obtained were named DLL3-scFv-1, DLL3-scFv-2, and DLL3-scFv-3.The chimeric antibodies were detected to have good binding activity to the cell SHP77 endogenously expressing DLL3 by flow cytometry assay (FACS). Biacore 8K (GE) instrument was used in the experiment. The affinity of the anti-DLL3 humanized antibodies to be detected for DLL3 antigen protein was determined using multi-cycle kinetics. The affinity of DLL3-scFv-1 was 3.81E-10 KD, the affinity of DLL3-scFv-2 was 2.16E-10 KD, and the affinity of DLL3-scFv-3 was 2.64E-10 KD.

The specific sequences are detailed in Tables 1 and 2.

Table 1. Amino acid sequences of variable regions of DLL3 chimeric antibodies

| Heavy and light chains | | Sequence No. | Amino acid sequence |
|---|---|---|---|
| DLL3-scFv-1 | VH | SEQ ID NO.1 | EVQLQQSGPVLVKPGASVKMSCKASGYTFTDYYMNWVKRSHGKSLEWIGIINPYNGGTSYNQKFRGKATLTVDKSSNTAYMELNSLTSEDSAVYYCSRYDYDYDVEGAMDYWGQGTSVTVSS |
| | VL | SEQ ID NO.2 | DIVMTQSQKFMSTSVGDRVSITCKASQNVGTAVAWYQQKPGQSPKLLIFSASIRYTGVPDRFTGSGSGTDFTLTISNMQSEDLADYFCQQYSSSPYTFGGGTKLEIK |
| DLL3-scFv-2 | VH | SEQ ID NO.3 | EVQLQQSGTVLARPGASVKMSCKTSGYTFTSYWMHWVKQSPGQGLEWIGTIYPGNSDTRYNQKFKDKAYVTAVTSASTAYMELSSLTNEESAVYYCTRSYDYGDLDYWGQGTTLTVSS |
| | VL | SEQ ID NO.4 | DIVMTQSPATLSVTPGDRVSLSCRASQTISDYLHWYQQRSHESPRLLIKYASQSISGIPSRFSGSGSGSDFTLSINSVEPEDVGVYYCQNGHSFPLTFGAGTKLELK |
| DLL3-scFv-3 | VH | SEQ ID NO.5 | QVQLQQSGAELVKPGASVRISCKASGYAFSNYWINWVKQRPGKGLEWIGQINPGDGDTIYNGKFKGKATLTADKSSSTAYMQLSSLTSEDSAVYFCEREGRNYGDFAYWGQGTTLTVSS |
| | VL | SEQ ID NO.6 | DIVLTQSPASLAVSLGQRATISCRASKSVSTSGFSYMHWYQQKPGQPPKLLIYLASNLESGVPARFSGSGSGTDFTLNIHPVEEEDAATYYCQHSRELPFTFGSGTKLEIK |

Table 2. Heavy and light chain CDR sequences of DLL3 chimeric antibodies

| **Kabat analysis** | | | | | | |
|---|---|---|---|---|---|---|
| **Antibody** | **Sequence No.** | **CDR1** | **Sequence No.** | **CDR2** | **Sequence No.** | **CDR3** |
| DLL3-scFv-1-VH | SEQ ID NO.7 | DYYMN | SEQ ID NO.8 | IINPYNGGTSYNQKFRG | SEQ ID NO.9 | YDYDYDVEGAMDY |
| DLL3-scFv-1-VL | SEQ ID NO.10 | KASQNVGTAVA | SEQ ID NO.11 | SASIRYT | SEQ ID NO.12 | QQYSSSPYT |
| DLL3-scFv-2-VH | SEQ ID NO.13 | SYWMH | SEQ ID NO.14 | TIYPGNSDTRYNQKFKD | SEQ ID NO.15 | SYDYGDLDY |
| DLL3-scFv-2-VL | SEQ ID NO.16 | RASQTISDYLH | SEQIDNO.17 | YASQSIS | SEQ ID NO.18 | QNGHSFPLT |
| DLL3-scFv-3-VH | SEQ ID NO.19 | NYWIN | SEQ ID NO.20 | QINPGDGDTIYNGKFKG | SEQ ID NO.21 | EGRNYGDFAY |
| DLL3-scFv-3-VL | SEQ ID NO.22 | RASKSVSTSGFSYMH | SEQ ID NO.23 | LASNLES | SEQ ID NO.24 | QHSRELPFT |

(continued)

| IMGT | | | | | | |
|---|---|---|---|---|---|---|
| Antibody | Sequence No. | CDR1 | Sequence No. | CDR2 | Sequence No. | CDR3 |
| DLL3-scFv-1-VH | SEQ ID NO.25 | GYTFTDYY | SEQ ID NO.26 | INPYNGGT | SEQ ID NO.27 | SRYDYDYDVEGAMDY |
| DLL3-scFv-1-VL | SEQ ID NO.28 | QNVGTA | SEQ ID NO.29 | SA | SEQ ID NO.30 | QQYSSSPYT |
| DLL3-scFv-2-VH | SEQ ID NO.31 | GYTFTSYW | SEQ ID NO.32 | IYPGNSDT | SEQ ID NO.33 | TRSYDYGDLDY |
| DLL3-scFv-2-VL | SEQ ID NO.34 | QTISDY | SEQ ID NO.35 | YA | SEQ ID NO.36 | QNGHSFPLT |
| DLL3-scFv-3-VH | SEQ ID NO.37 | GYAFSNYW | SEQ ID NO.38 | INPGDGDT | SEQ ID NO.39 | EREGRNYGDFAY |
| DLL3-scFv-3-VL | SEQ ID NO.40 | KSVSTSGFSY | SEQ ID NO.41 | LA | SEQ ID NO.42 | QHSRELPFT |
| **Chothia analysis** | | | | | | |
| DLL3-scFv-1-VH | SEQ ID NO.43 | GYTFTDY | SEQ ID NO.44 | NPYNGG | SEQ ID NO.45 | YDYDYDVEGAMDY |
| DLL3-scFv-1-VL | SEQ ID NO.46 | KASQNVGTAVA | SEQ ID NO.47 | SASIRYT | SEQ ID NO.48 | QQYSSSPYT |
| DLL3-scFv-2-VH | SEQ ID NO.49 | GYTFTSY | SEQ ID NO.50 | YPGNSD | SEQ ID NO.51 | SYDYGDLDY |
| DLL3-scFv-2-VL | SEQ ID NO.52 | RASQTISDYLH | SEQ ID NO.53 | YASQSIS | SEQ ID NO.54 | QNGHSFPLT |
| DLL3-scFv-3-VH | SEQ ID NO.55 | GYAFSNY | SEQ ID NO.56 | NPGDGD | SEQ ID NO.57 | EGRNYGDFAY |
| DLL3-scFv-3-VL | SEQ ID NO.58 | RASKSVSTSGFSYMH | SEQ ID NO.59 | LASNLES | SEQ ID NO.60 | QHSRELPFT |

**Example 2: Preparation of Retrovirus Expressing Second-Generation Chimeric Antigen Receptor**

In this example, retroviral shuttle plasmids expressing second-generation antigen receptors that bind to DLL3 were separately constructed: P-1, P-2, P-2-1, P-3, P-4, and P-5, and the number corresponding to CAR are DLL3-CAR01, DLL3-CAR02, DLL3-CAR02-1, DLL3-CAR03, DLL3-CAR04, and DLL3-CAR05, respectively. The specific steps were as follows:

**1. Plasmid construction**

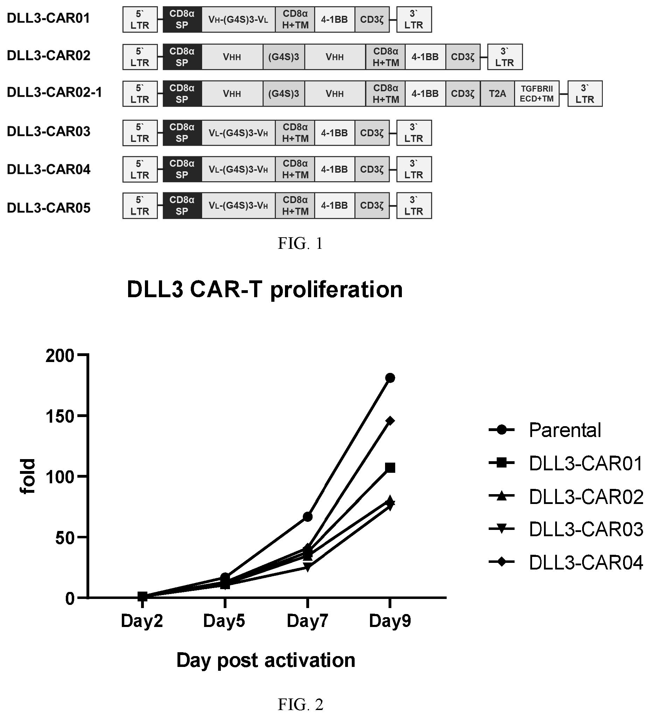

Referring to FIG. 1, a chimeric antigen receptor for T cells was constructed, a nucleic acid encoding the chimeric antigen receptor for T cells was cloned into a retroviral shuttle plasmid, and virus packaging was performed by the packaging plasmid.

The construction of the relevant plasmid was carried out using conventional molecular biology methods in the art. The chimeric antigen receptors used in this example are second-generation chimeric antigen receptors having the following elements: a CD8α signal peptide (SEQ ID NO: 61), an antigen-binding domain, a CD8α hinge region (SEQ ID NO: 62) or CD28 hinge region (SEQ ID NO: 63), a CD8α transmembrane domain (SEQ ID NO: 64) or a CD28 transmembrane domain (SEQ ID NO: 65), a 4-1BB intracellular co-stimulatory domain (SEQ ID NO: 66) or CD28 intracellular co-stimulatory domain (SEQ ID NO: 67), a CD3ζ signaling domain (SEQ ID NO: 68), a T2A cleavage peptide (SEQ ID NO: 69), or other intracellular domains. The amino acid sequences of the elements are shown in Table 3.

Table 3. sequence listing of elements of second-generation chimeric antigen receptor

| SEQ ID NO | Name of element | Amino acid sequence |
|---|---|---|
| 61 | CD8α signal peptide | MALPVTALLLPLALLLHAARP |
| 62 | CD8α hinge region | TTTPAPRPPTPAPTIASQPLSLRPEACRPAAGGAVHTRGLDFACD |
| 63 | CD28 hinge region | IEVMYPPPYLDNEKSNGTIIHVKGKHLCPSPLFPGPSKP |
| 64 | CD8α transmembrane domain | IYIWAPLAGTCGVLLLSLVITLYC |
| 65 | CD28 transmembrane domain | FWVLVVVGGVLACYSLLVTVAFIIFWV |
| 66 | 4-1BB intracellular costimulatory domain | KRGRKKLLYIFKQPFMRPVQTTQEEDGCSCRFPEEEEGGCEL |
| 67 | CD28 intracellular costimulatory domain | RSKRSRLLHSDYMNMTPRRPGPTRKHYQPYAPPRDFAAYRS |
| 68 | CD3ζ signaling domain | RVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 69 | T2A cleavage peptide | EGRGSLLTCGDVEENPGP |
| 70 | Linker | GGGGSGGGGSGGGGS |
| 71 | Linker | GGGGSGGGGSGGGGSGGGGS |
| 72 | DLL3-scFv-1 (construction of DLL3-CAR03) | DIVMTQSQKFMSTSVGDRVSITCKASQNVGTAVAWYQQKPGQSPKLLIFSASIRYTGVPDRFTGSGSGTDFTLTISNMQSEDLADYFCQQYSSSPYTFGGGTKLEIKGGGGSGGGGSGGGGSEVQLQQSGPVLVKPGASVKMSCKASGYTFTDYYMNWVKRSHGKSLEWIGIINPYNGGTSYNQKFRGKATLTVDKSSNTAYMELNSLTSEDSAVYYCSRYDYDYDVEGAMDYWGQGTSVTVSS |
| 73 | DLL3-scFv-2 (construction of DLL3-CAR04) | DIVMTQSPATLSVTPGDRVSLSCRASQTISDYLHWYQQRSHESPRLLIKYASQSISGIPSRFSGSGSGSDFTLSINSVEPEDVGVYYCQNGHSFPLTFGAGTKLELKGGGGSGGGGSGGGGSEVQLQQSGTVLARPGASVKMSCKTSGYTFTSYWMHWVKQSPGQGLEWIGTIYPGNSDTRYNQKFKDKAYVTAVTSASTAYMELSSLTNEESAVYYCTRSYDYGDLDYWGQGTTLTVSS |

(continued)

| SEQ ID NO | Name of element | Amino acid sequence |
|---|---|---|
| 74 | DLL3-scFv-3 (construction of DLL3-CAR05) | DIVLTQSPASLAVSLGQRATISCRASKSVSTSGFSYMHWYQQKPGQPPKLLIYLASNLESGVPARFSGSGSGTDFTLNIHPVEEEDAATYYCQHSRELPFTFGSGTKLEIKGGGGSGGGGSGGGGSQVQLQQSGAELVKPGASVRISCKASGYAFSNYWINWVKQRPGKGLEWIGQINPGDGDTIYNGKFKGKATLTADKSSSTAYMQLSSLTSEDSAVYFCEREGRNYGDFAYWGQGTTLTVSS |

The antibodies or the antigen-binding domains used for the chimeric antigen receptors in this example all bind to DLL3 antigen, wherein the antigen-binding domains of CAR constructs DLL3-CAR01, DLL3-CAR02, DLL3-CAR02-1, DLL3-CAR03, DLL3-CAR04, and DLL3-CAR05 are single-chain variable fragments (scFvs) or variable domain of heavy chain of heavy-chain antibody (VHH). Within the scFvs, heavy chain variable regions and light chain variable regions are linked by a linker, and the sequences are set forth in SEQ ID NO: 70 or SEQ ID NO: 71.

The DLL3-scFv-1 sequence of DLL3-CAR03 (plasmid number: P-3) is set forth in SEQ ID NO: 72, wherein the heavy chain variable region (variable domain of heavy chain, VH) has the amino acid sequence set forth in SED ID NO: 1, and has complementarity determining regions (CDRs) HCDR1, HCDR2, and HCDR3 set forth in SEQ ID NOs: 7-9, 25-27, or 43-45; the light chain variable region (variable domain of light chain, VL) has the amino acid sequence set forth in SED ID NO: 2, and has complementarity determining regions (CDRs) LCDR1, LCDR2, and LCDR3 set forth in SEQ ID NOs: 10-12, 28-30, or 46-48; the VH and the VL are linked by a linker, and the amino acid sequence is set forth in SEQ ID NO: 70. The CAR amino acid sequence of DLL3-CAR03 is SEQ ID NO: 77 (see Table 4 for details)

The DLL3-scFv-2 sequence of DLL3-CAR04 (plasmid number: P-4) is set forth in SEQ ID NO: 73, wherein the VH has the amino acid sequence set forth in SED ID NO: 3, and has HCDR1, HCDR2, and HCDR3 set forth in SEQ ID NOs: 13-15, 31-33, or 49-51; the VL has the amino acid sequence set forth in SED ID NO: 4, and has LCDR1, LCDR2, and LCDR3 set forth in SEQ ID NOs: 16-18, 34-36, or 52-54; the VH and the VL are linked by a linker, and the amino acid sequence is set forth in SEQ ID NO: 70. The CAR amino acid sequence of DLL3-CAR04 is SEQ ID NO: 78 (see Table 4 for details).

The DLL3-scFv-3 sequence of DLL3-CAR05 (plasmid number: P-5) is set forth in SEQ ID NO: 74, wherein the VH has the amino acid sequence set forth in SED ID NO: 5, and has HCDR1, HCDR2, and HCDR3 set forth in SEQ ID NOs: 19-21, 37-39, or 55-57; the VL has the amino acid sequence set forth in SED ID NO: 6, and has LCDR1, LCDR2, and LCDR3 set forth in SEQ ID NOs: 22-24, 40-42, or 58-60; the VH and the VL are linked by a linker, and the amino acid sequence is set forth in SEQ ID NO: 70. The CAR amino acid sequence of DLL3-CAR05 is SEQ ID NO: 79 (see Table 4 for details).

DLL3-CAR01 (plasmid number: P-1) is a positive control (from patent US20210107979A1). The chimeric antigen receptor uses a binding fragment that is scFv binding to DLL3, the VH and the VL are linked by a linker, and the amino acid sequence is set forth in SEQ ID NO: 71. The specific amino acid sequence of DLL3-CAR01 is SEQ ID NO: 75 (see Table 4 for details).

DLL3-CAR02 (plasmid number: P-2) is a positive control (from patent WO2021008610A1). The chimeric antigen receptor uses an antigen-binding fragment that is VHH binding to DLL3, the two VHHs are linked in tandem by a linker (SEQ ID NO: 70), and the specific amino acid sequence of DLL3-CAR02 is set forth in SEQ ID NO: 76 (see Table 4 for details).

DLL3-CAR02-1 (plasmid number: P-2-1) is a positive control (from patent WO2021008610A1), in which TGF-β-DNR is added by T2A (SEQ ID NO: 69) on the basis of the structure of DLL3-CAR02, and the specific amino acid sequence is set forth in SEQ ID NO: 80 (see Table 4 for details).

Table 4. Amino acid sequence listing of second-generation chimeric antigen receptor

| SEQ ID NO: | Plasmid number | CAR number | Amino acid sequence |
|---|---|---|---|
| 75 | P-1 | DLL3-CAR01 | MALPVTALLLPLALLLHAARPQLQLQESGPGLVKPSETLSLTCTVSGGSISSSSYYWGWIRQPPGKGLEWIGSIYYSGNIYHNPSLKSRVSISVDTSKNQFSLRLSSVTAADTAVYYCAREIIVGATHFDYWGQGTLVTVSSGGGGSGGGGSGGGGSGGGGSAIQMTQSPSSLSASVGDRVTITCRASQGIRNDLGWYQQKPGKAPELLIYAASSLQSGVPSRFSGSGSGTDFTLTISSLQPEDFATYYCLQDYNYPLTFGPGTKVDIKTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 76 | P-2 | DLL3-CAR02 | MALPVTALLLPLALLLHAARPQVQLVESGGGVVQPGGSLRLSCAASFSGYGVSTMAWFRQAPGKGLEGVAAITVGSGNTYYADSVKGRFTISRDNSKNTVYLQMNSLRAEDTAMYYCAVGYLSGGSWDVPGRYNYWGQGTLVTVSSGGGGSGGGGSGGGGSEVQLVESG |
| | | | GGLVQPGGSLRLSCAASGNTYSSNYMGWFRQAPGKGLEEVAVIYTRGGHTYYVDSVRGRFTISQDNAKNSLYLQMNSLRAEDTAVYYCAASSRHRLGLNNPRDYDYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 77 | P-3 | DLL3-CAR03 | MALPVTALLLPLALLLHAARPDIVMTQSQKFMSTSVGDRVSITCKASQNVGTAVAWYQQKPGQSPKLLIFSASIRYTGVPDRFTGSGSGTDFTLTISNMQSEDLADYFCQQYSSSPYTFGGGTKLEIKGGGGSGGGGSGGGGSEVQLQQSGPVLVKPGASVKMSCKASGYTFTDYYMNWVKRSHGKSLEWIGIINPYNGGTSYNQKFRGKATLTVDKSSNTAYMELNSLTSEDSAVYYCSRYDYDYDVEGAMDYWGQGTSVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |

(continued)

| SEQ ID NO: | Plasmid number | CAR number | Amino acid sequence |
|---|---|---|---|
| 78 | P-4 | DLL3-CAR04 | MALPVTALLLPLALLLHAARPDIVMTQSPATLSVTPGDRVSLSCRASQTISDYLHWYQQRSHESPRLLIKYASQSISGIPSRFSGSGSGSDFTLSINSVEPEDVGVYYCQNGHSFPLTFGAGTKLELKGGGGSGGGGSGGGGSEVQLQQSGTVLARPGASVKMSCKTSGYTFTSYWMHWVKQSPGQGLEWIGTIYPGNSDTRYNQKFKDKAYVTAVTSASTAYMELSSLTNEESAVYYCTRSYDYGDLDYWGQGTTLTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 79 | P-5 | DLL3-CAR05 | MALPVTALLLPLALLLHAARPDIVLTQSPASLAVSLGQRATISCRASKSVSTSGFSYMHWYQQKPGQPPKLLIYLASNLESGVPARFSGSGSGTDFTLNIHPVEEEDAATYYCQHSRELPFTFGSGTKLEIKGGGGSGGGGSGGGGSQVQLQQSGAELVKPGASVRISCKASGYAFSNYWINWVKQRPGKGLEWIGQINPGDGDTIYNGKFKGKATLTADKSSSTAYMQLSSLTSEDSAVYFCEREGRNYGDFAYWGQGTTLTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDA |
| | | | LHMQALPPR |

(continued)

| SEQ ID NO: | Plasmid number | CAR number | Amino acid sequence |
|---|---|---|---|
| 80 | P-2-1 | DLL3-CAR02-1 | MALPVTALLLPLALLLHAARPQVQLVESGGGVVQPGGSLRLSCAASFSGYGVSTMAWFRQAPGKGLEGVAAITVGSGNTYYADSVKGRFTISRDNSKNTVYLQMNSLRAEDTAMYYCAVGYLSGGSWDVPGRYNYWGQGTLVTVSSGGGGSGGGGSGGGGSEVQLVESGGGLVQPGGSLRLSCAASGNTYSSNYMGWFRQAPGKGLEEVAVIYTRGGHTYYVDSVRGRFTISQDNAKNSLYLQMNSLRAEDTAVYYCAASSRHRLGLNNPRDYDYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPRGSGEGRGSLLTCGDVEENPGPMGRGLLRGLWPLHIVLWTRIASTIPPHVQKSVNNDMIVTDNNGAVKFPQLCKFCDVRFSTCDNQKSCMSNCSITSICEKPQEVCVAVWRKNDENITLETVCHDPKLPYHDFILEDAASPKCIMKEKKKPGETFFMCSCSSDECNDNIIFSEEYNTSNPDLLLVIFQVTGISLLPPLGVAISVIIIFYCYRVNRQQKLSS |

A polynucleotide encoding the chimeric antigen receptor was synthesized, and an enzyme cutting site and a shuttle plasmid homologous sequence were added at the two ends thereof. The shuttle plasmid (Xitu Biotechnology) was digested with restriction endonucleases EcoRI (Thermo, Cat#FD0274) and SalI (Thermo, Cat#FD0644), and the linear plasmids were recovered and purified after agarose gel electrophoresis. The polynucleotide and the linearized vector synthesized in the above steps were linked via recombinase 5× In-FusionHD enzyme (TaKaRa, Cat#ST0344) in the following reaction system: 2 μL of polynucleotide fragment (50 ng/μL), 1 μL of linearized plasmid (50 ng/μL), 2 μL of 5× HD In-Fusion enzyme, and 5 μL of ddH$_2$O. The system was well mixed, briefly centrifuged, and reacted at 50 °C for 15 min. 10 μL of the recombinant reaction product was added to 100 μL of the bacterial competent cell stbl3 (Frdbio, Cat#MCC0910) and placed on ice for 5 min. The transformed bacterium solution was uniformly applied on an LB plate containing 100 μg/mL ampicillin and cultured in an inverted state in a thermostatic incubator for 12-16 h. From each plate, 3-5 clones were randomly picked for sequencing identification. The correctly sequenced bacterium solution was transferred to a 100 mL LB liquid medium containing 100 μg/mL ampicillin, and cultured overnight at 37 °C. The plasmids were extracted with MN endotoxin-free plasmid extraction Midi kit (MN, Cat#740420.50), and after quantification, diluted to 1000 ng/μL with endotoxin-free ultrapure water.

**2. Preparation of retrovirus**

293T cells (the Cell Bank of Type Culture Collection of Chinese Academy of Sciences, Cat#GNHu17) were inoculated onto a 100 mm culture dish for culture, and the medium was DMEM medium (Gibco, Cat#10566016) supplemented with 10% FBS (Gibco, Cat#10099141). When the cell fusion degree reached about 70%, plasmid transfection was performed: the retroviral shuttle plasmid expressing the chimeric antigen receptor was mixed with a retroviral packaging plasmid and added to 50 μL of Opti-MEM medium (Thermofisher Scientific, Cat#31985070), and 420 μL of Opti-MEM medium containing 30 μL of Fugene HD (Promega, Cat#04709691001) was added. The mixture was well mixed and incubated at room temperature for 15 min. Finally, the mixture was added to 293T cells and cultured at 37 °C with 5% CO$_2$ for 3 days. The supernatant was collected, filtered through a 0.45 μm filter membrane and concentrated for later use.

**Example 3: Preparation and Identification of DLL3 CAR-T**

### 1. Preparation of DLL3 CAR-T cell

T cells were isolated from fresh PBMC using Stemcell Easy Sep kit (Stemcell, Cat#19055). The isolated T cells were transferred to a culture dish previously coated with 1 μg/mL of CD3/CD28 antibody (MY-ebioscience-16-0289-85, MY-ebioscience-16-0037-85) and cultured. The medium components were X-VIVO15 (Lonza, Cat#BEBP02-054Q), 5% human AB serum (Gemini, Cat#100-512), 100 U/mL penicillin-streptomycin (Gibco, Cat#15140-122), and 200 IU/mL human IL2 (Beijing SL, Cat#S19991007).

The well-conditioned T cells described above were added to a 24-well cell culture plates coated with 5 μg/mL recombinant human fibrin (Takara, Cat#T100B), 500 μL of the concentrated retrovirus prepared in Example 2 was added, and the plate was centrifuged at 4 °C at 2000 g for 1 h. After the virus supernatant was removed, 3E5 T cells were added and centrifuged at 400 g for 5 min. After centrifugation, the cells were cultured at 37 °C with 5% $CO_2$, counted 2 times per week, and subcultured and expanded when the cell density reached $2.5 \times 10^6$/mL. The DLL3 CAR-T cells were expanded by fold as shown in FIG. 2. As can be seen from the results, all CAR-T cells had good amplification efficiency, with DLL3-CAR04 expansion efficiency being the most excellent, higher than the positive control.

### 2. FACS detection of CAR transfection efficiency and T cell phenotype

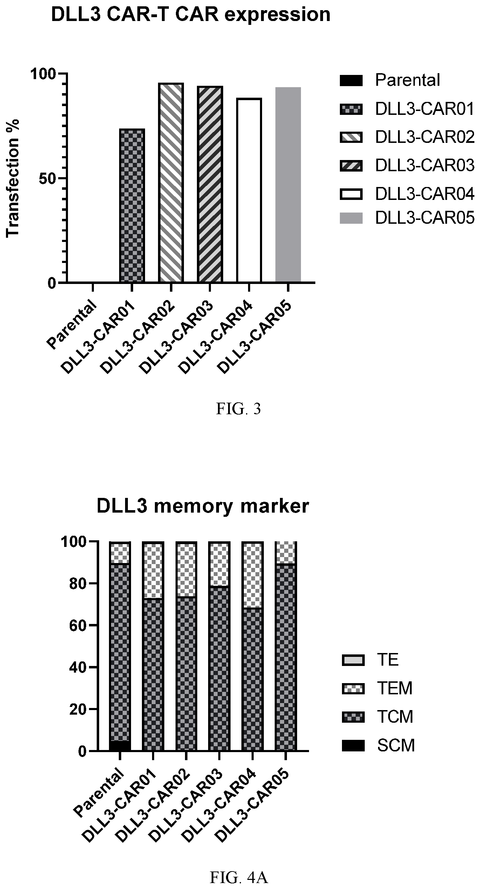

After washing the cells 2 times with PBS buffer, the cells were counted and diluted to 2E6 cells/mL with PBS buffer, and 50 μL of Fc blocking reagent (BioLegend, Cat#422302) was added. The mixture was incubated at room temperature for 10 min, and then added to a 96-well FACS reaction plate at 100 μL per well. 100 μL of his-tagged DLL3 antigen protein (3 μg/mL) was added, and the mixture was incubated at 4 °C for 30 min. The plate was centrifuged and washed 2 times with a FACS buffer, and a fluorescent (AF647)-labeled secondary antibody (GenScript, Cat#A01802) was added at 100 μL/well. The mixture was incubated at 4 °C for 20 min. After the plate was centrifuged and washed 3 times with a FACS buffer, the cells were suspended with a 200 μL FACS buffer, and assay and analysis were performed by FACS (CANTOII, purchased from BD). The results are shown in FIG. 3. The expression rate of CAR-T of DLL3-CAR03, DLL3-CAR04, and DLL3-CAR05 was 80% or more, showing good viral transfection efficiency.

The memory phenotype of T cells after 8 days of transfection was also assayed by flow cytometry, and the expression of central memory T cells (CM, $CD62L^+CD45RO^+$), effective memory T cells (EM, $CD62L^-CD45RO^+$), stem cell-like memory T cells (SCM, $CD62L^+CD45RO^-$), and effector T cells (EF, $CD62L^-CD45RO^-$) of the T cells was assayed. $2 \times 10^5$ cells were taken from the T cells in each group, placed in 96-well FACS reaction plates, and centrifuged at 400 g for 5 min. The supernatant was removed. T cell phenotype antibody mixture diluted with FACS buffer was added, and the mixture was incubated at 4 °C for 30 min. After the plate was centrifuged and washed 3 times with a FACS buffer, the cells were suspended with a 200 μL FACS buffer, and assay and analysis were performed by FACS (CANTOII, purchased from BD). The results show that the TCM and TEM phenotypes of DLL3 CAR-T cells were similar to untransfected T cells (Parental group), as shown in FIG. 4A.

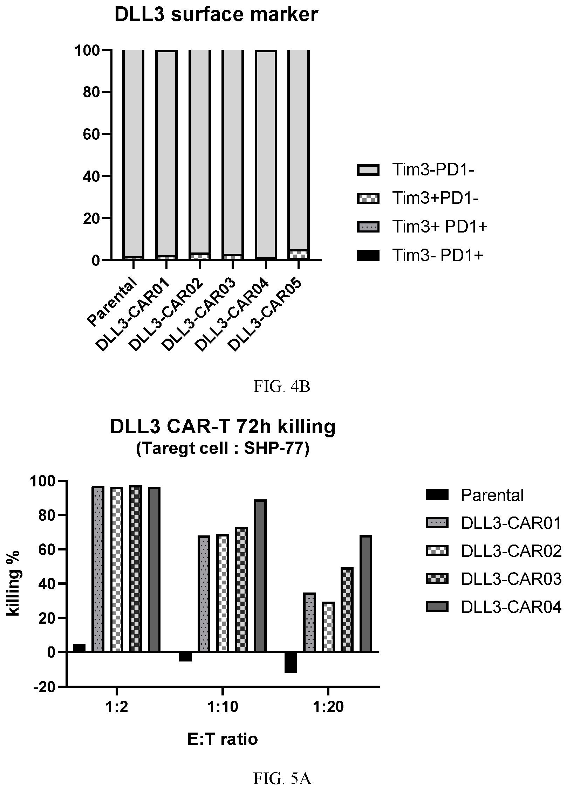

T cell depletion phenotype was detected 8 days after transfection using flow cytometry. The TIM3 and PD-1 expression of DLL3-CAR03, DLL3-CAR04, and DLL3-CAR05 groups was not significantly up-regulated compared with the positive reference DLL3-CAR01 and DLL3-CAR02 groups, as shown in FIG. 4B.

## Example 4: *In Vitro* Tumor Killing Function Assessment of DLL3 CAR-T

### 1. *In vitro* specific tumor cell killing function assessment of DLL3 CAR-T

To verify the *in vitro* killing toxicity of DLL3 CAR-T cells prepared in Example 3, *in vitro* tumor killing function assessment was performed using the tumor cell lines SHP-77 and NCI-H2171 (purchased from ATCC) expressing human DLL3.

The culture medium containing the suspended target cells in the flask was collected into a 50 mL centrifuge tube, centrifuged at 400 g for 5 min, and the supernatant was removed. Fresh medium was added, and the cells were pipetted uniformly using a pipette. After counting the concentration of the cell suspension with a counting plate, $1 \times 10^7$ cells were taken out, labeled with CFSE (Biolegend, Cat#423801), and added to a 96-well FACS plate at 40000 cells per well. T cells and empty T cells with different CAR structures were added according to T cell:target cell ratios of 1:2, 1:10, and 1:20, and after 72 h of co-incubation, centrifuged at 400 g for 5 min. The supernatant was removed. The cells were suspended with a 200 μL FACS buffer, and assay and analysis were performed by FACS (CANTOII, purchased from BD).

The 72 h specific killing results are shown in FIGs. 5A-B. The killing effect of DLL3-CAR03 and DLL3-CAR04 on the cell line SHP-77 highly expressing human DLL3 was equal to that of the positive reference DLL3-CAR01 and DLL3-CAR02 at a DLL3-CAR03:DLL3-CAR04 ratio of 1:2 and was significantly better than that of the two positive reference DLL3-CAR01 and DLL3-CAR02 as the killing ratio decreased, especially at a ratio of 1:20 (FIG. 5A); the killing effect of DLL3-CAR03, DLL3-CAR04, and DLL3-CAR05 was significantly better than that of the positive reference on the cell line

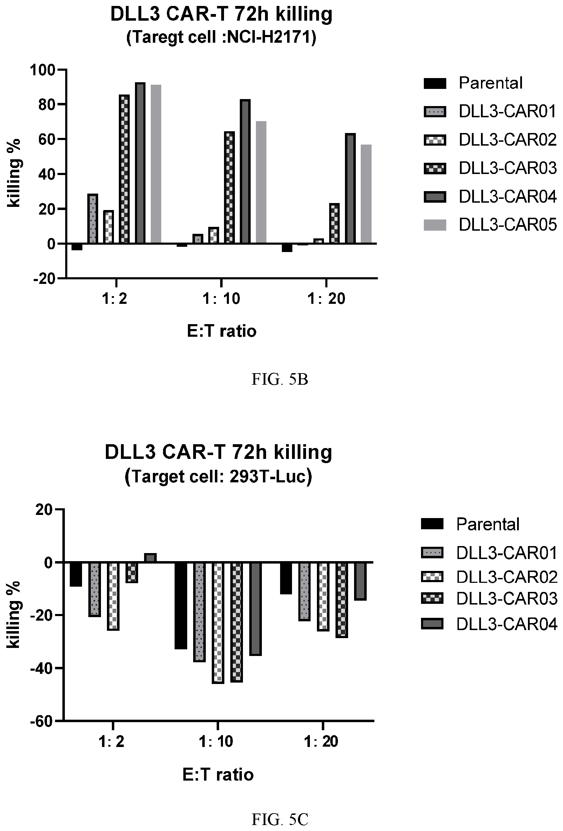

NCI-H2171 with low expression of human DLL3 under three different ratios (FIG. 5B).

### 2. *In vitro* non-specific cell killing function assessment of DLL3 CAR-T

To verify the non-specific killing toxicity of DLL3 CAR-T cells, a self-constructed negative cell 293T stably transfected cell strain 293T-luc highly expressing human bacterial luciferase was used. The 293T-luc cells were transformed with luciferase reporter gene by conventional gene manipulation methods. 293T-luc expressed luciferase. The luciferase reporter gene assay reagent was used for determining the fluorescence intensity and reflecting the cell viability and the killing effect of T cells. The killing rate calculation formula is as follows:

$$\text{Killing rate} = (\text{target cell well reading - assay well reading})/\text{target cell well reading} \times 100\%.$$

The specific assay method was as follows: non-specific target cells (293T-luc) were plated in 96-well plates at 40000 cells per well; T cells and empty T cells with different CAR structures were added according to T cell:target cell ratios of 1:2, 1:10, and 1:20; after 24 h and 72 h of co-incubation, firefly luciferase substrate D-Luciferin (Abcam, Cat#ab143655) was added, and biofluorescence reading and result analysis were carried out using a PE EnSight microplate reader. As shown in FIG. 5C, the DLL3 CAR-T selected structure did not exhibit any non-specific killing in killing experiments on the negative cell 293T-luc.

## Example 5: Preparation of Retrovirus and CAR-T Cell Expressing Second-Generation Humanized Antigen Receptor

### 1. Humanized design for DLL3 murine antibody

To eliminate possible immunogenicity issues with murine sequences, humanized design was performed on the antibody variable region sequences in the CAR structures of DLL3-CAR03, DLL3-CAR04, and DLL3-CAR05 CAR-T.

By alignment with the IMGT database (http://imgt.cines.fr) for germline genes from heavy and light chain variable regions of human antibodies, germline genes, with high homology with the murine antibodies, from heavy and light chain variable regions were selected as templates, and CDRs of the murine antibodies were separately grafted into corresponding human templates to give variable region sequences in the order of FR1-CDR1-FR2-CDR2-FR3-CDR3-FR4. A humanized monoclonal antibody was obtained by back-mutating key amino acids in a framework sequence to amino acids corresponding to the murine antibody as needed to ensure the original affinity. The amino acid residues of the CDRs of the antibodies were identified and annotated by the kabat numbering scheme.

### 2. Humanization of DLL3-scFv-1

For murine antibody DLL3-scFv-1, the humanized light chain templates were IGKV1-39*01/IGKV4-1*01 and IGKJ4*01, and the humanized heavy chain templates were IGHV1-3*01 and IGHJ6*01. The CDRs of murine antibody DLL3-scFv-1 were separately grafted into their human templates, so as to give the corresponding humanized versions. Key amino acids in FR sequences of the humanized antibodies of DLL3-CAR03 were back-mutated to amino acids corresponding to the murine antibody as needed to ensure the original affinity. Back mutation design is shown in Table 5.

Table 5. Back mutation design for humanized antibodies of DLL3-scFv-1

| VL | | VH | |
|---|---|---|---|
| L1 | Graft(IGKV1-39*01) + A43S, Y49F | H2a | Graft(IGHV1-3*01) + I70L, R72V, T74K, A97S + G56S |
| L3 | Graft(IGKV4-1*01) + P43S, Y49F | H5 | Graft(IGHV1-3*01) + Q39R, R72V, T74K, A97S |
| Note: Graft denotes that the CDRs of the murine antibody are grafted into the human germline template FR sequences; A43S denotes that A at position 43 of Graft is mutated to S, and so on for others. The numbering of the back-mutated amino acids is Kabat numbering. | | | |

Specific sequences of the variable regions of DLL3-scFv-1 humanized antibody are shown below, wherein the CDR regions determined by the Kabat numbering scheme are underlined (see Table 6 for the amino acid sequence), and the back mutations are shown with character borders:

DIQMTQSPSSLSASVGDRVTITCKASQNVGTAVAWYQQKPGKSPKLLIFSASIRYTGVPSRFS GSGSGTDFTLTISSLQPEDFATYYCQQYSSSPYTFGGGTKVEIK (DLL3-CAR03 VL1, SEQ ID NO: 81).

DIVMTQSPDSLAVSLGERATINCKASQNVGTAVAWYQQKPGQSPKLLIFSASIRYTGVPDRFS GSGSGTDFTLTISSLQAEDVAVYYCQQYSSSPYTFGGGTKVEIK (DLL3-CAR03 VL3, SEQ ID NO: 82).

EVQLVQSGAEVKKPGASVKVSCKASGYTFTDYYMNWVRQAPGQRLEWMGIINPYNSGTSY NQKFRGRVTLTVDKSASTAYMELSSLRSEDTAVYYCSRYDYDYDVEGAMDYWGQGTTVT VSS (DLL3-CAR03 VH2a, SEQ ID NO: 83).

EVQLVQSGAEVKKPGASVKVSCKASGYTFTDYYMNWVRRAPGQRLEWMGIINPYNGGTS YNQKFRGRVTITVDKSASTAYMELSSLRSEDTAVYYCSRYDYDYDVEGAMDYWGQGTTVT VSS (DLL3-CAR03 VH5, SEQ ID NO: 84).

Table 6. Kabat analysis results for CDR sequences of heavy/light chain variable regions of DLL3-scFv-1 humanized antibodies

| Heavy and light chain variable regions | CDR1 | CDR2 | CDR3 |
|---|---|---|---|
| L1 and L3 | KASQNVGTAVA (SEQ ID NO: 10) | SASIRYT (SEQ ID NO: 11) | QQYSSSPYT (SEQ ID NO: 12) |
| H2a | DYYMN (SEQ ID NO: 7) | IINPYNSGTSYNQKFRG (SEQ ID NO: 101) | YDYDYDVEGAMDY (SEQ ID NO: 9) |
| H5 | DYYMN (SEQ ID NO: 7) | IINPYNGGTSYNQKFRG (SEQ ID NO: 8) | YDYDYDVEGAMDY (SEQ ID NO: 9) |

From the back mutation design for the light chain and heavy chain variable regions of the DLL3-scFv-1 humanized antibodies, different light chain and heavy chain sequences were selected respectively for cross combination to finally obtain 3 humanized antibodies, as detailed in Table 7.

Table 7. DLL3-scFv-1 humanized antibody variable region combinations

| VH / VL | VH2a | VH5 |
|---|---|---|
| VL1 | DLL3-scFv-1-VH2aVL1 / DLL3-CAR06 | DLL3-scFv-1-VH5VL1 / DLL3-CAR07 |
| VL3 | | DLL3-scFv-1-VH5VL3 / DLL3-CAR08 |

### 3. Humanization of DLL3-scFv-2

For murine antibody DLL3-scFv-2, the humanized light chain templates were IGKV2-28*01 and IGKJ2*01, and the humanized heavy chain templates were IGHV1-3*01 and IGHJ6*01. The CDRs of murine antibody DLL3-scFv-2 were separately grafted into their human templates, so as to give the corresponding humanized versions. Key amino acids in FR sequences of the humanized antibodies of DLL3-scFv-2 were back-mutated to amino acids corresponding to the murine antibody as needed to ensure the original affinity. Back mutation design is shown in Table 8.

Table 8. Back mutation design for humanized antibodies of DLL3-scFv-2

| VL | | VH | |
|---|---|---|---|
| L1a | Graft(IGKV2-28*01) + Y49K, T69S + N90Q | H1 | Graft(IGHV1-3*01) + R72A, A97T |
| L2 | Graft(IGKV2-28*01) + Q45R, Y49K | | |

Specific sequences of the variable regions of DLL3-scFv-2 humanized antibody are shown below, wherein the CDR regions determined by the Kabat numbering scheme are underlined (see Table 9 for the amino acid sequence), and the back mutations are shown with character borders:

DIVMTQSPLSLPVTPGEPASISCRASQTISDYLHWYLQKPGQSPQLLIKYASQSISGVPDRFSGSGSGSDFTLKISRVEAEDVGVYYCQQGHSFPLTFGQGTKLEIK (DLL3-scFv-2 VL1a, SEQ ID NO: 85)

DIVMTQSPLSLPVTPGEPASISCRASQTISDYLHWYLQKPGQSPRLLIKYASQSISGVPDRFSGSGSGTDFTLKISRVEAEDVGVYYCQNGHSFPLTFGQGTKLEIK (DLL3-scFv-2 VL2, SEQ ID NO: 86)

EVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSS (DLL3-scFv-2VH1, SEQ ID NO: 87)

Table 9. Kabat analysis results for CDR sequences of heavy/light chain variable regions of DLL3-scFv-2 humanized antibodies

| Heavy and light chain variable regions | CDR1 | CDR2 | CDR3 |
|---|---|---|---|
| L1a | RASQTISDYLH (SEQ ID NO: 16) | YASQSIS (SEQ ID NO: 17) | QQGHSFPLT (SEQ ID NO: 88) |
| L2 | RASQTISDYLH (SEQ ID NO: 16) | YASQSIS (SEQ ID NO: 17) | QNGHSFPLT (SEQ ID NO: 18) |
| H1 | SYWMH | TIYPGNSDTRYNQKFKD | SYDYGDLDY |
| | (SEQ ID NO: 13) | (SEQ ID NO: 14) | (SEQ ID NO: 15) |

From the back mutation design for the light chain and heavy chain variable regions of the DLL3-scFv-2 humanized antibodies, different light chain and heavy chain sequences were selected respectively for cross combination to finally obtain 2 humanized antibodies, as detailed in Table 10.

Table 10. DLL3-scFv-2 humanized antibody variable region combinations

| VH / VL | VH |
|---|---|
| VL1a | DLL3-scFv-2-VH1VL1a / DLL3-CAR09 |
| VL2 | DLL3-scFv-2-VH1VL2 / DLL3-CAR10 |

**4. Humanization of DLL3-scFv-3**

For murine antibody DLL3-scFv-3, the humanized light chain templates were IGKV3-11*01 and IGKJ2*01, and the humanized heavy chain templates were IGHV1-69*02 and IGHJ6*01. The CDRs of murine antibody DLL3-scFv-3 were separately grafted into their human templates, so as to give the corresponding humanized versions. Key amino acids in FR sequences of the humanized antibodies of DLL3-scFv-3 were back-mutated to amino acids corresponding to the murine antibody as needed to ensure the original affinity. Back mutation design is shown in Table 11.

Table 11. Back mutation design for humanized antibodies of DLL3-scFv-3

| VL | | VH | |
|---|---|---|---|
| L2 | Graft(IGKV3-11*01) + A47P | H2a | Graft(IGHV1-69*02) + G27Y,T28A + G62S |

Specific sequences of the variable regions of DLL3-scFv-3 humanized antibody are shown below, wherein the CDR regions determined by the Kabat numbering scheme are underlined (see Table 12 for the amino acid sequence), and the back mutations are shown with character borders:

EIVLTQSPATLSLSPGERATLSCRASKSVSTSGFSYMHWYQQKPGQPPRLLIYLASNLESGIPARFSGSGSGTDFTLTISSLEPEDFAVYYCQHSRELPFTFGQGTKLEIK (DLL3-scFv-3 VL2, SEQ ID NO: 89)

EVQLVQSGAEVKKPGSSVKVSCKASGYAFSNYWINWVRQAPGQGLEWMGQINPGDGDTIYNSKFKGRVTITADKSTSTAYMELSSLRSEDTAVYYCEREGRNYGDFAYWGQGTTVTVSS (DLL3-scFv-3 VH2a, SEQ ID NO: 90)

Table 12. Kabat analysis results for CDR sequences of heavy/light chain variable regions of DLL3-scFv-3 humanized antibodies

| Heavy and light chain variable regions | CDR1 | CDR2 | CDR3 |
|---|---|---|---|
| L2 | RASKSVSTSGFSYMH (SEQ ID NO: 22) | LASNLES (SEQ ID NO: 23) | QHSRELPFT (SEQ ID NO: 24) |
| H2a | NYWIN (SEQ ID NO: 19) | QINPGDGDTIYNSKFKG (SEQ ID NO: 91) | EGRNYGDFAY (SEQ ID NO: 21) |

**5. Plasmid construction**

Retroviral shuttle plasmids expressing second-generation chimeric antigen receptors that bind to DLL3 were constructed: P-6 to P-14, and the number corresponding to CAR are DLL3-CAR06, DLL3-CAR07, DLL3-CAR08, DLL3-CAR09, DLL3-CAR10, DLL3-CAR11, DLL3-CAR12, DLL3-CAR13, and DLL3-CAR14, respectively.

The chimeric antigen receptors used in this example are second-generation chimeric antigen receptors, wherein the sequences of the CD8α signal peptide, the antigen-binding domain, the CD8α hinge region or CD28 hinge region, the CD8α transmembrane domain or CD28 transmembrane domain, the 4-1BB intracellular co-stimulatory domain or CD28

intracellular co-stimulatory domain, the CD3ζ signaling domain, the cleavage peptide, and the linker $(G4S)_3$ are shown in Table 3.

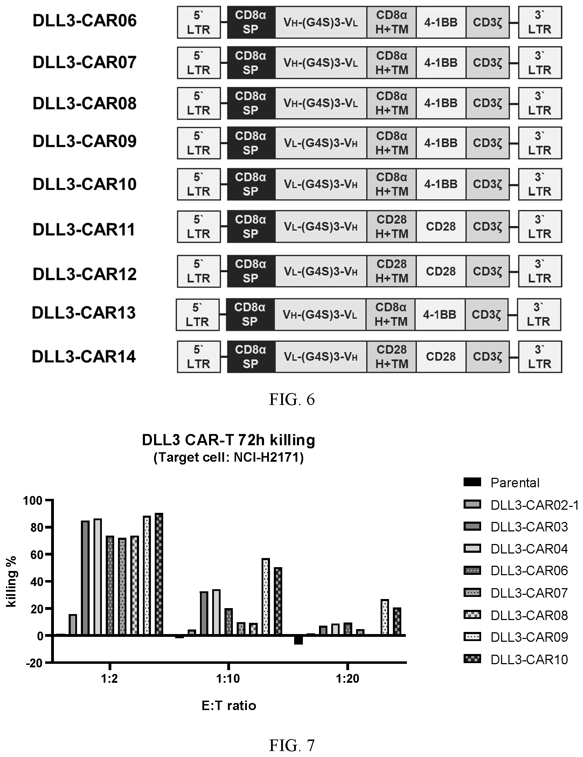

The CAR structures are shown in FIG. 6, and the sequences of DLL3-CAR06, DLL3-CAR07, DLL3-CAR08, DLL3-CAR09, DLL3-CAR10, DLL3-CAR11, DLL3-CAR12, DLL3-CAR13, and DLL3-CAR14 are shown in Table 13.

The antigen-binding domain of DLL3-CAR06 (plasmid number: P-6) is DLL3-scFv-1- VH2aVL1, and the CAR amino acid sequence of DLL3-CAR06 is SEQ ID NO: 92.

The antigen-binding domain of DLL3-CAR07 (plasmid number: P-7) is DLL3-scFv-1- VH5VL1, and the CAR amino acid sequence of DLL3-CAR07 is SEQ ID NO: 93.

The antigen-binding domain of DLL3-CAR08 (plasmid number: P-8) is DLL3-scFv-1- VH5VL3, and the CAR amino acid sequence of DLL3-CAR08 is SEQ ID NO: 94.

The antigen-binding domain of DLL3-CAR09 (plasmid number: P-9) is DLL3-scFv-2-VH1VL1a, and the CAR amino acid sequence of DLL3-CAR09 is SEQ ID NO: 95.

The antigen-binding domain of DLL3-CAR10 (plasmid number: P-10) is DLL3-scFv-2-VH1VL2, and the CAR amino acid sequence of DLL3-CAR10 is SEQ ID NO: 96.

The antigen-binding domain of DLL3-CAR11 (plasmid number: P-11) is DLL3-scFv-2-VH1VL1a, which differs from DLL3-CAR09 in that the 4-1BB co-stimulatory domain is replaced with a CD28 co-stimulatory domain. The CAR amino acid sequence of DLL3-CAR11 is SEQ ID NO: 97.

The antigen-binding domain of DLL3-CAR12 (plasmid number: P-12) is DLL3-scFv-2-VH1VL2, which differs from DLL3-CAR10 in that the 4-1BB co-stimulatory domain is replaced with a CD28 co-stimulatory domain. The CAR amino acid sequence of DLL3-CAR12 is SEQ ID NO: 98.

The antigen-binding domain of DLL3-CAR13 (plasmid number: P-13) is DLL3-scFv-3 -VH2aVL2, and the CAR amino acid sequence of DLL3-CAR13 is SEQ ID NO: 99.

The antigen-binding domain of DLL3-CAR14 (plasmid number: P-14) is DLL3-scFv-3-VH2aVL2, which differs from DLL3-CAR13 in that the 4-1BB co-stimulatory domain is replaced with a CD28 co-stimulatory domain. The CAR amino acid sequence of DLL3-CAR14 is SEQ ID NO: 100.

Table 13. Amino acid sequence listing of second-generation chimeric antigen receptor

| SEQ ID NO: | Plasmid number | Sequence name | Amino acid sequence |
|---|---|---|---|
| 92 | P-6 | DLL3-CAR06 | MALPVTALLLPLALLLHAARPDIQMTQSPSSLSASVGDRVTIT CKASQNVGTAVAWYQQKPGKSPKLLIFSASIRYTGVPSRFSGS GSGTDFTLTISSLQPEDFATYYCQQYSSSPYTFGGGTKVEIKG GGGSGGGGSGGGGSEVQLVQSGAEVKKPGASVKVSCKASG YTFTDYYMNWVRQAPGQRLEWMGIINPYNSGTSYNQKFRG RVTLTVDKSASTAYMELSSLRSEDTAVYYCSRYDYDYDVEGA MDYWGQGTTVTVSSTTTPAPRPPTPAPTIASQPLSLRPEACRP AAGGAVHTRGLDFACDIYIWAPLAGTCGVLLLSLVITLYCKR GRKKLLYIFKQPFMRPVQTTQEEDGCSCRFPEEEEGGCELRV KFSRSADAPAYQQGQNQLYNELNLGRREEYDVLDKRRGRDP EMGGKPRRKNPQEGLYNELQKDKMAEAYSEIGMKGERRRG KGHDGLYQGLSTATKDTYDALHMQALPPR |

(continued)

| SEQ ID NO: | Plasmid number | Sequence name | Amino acid sequence |
|---|---|---|---|
| 93 | P-7 | DLL3-CAR07 | MALPVTALLLPLALLLHAARPDIQMTQSPSSLSASVGDRVTITCKASQNVGTAVAWYQQKPGKSPKLLIFSASIRYTGVPSRFSGSGSGTDFTLTISSLQPEDFATYYCQQYSSSPYTFGGGTKVEIKGGGGSGGGGSGGGGSEVQLVQSGAEVKKPGASVKVSCKASGYTFTDYYMNWVRRAPGQRLEWMGIINPYNGGTSYNQKFRGRVTITVDKSASTAYMELSSLRSEDTAVYYCSRYDYDYDVEGAMDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 94 | P-8 | DLL3-CAR08 | MALPVTALLLPLALLLHAARPDIVMTQSPDSLAVSLGERATINCKASQNVGTAVAWYQQKPGQSPKLLIFSASIRYTGVPDRFSGSGSGTDFTLTISSLQAEDVAVYYCQQYSSSPYTFGGGTKVEIKGGGGSGGGGSGGGGSEVQLVQSGAEVKKPGASVKVSCKASGYTFTDYYMNWVRRAPGQRLEWMGIINPYNGGTSYNQKFRGRVTITVDKSASTAYMELSSLRSEDTAVYYCSRYDYDYDVEGAMDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 95 | P-9 | DLL3-CAR09 | MALPVTALLLPLALLLHAARPDIVMTQSPLSLPVTPGEPASISCRASQTISDYLHWYLQKPGQSPQLLIKYASQSISGVPDRFSGSGSGSDFTLKISRVEAEDVGVYYCQQGHSFPLTFGQGTKLEIKGGGGSGGGGSGGGGSEVQLVQSGAEVKKPGASVKVSCKAS |
| | | | GYTFTSYWMHWVRQAPGQRLEWMGTIYPGNSDTRYNQKFKDRVTITADTSASTAYMELSSLRSEDTAVYYCTRSYDYGDLDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |

(continued)

| SEQ ID NO: | Plasmid number | Sequence name | Amino acid sequence |
|---|---|---|---|
| 96 | P-10 | DLL3-CAR10 | MALPVTALLLPLALLLHAARPDIVMTQSPLSLPVTPGEPASISCRASQTISDYLHWYLQKPGQSPRLLIKYASQSISGVPDRFSGSGSGTDFTLKISRVEAEDVGVYYCQNGHSFPLTFGQGTKLEIKGGGGSGGGGSGGGGSEVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 97 | P-11 | DLL3-CAR11 | MALPVTALLLPLALLLHAARPDIVMTQSPLSLPVTPGEPASISCRASQTISDYLHWYLQKPGQSPQLLIKYASQSISGVPDRFSGSGSGSDFTLKISRVEAEDVGVYYCQQGHSFPLTFGQGTKLEIKGGGGSGGGGSGGGGSEVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSSAAAIEVMYPPPYLDNEKSNGTIIHVKGKHLCPSPLFPGPSKPFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 98 | P-12 | DLL3-CAR12 | MALPVTALLLPLALLLHAARPDIVMTQSPLSLPVTPGEPASISCRASQTISDYLHWYLQKPGQSPRLLIKYASQSISGVPDRFSGSGSGTDFTLKISRVEAEDVGVYYCQNGHSFPLTFGQGTKLEIKGGGGSGGGGSGGGGSEVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSSAAAIEVMYPPPYLDNEKSNGTIIHVKGKHLCPSPLFPGPSKPFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPRRKNPQEGLYNELQKDKMAEAYSEIGMKGERRRGK |
| | | | GHDGLYQGLSTATKDTYDALHMQALPPR |

(continued)

| SEQ ID NO: | Plasmid number | Sequence name | Amino acid sequence |
|---|---|---|---|
| 99 | P-13 | DLL3-CAR13 | MALPVTALLLPLALLLHAARPEIVLTQSPATLSLSPGERATLSCRASKSVSTSGFSYMHWYQQKPGQPPRLLIYLASNLESGIPARFSGSGSGTDFTLTISSLEPEDFAVYYCQHSRELPFTFGQGTKLEIKGGGGSGGGGSGGGGSEVQLVQSGAEVKKPGSSVKVSCKASGYAFSNYWINWVRQAPGQGLEWMGQINPGDGDTIYNSKFKGRVTITADKSTSTAYMELSSLRSEDTAVYYCEREGRNYGDFAYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 100 | P-14 | DLL3-CAR14 | MALPVTALLLPLALLLHAARPEIVLTQSPATLSLSPGERATLSCRASKSVSTSGFSYMHWYQQKPGQPPRLLIYLASNLESGIPARFSGSGSGTDFTLTISSLEPEDFAVYYCQHSRELPFTFGQGTKLEIKGGGGSGGGGSGGGGSEVQLVQSGAEVKKPGSSVKVSCKASGYAFSNYWINWVRQAPGQGLEWMGQINPGDGDTIYNSKFKGRVTITADKSTSTAYMELSSLRSEDTAVYYCEREGRNYGDFAYWGQGTTVTVSSAAAIEVMYPPPYLDNEKSNGTIIHVKGKHLCPSPLFPGPSKPFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |

Referring to the CAR structures in FIG. 6, the corresponding shuttle plasmids were prepared according to the methods in Examples 2 and 3, and subjected to virus packaging, T cell *in vitro* expansion, and virus infection.

**Example 6**: ***In vitro* Specific Tumor Cell Killing Function Assessment of DLL3 Humanized CAR-T Cells**

**1. *In vitro* specific tumor cell killing function assessment of CAR-T cells using 4-1BB costimulatory domain**

To verify the *in vitro* killing toxicity of DLL3 CAR-T cells prepared in Example 5, *In vitro* tumor killing function assessment was performed using the tumor cell line NCI-H2171 (purchased from ATCC) expressing human DLL3 using a method similar to that in Example 4.

*In vitro* killing experiments were carried out using transfected CAR-T. The killing results are shown in FIG. 7. The killing effect of DLL3-CAR06, DLL3-CAR07, and DLL3-CAR08 constructed by 3 groups of scFvs from the humanization of DLL3-scFv-1 on the cell line NCI-H2171 with a low expression was not as good as that of DLL3-CAR03 constructed by a murine antigen-binding domain DLL3-scFv-1;

The killing effect of DLL3-CAR09 and DLL3-CAR10 constructed by 2 groups of scFvs from the humanization of DLL3-scFv-2 on the cell line NCI-H2171 with a low expression was both better than that of DLL3-CAR04 constructed by a murine antigen-binding domain DLL3-scFv-2.

However, whether humanized or murine antigen-binding domains DLL3-scFv-1 and DLL3-scFv-2 were used, the killing effect of the constructed CAR-T cells on the cell line NCI-H2171 with a low expression was both better than that of the positive control DLL3-CAR02-1.

### 2. *In vitro* specific tumor cell killing function assessment of CAR-T cells using CD28 costimulatory domain

Considering the possible effects of different vectors, the 4-1BB co-stimulatory domain of DLL3-CAR09 was replaced by CD28 co-stimulatory domain to construct DLL3-CAR11; the 4-1BB costimulatory domain of DLL3-CAR10 was replaced by CD28 co-stimulatory domain to construct DLL3-CAR12. The structures of DLL3-CAR11 and DLL3-CAR12 are shown in FIG. 6, and the preparation methods are described in Examples 2 and 3.

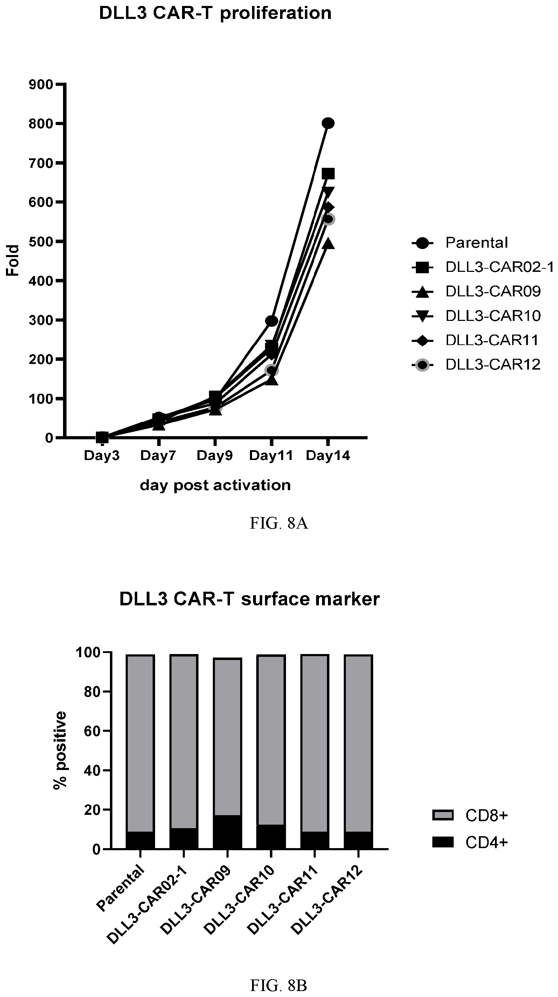

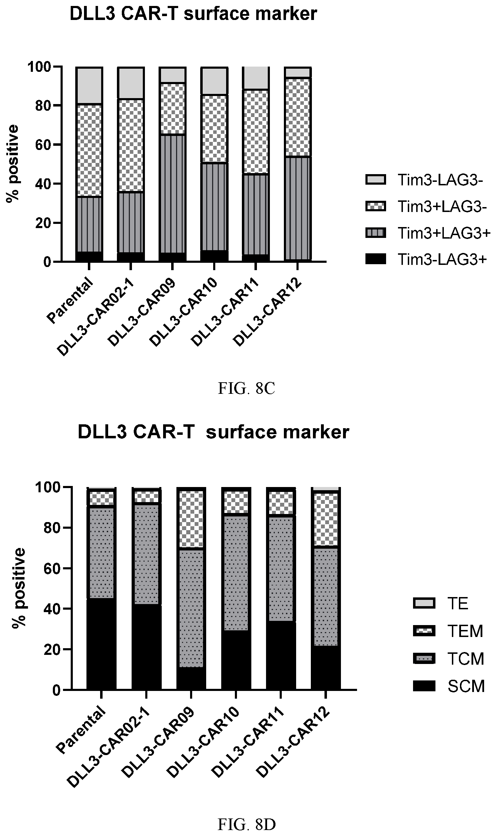

T cell characteristics of the positive reference DLL3-CAR02-1 and DLL3-CAR09, DLL3-CAR10, DLL3-CAR11, and DLL3-CAR12 after *in vitro* expansion were assessed using the method of Example 3. As shown in FIG. 8A, CAR-T cells with different vector structures had good expansion efficiency. The CAR-T cell phenotypes on day 15 of T cell activation is shown in FIGs. 8B, 8C, and 8D, with the $CD4^+$ T cell content of DLL3-CAR09 and DLL3-CAR10 groups being slightly higher than that of the other groups; for the memory phenotype, DLL3-CAR09 group had the lowest proportion of SCM cells, and TEM and TCM phenotypes of CAR-T with a transfected CAR structure were both higher than those of the untransfected T cells; for the depletion phenotype, the depletion phenotype of DLL3-CAR09 and DLL3-CAR12 groups was slightly higher than that of the other groups.

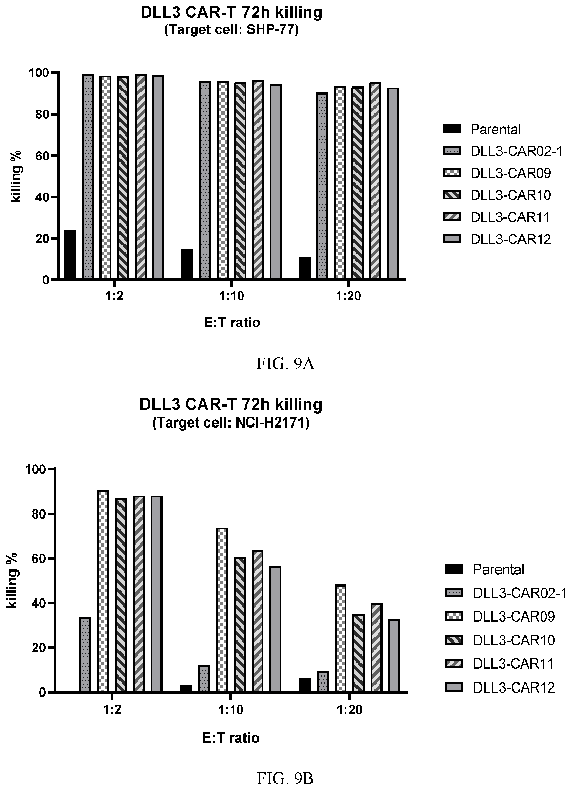

The killing effect of the positive reference DLL3-CAR02-1 and DLL3-CAR09, DLL3-CAR10, DLL3-CAR11, and DLL3-CAR12 on the cell line SHP-77 with a high expression and the cell line NCI-H2171 with a low expression was assessed using the method of Example 4. The results of 72 h *in vitro* killing experiments are shown in FIGs. 9A and 9B. The killing ability of CAR-T cells with different vector structures and the positive reference on the cell line SHP-77 highly expressing DLL3 was equivalent (FIG. 9A); the killing effect of DLL3-CAR09 to DLL3-CAR12 was all stronger than that of the positive control DLL3-CAR02-1 on the cell line NCI-H2171 with a low expression, wherein DLL3-CAR09 and the vector structure DLL3-CAR11 with the CD28 co-stimulatory domain replacement both showed stronger killing effect than that of DLL3-CAR10 and DLL3-CAR12 (FIG. 9B).

The killing effect of the positive reference DLL3-CAR01 and DLL3-CAR02-1, and DLL3-CAR09 and DLL3-CAR11 using DLL3-scFv-2-VH1VL1a as the antigen-binding domain on the cell line SHP-77 with a high expression, the cell line NCI-H82 with a medium expression, and the cell line NCI-H2171 with a low expression was assessed using the method of Example 4. The *in vitro* killing results are shown in FIG. 10. In FIGs. 10A-10C, the killing results of DLL3-CAR09 and DLL3-CAR11 on three cells for 72 h were consistent with the previous results, i.e., on cells with high and medium expression of DLL3, the killing effect was similar to the effect of the positive control, but on cells with a low expression of DLL3, the killing effect of both DLL3-CAR09 and DLL3-CAR11 was stronger than that of the positive control. 72-h T cells were collected simultaneously with the 72-h killing plating assay: the cell culture supernatant at a T cell:target cell ratio of 1:2 was subjected to Human IFN-γ ELISA (BD, Cat#555142). The assay results are shown in FIG. 11. On three cells expressing DLL3, IFN-γ secreted by DLL3-CAR09 and DLL3-CAR11 were significantly higher than that secreted by the positive reference DLL3-CAR01 and DLL3-CAR02-1.

### 3. *In vitro* specific tumor cell killing function assessment of CAR-T cells using DLL3-scFv-3-VH2aVL2 as antigen-binding domain

To verify 1 group of scFv humanized from the newly obtained murine DLL3-scFv-3 (DLL3-CAR05), the *in vitro* killing functions of DLL3-CAR13 with the 4-1BB co-stimulatory domain and DLL3-CAR14 with the CD28 co-stimulatory domain (as shown in FIG. 6) were constructed.

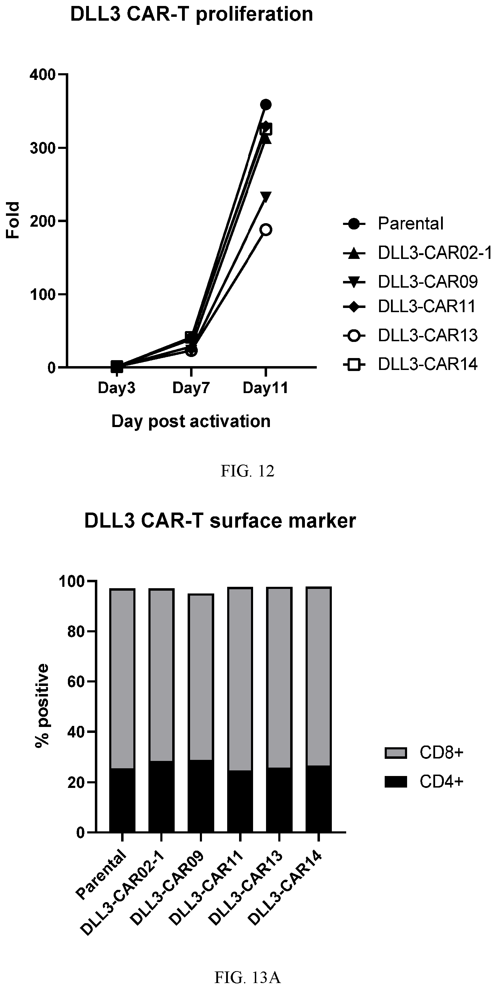

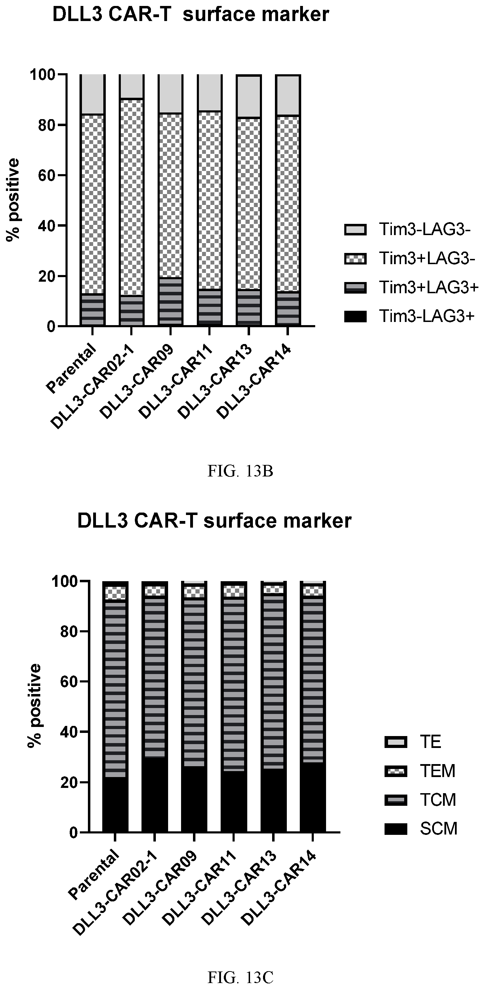

T cell characteristics of the positive reference DLL3-CAR02-1 and DLL3-CAR09, DLL3-CAR11, DLL3-CAR13, and DLL3-CAR14 after *in vitro* expansion were assessed using the method of Example 3. In terms of cell expansion, the expansion efficiency of DLL3-CAR13 and DLL3-CAR14 was good, and DLL3-CAR13 proliferation was significantly weaker than that of DLL3-CAR14, as shown in FIG. 12. CAR-T cell phenotype on day 10 of T cell activation is shown in FIGs. 13A, 13B, and 13C. The $CD4^+$ T cell content of DLL3-CAR13 group and DLL3-CAR14 group was between DLL3-CAR09 group and DLL3-CAR11 group; for the memory phenotype, the SCM content of DLL3-CAR14 group was the highest; for the depletion phenotype, DLL3-CAR13 group and DLL3-CAR14 group were similar to DLL3-CAR11 group.

The *in vitro* killing effect of the positive reference DLL3-CAR02-1 and DLL3-CAR09, DLL3-CAR11, DLL3-CAR13, and DLL3-CAR14 on the tumor cell line with a low expression of DLL3 was assessed using the method of Example 4. The killing effect of the T cells is shown in FIG. 14. On the cell line NCI-H2171 with a low expression, the structures of the 4 groups did not show a significant difference, and the killing effect was all significantly better than that of the positive control DLL3-CAR02-1.

## Example 7: Assay of Anti-Tumor Efficacy of DLL3 CAR-T Cells on Mouse Subcutaneous Tumor Model

### 1. Assay of anti-tumor efficacy of DLL3 CAR-T cells on SHP-77 mouse subcutaneous tumor model

The anti-tumor effect of DLL3 CAR-T cells was evaluated using the mouse subcutaneous tumor CDX model. The

procedures are as follows:
NPG mice (combined immunodeficient mice, purchased from Beijing Vitalstar Biotechnology Co., Ltd.) were inoculated subcutaneously with $5 \times 10^6$ SHP-77 cells in logarithmic growth phase and in good growth condition.

The long and short diameters a and b of the tumor of the mice were measured on the 3rd day after inoculation. The tumor volume of the mice V ($mm^3$) = $1/2 \times (a \times b^2)$ was calculated, and the mice with the tumor volume around 50 $mm^3$ were randomly grouped according to the random number principle. On the 4th day after tumor inoculation, CAR-T cells ($8 \times 10^6$ CAR+ cells/mouse) were injected via the tail vein. The day of CAR-T cell injection was recorded as day 0 (D0), single administration. The specific grouping and administration are shown in Table 14.

Table 14. Grouping of SHP-77 model *in vivo* anti-tumor experiment

| Group | Number of animals | Therapeutic | Dose | Route of administration | Time of administration |
|---|---|---|---|---|---|
| G1 | 5 | PBS | 200μL PBS | i.v. | Day0 |
| G2 | 5 | DLL3-CAR02-1 | 8e6 CAR+/200μL | i.v. | Day0 |
| G3 | 5 | DLL3-CAR09 | 8e6 CAR+/200μL | i.v. | Day0 |
| G4 | 5 | DLL3-CAR11 | 8e6 CAR+/200μL | i.v. | Day0 |

Tumor volume was continuously observed and measured 2 times weekly, and 70 μL of blood was taken each time to identify the CAR-T cell expansion in peripheral blood of the mice.

The whole blood taken out was added into 1× red blood cell lysing buffer (Invitrogen, Cat#00-4300-54) to lyse red blood cells and well mixed. The mixture was placed at room temperature for 10 min, and centrifuged at 400 g for 5 min. The supernatant was removed. The mixture was resuspended and washed once by adding 1 mL of PBS, and centrifuged at 400 g for 5 min. The supernatant was removed. Blocking was performed for 10 min by adding 2 μL of anti-mouse FCX (Biolegend, Cat#101320) and 2 μL of anti-human FCX (Biolegend, Cat#422302). A prepared surface antibody was added, and the mixture was incubated at room temperature for 20-30 min in the dark. After incubation, 1 mL of PBS was added for washing for 1-2 times, and the mixture was centrifuged at 400 g for 5 min. The supernatant was removed. The mixture was resuspended in 200 μL of PBS, filtered, transferred to a 96-well U-bottom plate, and stored at 4 °C in the dark for later assay.

The results of the tumor volume measurement in mice are shown in FIG. 15A, the weight change is shown in FIG. 15B, and the survival curve is shown in FIG. 15C: the PBS control group was euthanized on day 23 due to tumor volume exceeding 2000 $mm^3$; the weight status of the animals in the treatment groups on day 40 was observed to be normal; for the tumor of the animals in the CAR-T DLL3-CAR11 group, a clearance effect was observed on day 6, and the tumor clearance rate of the group was 5/5 on day 14, which showed the fastest efficacy; for the tumor of the animals in the CAR-T DLL3-CAR09 group, a clearance effect was observed on day 9, and the tumor clearance rate of the group was 5/5 on day 17; for the tumor of the animals in the positive reference DLL3-CAR02-1 group, a clearance effect was observed on day 14, and the tumor clearance rate of the group was 3/5 on day 40, which showed slightly poor efficacy.

The results of peripheral blood T cell assay in mice are shown in FIG. 15D, and DLL3 CAR-T cells were all expanded to a certain level for tumor clearance after injection *in vivo*, with slightly earlier T cell expansion time for DLL3-CAR11 group and highest T cell expansion level for DLL3-CAR09 group.

DLL3-CAR09 and DLL3-CAR11 achieved the treatment effect of complete tumor clearance on subcutaneous tumors of the tumor cell line SHP-77 with a high expression of DLL3, wherein DLL3-CAR11 showed the fastest efficacy, and DLL3-CAR09 showed the second. Also, the CAR-T treatment was good in safety, did not have dramatical expansion and did not cause death of animals, as can be seen from the weight state of the animals and the expansion condition of T cells in peripheral blood.

**2. Assay of anti-tumor efficacy of DLL3 CAR-T cells on NCI-H2171 mouse subcutaneous tumor model**

The anti-tumor effect of DLL3 CAR-T cells was evaluated using the mouse subcutaneous tumor CDX model: NPG mice (combined immunodeficient mice, purchased from Beijing Vitalstar Biotechnology Co., Ltd.) were inoculated subcutaneously with $3 \times 10^6$ NCI-H2171 cells in logarithmic growth phase and in good growth condition.

The mice with the tumor volume around 30 $mm^3$ were randomly grouped according to the random number principle. On the 3rd day after tumor inoculation, CAR-T cells ($1.5 \times 10^7$ CAR+ cells/mouse) were injected via the tail vein.

The day of CAR-T cell injection was recorded as day 0 (D0), single administration. The specific grouping and administration are shown in Table 15.

Table 15. Grouping of NCI-2171 model *in vivo* anti-tumor experiment

| Group | Number of animals | Therapeutic | Dose | Route of administration | Time of administration |
|---|---|---|---|---|---|
| G1 | 5 | PBS | 300μL PBS | i.v. | Day0 |
| G2 | 5 | DLL3-CAR02-1 | 1.5e7 CAR+/300μL | i.v. | Day0 |
| G3 | 5 | DLL3-CAR09 | 1.5e7 CAR+/300μL | i.v. | Day0 |
| G4 | 5 | DLL3-CAR11 | 1.5e7 CAR+/300μL | i.v. | Day0 |
| G5 | 5 | DLL3-CAR13 | 1.5e7 CAR+/300μL | i.v. | Day0 |
| G6 | 5 | DLL3-CAR14 | 1.5e7 CAR+/300μL | i.v. | Day0 |

The results of the tumor volume measurement in mice are shown in FIG. 15E, and the weight change is shown in FIG. 15F: 4 animals in PBS and the positive reference DLL3-CAR02-1 groups were euthanized on day 22 due to tumor mean exceeding 2000 $mm^3$, and the tumor volume of 1 remaining mouse reached euthanization criteria on D33;

The observation was continued until day 33, and animals in DLL3-CAR09 group, DLL3-CAR11 group, DLL3-CAR13 group, and DLL3-CAR14 group were in normal weight status.

For the tumor volume growth condition: no tumor inhibiting or eliminating effect was observed in the positive reference DLL3-CAR02-1 group after CAR-T injection; both DLL3-CAR09 and DLL3-CAR11 group CAR-T cleared tumors around 7 days after treatment, in which DLL3-CAR09 group continued to be in tumor clearance state, and tumors of DLL3-CAR11 group recurred one after another after day 21; DLL3-CAR14 group CAR-T cleared tumors around 11 days after treatment, which recurred one after another after day 18; 4 animals in DLL3-CAR13 group showed tumor clearance around 11 days after treatment and continued to be in a clearance state, and 1 animal showed continuous tumor growth due to individual differences; DLL3-CAR09 group had the best treatment effect with a survival curve as shown in FIG. 15G.

Also, the high-dose DLL3 CAR-T treatment was good in safety, did not have dramatical expansion and did not cause death of animals, as can be seen from the weight state of the animals and the expansion of T cells in peripheral blood. On day 5, the number of T cells in DLL3-CAR09 group was the highest; on day 19, T cells in peripheral blood of all groups were near or below the limit of detection, as shown in FIG. 15H.

**Example 8: Preparation and Identification of Secretable CD70 Armored DLL3 CAR-T Cells**

**1. Plasmid construction and preparation of CAR-T cells**

Referring to FIG. 16, a retroviral shuttle plasmid that expresses the second-generation chimeric antigen receptor of DLL3 and secretable CD70 (sCD70) was constructed: P15, and the number corresponding to CAR is DLL3-CAR15. The chimeric antigen receptor used by DLL3-CAR15 is a second-generation chimeric antigen receptor, wherein the sequences of the CD8α signal peptide, the CD8α hinge region, the CD8α transmembrane domain, the 4-1BB intracellular co-stimulatory domain, the CD3ζ signaling domain, and the linker (G4S)3 are shown in Table 3;

the sequence of P2A is ATNFSLLKQAGDVEENPGP (SEQ ID NO: 106);
the sequence of the sCD70 is as follows:

QRFAQAQQQLPLESLGWDVAELQLNHTGPQQDPRLYWQGGPALGRSFLHGPELDKGQLRIH RDGIYMVHIQVTLAICSSTTASRHHPTTLAVGICSPASRSISLLRLSFHQGCTIASQRLTPLARG DTLCTNLTGTLLPSRNTDETFFGVQWVRP (SEQ ID NO: 102).

The antigen-binding domain of DLL3-CAR15 (plasmid number: P-15) is DLL3-scFv-2-VH1VL1a, and the CAR

sequence of DLL3-CAR15 is as follows:

MALPVTALLLPLALLLHAARPDIVMTQSPLSLPVTPGEPASISCRASQTISDYLHWYLQKPGQSPQLLIKYASQSISGVPDRFSGSGSGSDFTLKISRVEAEDVGVYYCQQGHSFPLTFGQGTKLEIKGGGGSGGGGSGGGGSEVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPRKRRSGSGATNFSLLKQAGDVEENPGPMALPVTALLLPLALLLHAARPQRFAQAQQQLPLESLGWDVAELQLNHTGPQQDPRLYWQGGPALGRSFLHGPELDKGQLRIHRDGIYMVHIQVTLAICSSTTASRH

HPTTLAVGICSPASRSISLLRLSFHQGCTIASQRLTPLARGDTLCTNLTGTLLPSRNTDETFFGVQWVRP (SEQ ID NO: 103)

Referring to the CAR structures in FIG. 16, the corresponding shuttle plasmids P15 were prepared according to the methods in Examples 2 and 3, and subjected to virus packaging, T cell *in vitro* expansion, and virus infection to prepare the secretable CD70 armored DLL3 CAR-T cell DLL3-CAR15. The DLL3-CAR15 has an additional armor of soluble CD70 (SEQ ID NO: 102) on the basis of DLL3-CAR09.

**2. FACS detection of CAR transfection efficiency and T cell phenotype**

T cell characteristics of DLL3-CAR09 and DLL3-CAR15 after *in vitro* expansion were assessed using the method of Example 3.

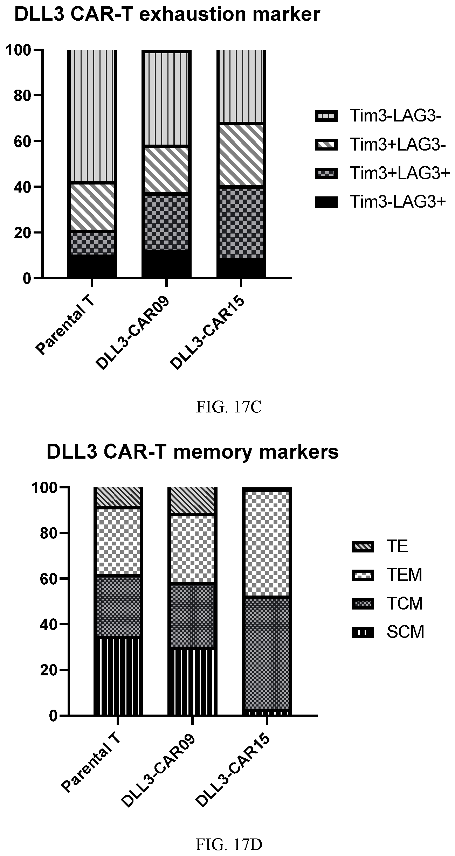

The expression levels of DLL3 CAR by T cells after viral transfection and one week of *in vitro* expansion are shown in FIG. 17A. The CAR expression levels of DLL3-CAR09 and DLL3-CAR15 were 69% and 91%, respectively. Flow cytometry results for T cell phenotype are shown in FIGs. 17B, 17C, and 17D. The CD4+ T cell content of DLL3-CAR15 group was slightly higher than that of DLL3-CAR09 and untransfected T cells (FIG. 17B); the TIM3+ LAG3+ double positive depletion phenotype of both DLL3-CAR09 and DLL3-CAR15 groups was slightly higher than that of the untransfected T cell group (FIG. 17C); the TCM and TEM of DLL3-CAR15 group were significantly higher than those of the other groups (FIG. 17D).

**3. Assay on level of CD70 protein secreted by CAR-T cells by ELISA**

During *in vitro* expansion culture, culture supernatants were collected to assay the level of CD70 protein secreted by CAR-T cells. A culture supernatant (cumulative culture for 3 days) whose CAR-T cell density was maintained at about 2E6 was centrifuged, the cell debris was removed, and assay was performed using Human CD70 ELISA kit (Abcam, ab264621). The results are shown in FIG. 17E. Only the culture supernatant of DLL3-CAR15 group was detected an amount of CD70 protein secreted at a level of 3.7 ng/mL. The above results demonstrate that secretable CD70 armored DLL3 CAR-T cells can normally express the DLL3 CAR structure and secrete CD70 protein, and the armor structure may increase the activation degree of DLL3 CAR-T cells during expansion, thereby significantly increasing the proportions of central memory T cells and effector memory T cells.

**Example 9: *In Vitro* Tumor Killing Function Assessment of Secretable CD70 Armored DLL3 CAR-T Cells**

**1. *In vitro* specific tumor cell killing function assessment of secretable CD70 armored DLL3 CAR-T**

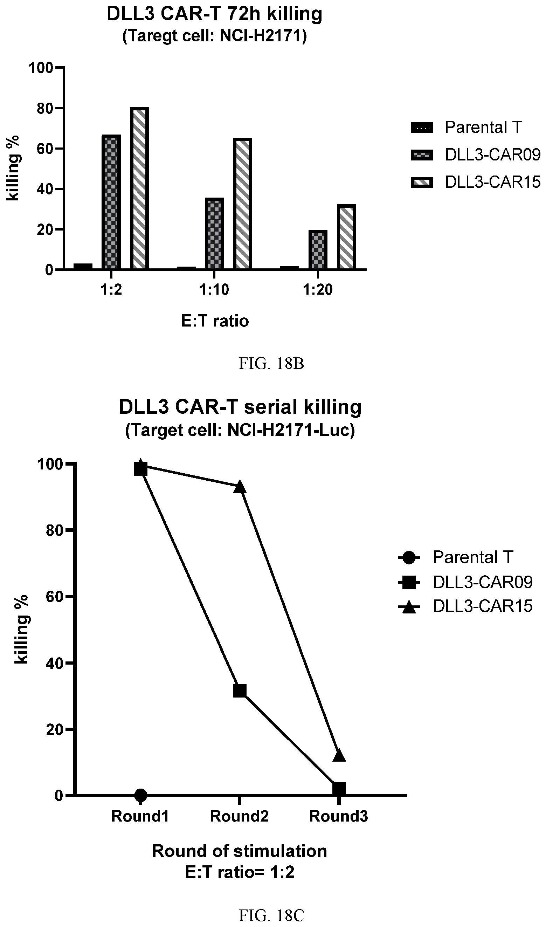

The killing effect of DLL3-CAR09 and DLL3-CAR15 on the cell line SHP-77 with a high expression of DLL3 and the cell line NCI-H2171 with a low expression was assessed using the method of Example 4. As shown in FIGs. 18A and 18B, CAR-T cells with the secretable CD70 armored DLL3-CAR15-structure were significantly more effective than the DLL3-CAR09 structure in killing SHP-77 target cells and NCI-H2171 target cells at different ET ratios during 72-h killing.

**2. *In vitro* specific tumor cell long-lasting killing function assessment of secretable CD70 armored DLL3 CAR-T**

To verify the specific long-lasting killing toxicity of secretable CD70 armored DLL3 CAR-T cells, a continuous *in vitro* killing experiment was performed using a self-constructed NCI-H2171 stably transfected cell strain NCI-H2171-luc highly expressing human bacterial luciferase. The NCI-H2171-luc cells were transformed with luciferase reporter gene by conventional gene manipulation methods. Specifically, NCI-H2171-luc cells were plated in a 24-well plate at 4E5 cells per well; T cells with different CAR structures and untransfected T cells were added according to E:T = 1:2; after 72 h of co-culture, 200 μL of the cell mixture was taken from each well and added to a blank 96-well plate, firefly luciferase substrate D-Luciferin (Abcam, Cat#ab143655) was added, and biofluorescence reading and result analysis were carried out using a PE EnSight microplate reader. Meanwhile, the remaining cells in each group were counted for viable cells, and the number of T cells in the remaining mixture was calculated based on the number of viable cells and the killing result. 2E5 CAR-T cells were replated and 4E5 fresh NCI-H2171-luc target cells were added per well for a second round of killing experiments. By analogy, 3 rounds of killing were completed.

The results are shown in FIG. 18C. The secretable CD70 armor structure effectively improved the killing ability of DLL3 CAR-T cells on NCI-H2171-luc target cells, and significantly extended the killing durability to the third round. The above results indicate that CD70 armor can further improve the killing function of the DLL3-CAR09 structure on SCLC tumor cell lines with different expression levels of DLL3, expand the use window of DLL3 CAR-T cells in clinical application and possibly reduce the amount to ensure the efficacy of treatment and improve the safety of cell treatment.

**Example 10: Assay of Anti-Tumor Efficacy of Secretable CD70 Armored DLL3 CAR-T Cells on Mouse Subcutaneous Tumor Model**

**1. Assay of anti-tumor efficacy of secretable CD70 armored DLL3 CAR-T cells on SHP-77 mouse subcutaneous tumor model under low-dose condition**

The anti-tumor effect of DLL3 CAR-T cells was evaluated using the mouse subcutaneous tumor CDX model. The procedures are as follows:
NPG mice (combined immunodeficient mice, purchased from Beijing Vitalstar Biotechnology Co., Ltd.) were inoculated subcutaneously with $3 \times 10^6$ SHP-77 cells in logarithmic growth phase and in good growth condition.

The long and short diameters a and b of the tumor of the mice were measured on the 3rd day after inoculation. The tumor volume of the mice V ($mm^3$) = $1/2 \times (a \times b^2)$ was calculated, and the mice with the tumor volume around 45 $mm^3$ were randomly grouped according to the random number principle. On the 10th day after tumor inoculation, CAR-T cells ($4 \times 10^6$ CAR+ cells/mouse) were injected at a low dose via the tail vein. The day of CAR-T cell injection was recorded as day 0 (D0), single administration. The specific grouping and administration are shown in Table 16.

Table 16. Grouping of SHP-77 model *in vivo* anti-tumor experiment

| Group | Number of animals | Therapeutic | Dose | Route of administration | Time of administration |
|---|---|---|---|---|---|
| G1 | 5 | PBS | PBS/200μL | i.v. | Day0 |
| G2 | 5 | DLL3-CAR09 | 4e6 CAR+/200μL | i.v. | Day0 |
| G3 | 5 | DLL3-CAR15 | 4e6 CAR+/200μL | i.v. | Day0 |

Tumor volume was continuously observed and measured 2 times weekly, and 70 μL of blood was taken each time to identify the CAR-T cell expansion in peripheral blood of the mice.

The results of the tumor volume measurement in mice are shown in FIG. 19A, and the weight change is shown in FIG. 19B: the PBS control group was euthanized on day 26 due to tumor volume exceeding 2000 $mm^3$; the weight status of the animals in the PBS control group was slightly reduced, the animal status was not good probably due to tumor progression, and the weight of the animals in other treatment groups was normal; CAR-T DLL3-CAR09 group did not exhibit significant efficacy, and the tumor growth speed was similar to that of the PBS control group; tumors in animals of the CAR-T DLL3-CAR15 group exhibited significant tumor growth inhibition efficacy on day 15.

The results of peripheral blood T cell assay in mice are shown in FIG. 19C. No significant T cell expansion was detected in peripheral blood of animals in DLL3-CAR09 group; T cell expansion was detected in DLL3-CAR15 group on

day 15, coinciding with the time of tumor inhibition efficacy.

In Example 7, DLL3-CAR09 showed efficacy for tumor clearance at a high dose (8E6 CAR+/200 μL/mouse), and to test the efficacy advantage of secretable CD70 armor over the non-armor structure, a low dose was used in this example. At a low dose of 4E6CAR+/200 μL/mouse, DLL3-CAR09 failed to exhibit significant therapeutic efficacy. In contrast, a significant T cell expansion was observed in the DLL3-CAR15 group armored with secretable CD70, and the corresponding tumor-inhibiting efficacy was observed.

This results demonstrate that secretable CD70 armor structure can better improve the activity and persistence of DLL3 CAR-T cells *in vivo* and ensure that the efficacy is still exhibited at low doses. The CAR-T cell therapy needs to be carried out by adopting the autologous T cells of the patient, and the activity difference of the autologous T cells of the patient is large. The secretable CD70 armor can make up the defect of poor activity of the autologous DLL3 CAR-T cells by secreting CD70 protein, which ensures the durability *in vivo* and improves the possibility of exerting the efficacy.

**2. Assay of anti-tumor efficacy of DLL3 CAR-T cells on NCI-H2171 mouse subcutaneous tumor model under medium-dose condition**

The anti-tumor effect of DLL3 CAR-T cells was evaluated using the mouse subcutaneous tumor CDX model: NPG mice (combined immunodeficient mice, purchased from Beijing Vitalstar Biotechnology Co., Ltd.) were inoculated subcutaneously with $3 \times 10^6$ NCI-H2171 cells in logarithmic growth phase and in good growth condition.

The mice with the tumor volume around 30 $mm^3$ were randomly grouped according to the random number principle. On the 10th day after tumor inoculation, CAR-T cells ($8 \times 10^6$ CAR+ cells/mouse) were injected via the tail vein. The day of CAR-T cell injection was recorded as day 0 (D0), single administration. The specific grouping and administration are shown in Table 17.

Table 17. Grouping of NCI-2171 model *in vivo* anti-tumor experiment

| Group | Number of animals | Therapeutic | Dose | Route of administration | Time of administration |
|---|---|---|---|---|---|
| G1 | 5 | PBS | PBS/200μL | i.v. | Day0 |
| G2 | 5 | DLL3-CAR09 | 8e6 CAR+/200μL | i.v. | Day0 |
| G3 | 5 | DLL3-CAR15 | 8e6 CAR+/200μL | i.v. | Day0 |

The results of the tumor volume measurement in mice are shown in FIG. 19D, the weight change is shown in FIG. 19E, and the tumor growth inhibition is shown in FIG. 19F: animals in PBS and DLL3-CAR09 groups were euthanized on day 17 due to tumor mean exceeding 2000 $mm^3$. The weight status of each group of animals was normal.

No T cell expansion was detected in peripheral blood due to the low dose used. NCI-H2171 tumor cells exhibited a low expression of DLL3, fast tumor growth speed, and high treatment difficulty. In the medium-dose CAR-T cell treatment plan, DLL3-CAR09 did not exert the efficacy, and a significant decrease in tumor growth speed was observed in all 5 animals in DLL3-CAR15 treatment group, exhibiting 41% tumor inhibition efficiency compared with the PBS control group.

**Example 11: Preparation and Comparison of Secretable CD70 Armored and Membrane-bound CD70 Armored DLL3 CAR-T Cells**

**1. Plasmid construction and preparation of CAR-T cells**

Referring to FIG. 20, a retroviral shuttle plasmid that expresses the second-generation chimeric antigen receptor of DLL3 and membrane-bound CD70 (mCD70) were constructed: P-16, and the number corresponding to CAR is DLL3-CAR16. The chimeric antigen receptor used by DLL3-CAR16 is a second-generation chimeric antigen receptor, wherein the sequences of the CD8α signal peptide, the CD8α hinge region, the CD8α transmembrane domain, the 4-1BB intracellular co-stimulatory domain, the CD3ζ signaling domain, and the linker (G4S)3 are shown in Table 3;

the sequence of P2A is ATNFSLLKQAGDVEENPGP (SEQ ID NO: 106);
the sequence of the mCD70 is as follows:

MPEEGSGCSVRRRPYGCVLRAALVPLVAGLVICLVVCIQRFAQAQQQLPLESLGWDVAELQLNHTGPQQDPRLYWQGGPALGRSFLHGPELDKGQLRIHRDGIYMVHIQVTLAICSSTTASRHHPTTLAVGICSPASRSISLLRLSFHQGCTIASQRLTPLARGDTLCTNLTGTLLPSRNTDETFFGVQWVRP (SEQ ID NO: 104).

The antigen-binding domain of DLL3-CAR16 is DLL3-scFv-2-VH1VL1a, and the CAR sequence of DLL3-CAR16 is as follows:

MALPVTALLLPLALLLHAARPDIVMTQSPLSLPVTPGEPASISCRASQTISDYLHWYLQKPGQSPQLLIKYASQSISGVPDRFSGSGSGSDFTLKISRVEAEDVGVYYCQQGHSFPLTFGQGTKLEIKGGGGSGGGGSGGGGSEVQLVQSGAEVKKPGASVKVSCKASGYTFTSYWMHWVRQAPGQRLEWMGTIYPGNSDTRYNQKFKDRVTITADTSASTAYMELSSLRSEDTAVYYCTRSYDYGDLDYWGQGTTVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPRKRRSGSGATNFSLLKQAGDVEENPGPMPEEGSGCSVRRRPYGCVLRAALVPLVAGLVICLVVCIQRFAQAQQQLPLESLGWDVAELQLNHTGPQQDPRLYWQGGPALGRSFLHGPELDKGQLRIHRDGIYMVHIQVTLAICSSTTASRHHPTTLAVGICSPASRSISLLRLSFHQGCTIASQRLTPLARGDTLCTNLTGTLLPSRNTDETFFGVQWVRP (SEQ ID NO: 105)

Referring to the CAR structure in FIG. 20, the corresponding shuttle plasmids P09, P15, and P16 were prepared according to the methods in Examples 2 and 3, and subjected to virus packaging, T cell *in vitro* expansion, and virus infection to prepare non-armor DLL3 CAR-T cell DLL3-CAR09, secretable CD70 armored DLL3 CAR-T cell DLL3-CAR15, and membrane-bound CD70 armored DLL3 CAR-T cell DLL3-CAR16, wherein DLL3-CAR16 has an additional armor of membrane-bound CD70 (SEQ ID NO: 104) on the basis of DLL3-CAR09.

**2. FACS detection of CAR transfection efficiency and T cell phenotype**

T cell characteristics of DLL3-CAR09, DLL3-CAR15, and DLL3-CAR16 after *in vitro* expansion were assessed using the method of Example 3.

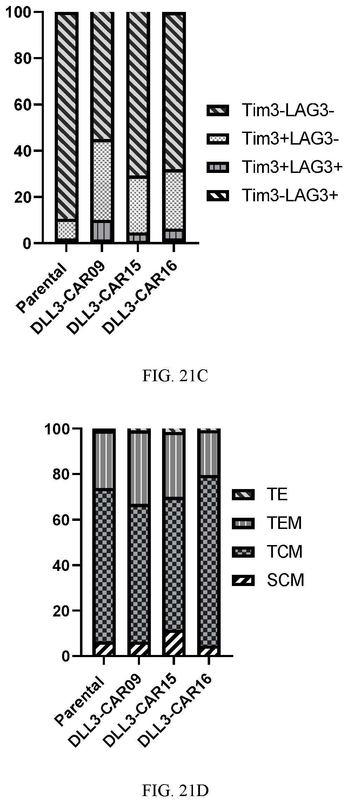

The expression levels of DLL3 CAR by T cells after viral transfection and one week of *in vitro* expansion are shown in FIG. 21A. The CAR expression levels of DLL3-CAR09, DLL3-CAR15, and DLL3-CAR16 were 74%, 64%, and 71%, respectively. Flow cytometry results for T cell phenotype are shown in FIGs. 21B, 21C, and 21D. The CD4+ T cell content of DLL3-CAR15 group was similar to that of DLL3-CAR09 and untransfected T cells, and the CD4+ T cell content of DLL3-CAR16 group was significantly higher than that of the other groups (FIG. 21B); the TIM3+ LAG3+ double positive depletion phenotype of both DLL3-CAR09 and DLL3-CAR15 groups was higher than that of the untransfected T cell group, with the highest depletion proportion in DLL3-CAR09 group (FIG. 21C); the T cell memory phenotype of each group was not significantly changed compared with the untransfected group (FIG. 21D).

**3. Assay on level of CD70 protein secreted by CAR-T cells by ELISA**

During *in vitro* expansion culture, culture supernatants were collected to assay the level of CD70 protein secreted by CAR-T cells. A culture supernatant (cumulative culture for 5 days) whose CAR-T cell density was maintained at about 2E6 was centrifuged, the cell debris was removed, and assay was performed using Human CD70 ELISA kit (Abcam, ab264621). The results are shown in FIG. 21E. A CD70 protein secretion amount at a level of 22 ng/mL was detected in the culture supernatant of DLL3-CAR15 group, and a CD70 protein secretion amount of 4.3 ng/mL was detected at a level in the culture of DLL3-CAR16 group. The above results demonstrate that secretable CD70 armored DLL3 CAR-T cells can normally express the DLL3 CAR structure and secrete CD70 protein, which were significantly higher than that of non-armor and membrane-bound armored CAR-T cells. The expression detection results of T cell surface CD70 and CD27 are

shown in FIG. 21F. The cell membrane surface CD70 expression of DLL3-CAR16 group was 80%, which was significantly higher than that of non-armor (17%) and CD70-secretable (29%) groups, and correspondingly, the membrane surface CD27 expression of DLL3-CAR16 group was 28%, which was significantly lower than that of non-transfected and other CAR-T groups (90% or more). The above results demonstrate that membrane-bound armored DLL3 CAR-T cells can normally express CD70 in the membrane-bound form, while too strong CD70 and CD27 binding results in shedding of CD27 from the T cell surface, affecting the activation extent of CAR-T cells.

**4. *In vitro* killing experiment of secretable CD70 armored and membrane-bound CD70 armored DLL3 CAR-T Cells**

The killing effect of each group of CAR-T cells on the cell line SHP-77 with a high expression and the cell line NCI-H2171 with a low expression was assessed using the method of Example 4. As shown in FIGs. 21G and 21H, CAR-T cells with secretable CD70 armored DLL3-CAR15-structure were significantly more effective than the DLL3-CAR09 and DLL3-CAR16 structures in killing SHP-77 target cells and NCI-H2171 target cells at different E:T ratios during 72-h killing. The membrane-bound CD70 armored DLL3-CAR16 structure was superior to the non-armor structure in killing high-expression target cells, but weaker than the non-armor structure in killing low-expression target cells, possibly affecting CAR-T cell function due to the CD27 expression being significantly lower than that of the non-armor and secretable CD70 armor groups. The above results prove that the secretable CD70 armor structure can improve the activation degree and the specific killing capacity of CAR-T without influencing the expression level of CD27, and has the advantage of improving the CAR-T function compared with a membrane-bound CD70 armor structure.

**5. *In vivo* tumor inhibition experiment of secretable CD70 armored and membrane-bound CD70 armored DLL3 CAR-T Cells**

The anti-tumor effect of DLL3 CAR-T cells was evaluated using the mouse subcutaneous tumor CDX model: NPG mice (combined immunodeficient mice, purchased from Beijing Vitalstar Biotechnology Co., Ltd.) were inoculated subcutaneously with $3 \times 10^6$ NCI-H2171 cells in logarithmic growth phase and in good growth condition.

The mice with the tumor volume around 30 $mm^3$ were randomly grouped according to the random number principle. On the 10th day after tumor inoculation, CAR-T cells ($1.5 \times 10^7$ CAR+ cells/mouse) were injected via the tail vein. The day of CAR-T cell injection was recorded as day 0 (D0), single administration. The specific grouping and administration are shown in Table 18.

Table 18. Grouping of NCI-2171 model *in vivo* anti-tumor experiment

| Group | Number of animals | Therapeutic | Dose | Route of administration | Time of administration |
|---|---|---|---|---|---|
| G1 | 5 | PBS | PBS/200μL | i.v. | Day0 |
| G2 | 5 | DLL3-CAR09 | 1.5e7 CAR+/200μL | i.v. | Day0 |
| G3 | 5 | DLL3-CAR15 | 1.5e7 CAR+/200μL | i.v. | Day0 |
| G4 | 5 | DLL3-CAR16 | 1.5e7 CAR+/200μL | i.v. | Day0 |

The results of the tumor volume measurement in mice are shown in FIG. 22A. CAR-T DLL3-CAR16 group did not exhibit significant efficacy; tumors of mice in CAR-T DLL3-CAR09 group showed a clear trend of slowed-down growth from day 11; tumors of animals in CAR-T DLL3-CAR15 group showed significant efficacy in inhibiting tumor growth from day 8. Weight change is shown in FIG. 22B. Animals in the PBS group were euthanized on day 18 due to tumor mean exceeding 2000 $mm^3$, and other groups of animals were in normal weight status. The results of peripheral blood T cell assay in mice are shown in FIG. 22C. The content of the T cells in the peripheral blood of the animals in DLL3-CAR09 group showed continuous expansion and reached the maximum on day 11; DLL3-CAR15 group showed significant T cell expansion on day 4, which reached the peak of expansion on day 8, coinciding with the time of tumor inhibition efficacy; compared with the other two groups, DLL3-CAR16 group showed significantly lower T cell expansion and slower expansion speed, and did not show significant efficacy. The expression detection results of mouse peripheral blood T cell surface CD70 and CD27 are shown in FIGs. 22D and 22E. The membrane surface CD70 expression of the CAR-T cells of DLL3-CAR16 group was significantly higher than that of the other two groups, and the CD27 expression showed a continuous reduction trend along with the CAR-T expansion. The above results show that the *in vivo* efficacy results and the *in vitro* killing results of the low-expression cell line mouse model are consistent, indicating that the secretable CD70 armor structure can improve the activation degree and the specific killing capacity of CAR-T without influencing the expression level of CD27, and has the advantage of improving the CAR-T function compared with a membrane-bound CD70 armor structure.

## Claims

1. A chimeric antigen receptor (CAR), comprising an extracellular binding region that specifically recognizes a delta-like ligand 3 (DLL3) protein, a hinge region, a transmembrane region, an intracellular co-stimulatory domain, and an intracellular signaling domain, wherein the extracellular binding region comprises an antibody or an antigen-binding fragment thereof that specifically recognizes delta-like ligand 3 (DLL3), and the antibody or the antigen-binding fragment thereof comprises HCDR1, HCDR2, and HCDR3 contained in the heavy chain variable region (VH) set forth in SEQ ID NO: 1, 3, 5, 83-84, 87, or 90, and LCDR1, LCDR2, and LCDR3 contained in the light chain variable region (VL) set forth in SEQ ID NO: 2, 4, 6, 81-82, 85-86, or 89.

2. The chimeric antigen receptor according to claim 1, wherein the antibody or the antigen-binding fragment thereof that specifically recognizes delta-like ligand 3 (DLL3) comprises the LCDR and HCDR below defined according to the Kabat numbering scheme:

(1) the LCDR1, the LCDR2, and the LCDR3 set forth in SEQ ID NOs: 10-12, and the HCDR1, the HCDR2, and the HCDR3 set forth in SEQ ID NOs: 7-9; or such 6 CDRs wherein one or more have 5, 4, 3, 2, or 1 amino acid substitution, deletion, or addition compared with the corresponding CDRs of the 6 CDRs described above;
(2) the LCDR1, the LCDR2, and the LCDR3 set forth in SEQ ID NOs: 10-12, and the HCDR1, the HCDR2, and the HCDR3 set forth in SEQ ID NOs: 7, 101, and 9; or such 6 CDRs wherein one or more have 5, 4, 3, 2, or 1 amino acid substitution, deletion, or addition compared with the corresponding CDRs of the 6 CDRs described above;
(3) the LCDR1, the LCDR2, and the LCDR3 set forth in SEQ ID NOs: 16-18, and the HCDR1, the HCDR2, and the HCDR3 set forth in SEQ ID NOs: 13-15; or such 6 CDRs wherein one or more have 5, 4, 3, 2, or 1 amino acid substitution, deletion, or addition compared with the corresponding CDRs of the 6 CDRs described above;
(4) the LCDR1, the LCDR2, and the LCDR3 set forth in SEQ ID NOs: 16, 17, and 88, and the HCDR1, the HCDR2, and the HCDR3 set forth in SEQ ID NOs: 13-15; or such 6 CDRs wherein one or more have 5, 4, 3, 2, or 1 amino acid substitution, deletion, or addition compared with the corresponding CDRs of the 6 CDRs described above;
(5) the LCDR1, the LCDR2, and the LCDR3 set forth in SEQ ID NOs: 22-24, and the HCDR1, the HCDR2, and the HCDR3 set forth in SEQ ID NOs: 19-21; or such 6 CDRs wherein one or more have 5, 4, 3, 2, or 1 amino acid substitution, deletion, or addition compared with the corresponding CDRs of the 6 CDRs described above;
(6) the LCDR1, the LCDR2, and the LCDR3 set forth in SEQ ID NOs: 22-24, and the HCDR1, the HCDR2, and the HCDR3 set forth in SEQ ID NOs: 19, 91, and 21; or such 6 CDRs wherein one or more have 5, 4, 3, 2, or 1 amino acid substitution, deletion, or addition compared with the corresponding CDRs of the 6 CDRs described above; or

the antibody or the antigen-binding fragment thereof that specifically recognizes delta-like ligand 3 (DLL3) comprises the LCDR and HCDR below defined according to the IMGT numbering scheme:

(7) the LCDR1, the LCDR2, and the LCDR3 set forth in SEQ ID NOs: 28-30, and the HCDR1, the HCDR2, and the HCDR3 set forth in SEQ ID NOs: 25-27; or such 6 CDRs wherein one or more have 5, 4, 3, 2, or 1 amino acid substitution, deletion, or addition compared with the corresponding CDRs of the 6 CDRs described above;
(8) the LCDR1, the LCDR2, and the LCDR3 set forth in SEQ ID NOs: 34-36, and the HCDR1, the HCDR2, and the HCDR3 set forth in SEQ ID NOs: 31-33; or such 6 CDRs wherein one or more have 5, 4, 3, 2, or 1 amino acid substitution, deletion, or addition compared with the corresponding CDRs of the 6 CDRs described above;
(9) the LCDR1, the LCDR2, and the LCDR3 set forth in SEQ ID NOs: 40-42, and the HCDR1, the HCDR2, and the HCDR3 set forth in SEQ ID NOs: 37-39; or such 6 CDRs wherein one or more have 5, 4, 3, 2, or 1 amino acid substitution, deletion, or addition compared with the corresponding CDRs of the 6 CDRs described above; or

the antibody or the antigen-binding fragment thereof that specifically recognizes delta-like ligand 3 (DLL3) comprises the LCDR and HCDR below defined according to the Chothia numbering scheme:

(10) the LCDR1, the LCDR2, and the LCDR3 set forth in SEQ ID NOs: 46-48, and the HCDR1, the HCDR2, and the HCDR3 set forth in SEQ ID NOs: 43-45; or such 6 CDRs wherein one or more have 5, 4, 3, 2, or 1 amino acid substitution, deletion, or addition compared with the corresponding CDRs of the 6 CDRs described above;
(11) the LCDR1, the LCDR2, and the LCDR3 set forth in SEQ ID NOs: 52-54, and the HCDR1, the HCDR2, and the HCDR3 set forth in SEQ ID NOs: 49-51; or such 6 CDRs wherein one or more have 5, 4, 3, 2, or 1 amino acid substitution, deletion, or addition compared with the corresponding CDRs of the 6 CDRs described above; or
(12) the LCDR1, the LCDR2, and the LCDR3 set forth in SEQ ID NOs: 58-60, and the HCDR1, the HCDR2, and the HCDR3 set forth in SEQ ID NOs: 55-57; or such 6 CDRs wherein one or more have 5, 4, 3, 2, or 1 amino acid substitution, deletion, or addition compared with the corresponding CDRs of the 6 CDRs described above.

**3.** The chimeric antigen receptor according to any one of claims 1-2, wherein the antibody or the antigen-binding fragment thereof comprises a heavy chain variable region (VH) set forth in SEQ ID NO: 1, 3, 5, 83-84, 87, or 90, or a VH having 99%, 98%, 97%, 96%, 95%, 90%, 85%, or 80% identity to any one of the VHs; and a light chain variable region (VL) set forth in SEQ ID NO: 2, 4, 6, 81-82, 85-86, or 89, or a VL having 99%, 98%, 97%, 96%, 95%, 90%, 85%, or 80% identity to any one of the VLs;

preferably, the antibody or the antigen-binding fragment thereof comprises the VH set forth in SEQ ID NO: 1, and the VL set forth in SEQ ID NO: 2; preferably, the antibody or the antigen-binding fragment thereof comprises the VH set forth in SEQ ID NO: 3, and the VL set forth in SEQ ID NO: 4; preferably, the antibody or the antigen-binding fragment thereof comprises the VH set forth in SEQ ID NO: 5, and the VL set forth in SEQ ID NO: 6; preferably, the antibody or the antigen-binding fragment thereof comprises the VH set forth in SEQ ID NO: 83 or 84, and the VL set forth in SEQ ID NO: 81; preferably, the antibody or the antigen-binding fragment thereof comprises the VH set forth in SEQ ID NO: 84, and the VL set forth in SEQ ID NO: 82; preferably, the antibody or the antigen-binding fragment thereof comprises the VH set forth in SEQ ID NO: 87, and the VL set forth in SEQ ID NO: 85 or 86; preferably, the antibody or the antigen-binding fragment thereof comprises the VH set forth in SEQ ID NO: 90, and the VL set forth in SEQ ID NO: 89;
preferably, the antibody or the antigen-binding fragment is in the form of an scFv having the structure of VH-linker-VL or VL-linker-VH;
preferably, the linker has the sequence set forth in SEQ ID NO: 70 or 71.

**4.** The chimeric antigen receptor according to any one of claims 1-3, wherein the chimeric antigen receptor further comprises a signal peptide, and the signal peptide is optionally a CD8 signal peptide; preferably, the CD8 signal peptide has the amino acid sequence set forth in SEQ ID NO: 61.

**5.** The chimeric antigen receptor according to any one of claims 1-4, wherein the hinge region is selected from a CD28 hinge region and a CD8α hinge region;
preferably, the hinge region comprises the amino acid sequence set forth in SEQ ID NO: 62 or 63.

**6.** The chimeric antigen receptor according to any one of claims 1-5, wherein the transmembrane region is selected from CD4, CD8α, CD28, PD1, and/or 4-1BB transmembrane regions;
preferably, the transmembrane region is CD28 transmembrane region or CD8α transmembrane region; more preferably, the transmembrane region has the amino acid sequence set forth in SEQ ID NO: 64 or 65.

**7.** The chimeric antigen receptor according to any one of claims 1-6, wherein the intracellular co-stimulatory signaling domain is selected from CD28 and 4-1BB intracellular co-stimulatory domains; preferably, the intracellular co-stimulatory signaling domain has the amino acid sequence set forth in SEQ ID NO: 66 or 67.

**8.** The chimeric antigen receptor according to any one of claims 1-7, wherein the intracellular signaling domain is a CD3ζ intracellular signaling domain;
preferably, the CD3ζ intracellular signaling domain has the sequence set forth in SEQ ID NO: 68.

**9.** The chimeric antigen receptor according to any one of claims 1-8, wherein the chimeric antigen receptor further comprises a CD70 domain;

preferably, the CD70 domain has the sequence set forth in SEQ ID NO: 102 or 104;
preferably, the CD70 domain is linked to the intracellular signaling domain by P2A, the P2A having the sequence set forth in SEQ ID NO: 106.

**10.** The chimeric antigen receptor according to any one of claims 1-9, wherein the chimeric antigen receptor comprises the amino acid sequence set forth in SEQ ID NO: 77, SEQ ID NO: 78, SEQ ID NO: 79, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 103, or SEQ ID NO: 105, or an amino acid sequence comprising at least 85%, at least 90%, at least 91%, at least 92%, at least 93%, at least 94%, at least 95%, at least 96%, at least 97%, at least 98%, at least 99%, or 100% sequence identity to any one of the amino acid sequences described above.

**11.** An isolated nucleic acid molecule, encoding the chimeric antigen receptor according to any one of claims 1-10.

**12.** The nucleic acid molecule according to claim 11, wherein the nucleic acid molecule is a DNA or an RNA, wherein the

RNA is preferably an mRNA.

**13.** An expression vector, comprising the isolated nucleic acid molecule according to claim 11 or 12.

**14.** A cell, expressing the chimeric antigen receptor according to any one of claims 1-10, or comprising the nucleic acid molecule according to any one of claims 11-12, or comprising the expression vector according to claim 13.

**15.** The cell according to claim 14, wherein the cell is an immune cell; preferably, the immune cell is selected from T lymphocytes, NK cells, hematopoietic stem cells, pluripotent stem cells, and immune cells differentiated and cultured from embryonic stem cells.

**16.** A method for preparing a chimeric antigen receptor-modified immune cell, wherein the method comprises the step of delivering the nucleic acid molecule according to any one of claims 11-12 or the expression vector according to claim 13 to an immune cell to be modified;
preferably, the means of delivery comprises viral transfection or delivery to the immune cells via cationic liposomes (e.g., LNP); preferably, the method further comprises isolating and activating the immune cells prior to delivery; preferably, the immune cell is selected from T lymphocytes, NK cells, hematopoietic stem cells, pluripotent stem cells, and immune cells differentiated and cultured from embryonic stem cells.

**17.** A pharmaceutical composition, comprising the chimeric antigen receptor according to any one of claims 1-10, the nucleic acid molecule according to any one of claims 11-12, the expression vector according to claim 13, the cell according to any one of claims 14-15, or a product prepared by the method according to claim 16, and a pharmaceutically acceptable carrier.

**18.** The pharmaceutical composition according to claim 17 for use in preventing and/or treating a tumor or cancer;

preferably, the tumor or cancer is selected from a solid tumor, a hematologic tumor, and a DLL3-expressing infiltrative cancer;
preferably, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

**19.** Use of the chimeric antigen receptor according to any one of claims 1-10, the nucleic acid molecule according to any one of claims 11-12, the expression vector according to claim 13, the cell according to any one of claims 14-15, or a product prepared by the method according to claim 16, or the pharmaceutical composition according to claim 17 in preparing a composition for preventing and/or treating a tumor or cancer;

preferably, the tumor or cancer is selected from a solid tumor, a hematologic tumor, and a DLL3-expressing infiltrative cancer;
preferably, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

**20.** A method for preventing and/or treating a tumor or cancer, comprising administering to a patient in need thereof an effective amount of the chimeric antigen receptor according to any one of claims 1-10, the nucleic acid molecule according to any one of claims 11-12, the expression vector according to claim 13, the cell according to any one of claims 14-15, or a product prepared by the method according to claim 16, or the pharmaceutical composition according to claim 17;

preferably, the tumor or cancer is selected from a solid tumor, a hematologic tumor, and a DLL3-expressing infiltrative cancer;
preferably, the tumor or cancer is selected from small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

DLL3-CAR01
5` LTR
CD8α SP
VH-(G4S)3-VL
CD8α H+TM
4-1BB
CD3ζ
3` LTR
DLL3-CAR02
5` LTR
CD8α SP
VHH
(G4S)3
VHH
CD8α H+TM
4-1BB
CD3ζ
3` LTR
DLL3-CAR02-1
5` LTR
CD8α SP
VHH
(G4S)3
VHH
CD8α H+TM
4-1BB
CD3ζ
T2A
TGFBRII ECD+TM
3` LTR
DLL3-CAR03
5` LTR
CD8α SP
VL-(G4S)3-VH
CD8α H+TM
4-1BB
CD3ζ
3` LTR
DLL3-CAR04
5` LTR
CD8α SP
VL-(G4S)3-VH
CD8α H+TM
4-1BB
CD3ζ
3` LTR
DLL3-CAR05
5` LTR
CD8α SP
VL-(G4S)3-VH
CD8α H+TM
4-1BB
CD3ζ
3` LTR

FIG. 1

DLL3 CAR-T proliferation
200
150
100
50
0
fold
Day2
Day5
Day7
Day9
Day post activation
Parental
DLL3-CAR01
DLL3-CAR02
DLL3-CAR03
DLL3-CAR04

FIG. 2

DLL3 CAR-T CAR expression
Transfection %
100
50
0
Parental
DLL3-CAR01
DLL3-CAR02
DLL3-CAR03
DLL3-CAR04
DLL3-CAR05
Parental
DLL3-CAR01
DLL3-CAR02
DLL3-CAR03
DLL3-CAR04
DLL3-CAR05

FIG. 3

DLL3 memory marker
100
80
60
40
20
0
Parental
DLL3-CAR01
DLL3-CAR02
DLL3-CAR03
DLL3-CAR04
DLL3-CAR05
TE
TEM
TCM
SCM

FIG. 4A

DLL3 surface marker
100
80
60
40
20
0
Parental
DLL3-CAR01
DLL3-CAR02
DLL3-CAR03
DLL3-CAR04
DLL3-CAR05
Tim3-PD1-
Tim3+PD1-
Tim3+ PD1+
Tim3- PD1+

FIG. 4B

DLL3 CAR-T 72h killing
(Taregt cell : SHP-77)
killing %
100
80
60
40
20
0
-20
1:2
1:10
1:20
E:T ratio
Parental
DLL3-CAR01
DLL3-CAR02
DLL3-CAR03
DLL3-CAR04

FIG. 5A

DLL3 CAR-T 72h killing
(Taregt cell :NCI-H2171)
100
80
60
40
20
0
-20
killing %
1: 2
1: 10
1: 20
E:T ratio
Parental
DLL3-CAR01
DLL3-CAR02
DLL3-CAR03
DLL3-CAR04
DLL3-CAR05

FIG. 5B

DLL3 CAR-T 72h killing
(Target cell: 293T-Luc)
20
0
-20
-40
-60
killing %
1: 2
1: 10
1: 20
E:T ratio
Parental
DLL3-CAR01
DLL3-CAR02
DLL3-CAR03
DLL3-CAR04

FIG. 5C

DLL3-CAR06
DLL3-CAR07
DLL3-CAR08
DLL3-CAR09
DLL3-CAR10
DLL3-CAR11
DLL3-CAR12
DLL3-CAR13
DLL3-CAR14
5` LTR
CD8α SP
VH-(G4S)3-VL
VL-(G4S)3-VH
CD8α H+TM
CD28 H+TM
4-1BB
CD28
CD3ζ
3` LTR

FIG. 6

DLL3 CAR-T 72h killing
(Target cell: NCI-H2171)
killing %
E:T ratio
1:2
1:10
1:20
Parental
DLL3-CAR02-1
DLL3-CAR03
DLL3-CAR04
DLL3-CAR06
DLL3-CAR07
DLL3-CAR08
DLL3-CAR09
DLL3-CAR10

FIG. 7

DLL3 CAR-T proliferation
Fold
900
800
700
600
500
400
300
200
100
0
Day3
Day7
Day9
Day11
Day14
day post activation
Parental
DLL3-CAR02-1
DLL3-CAR09
DLL3-CAR10
DLL3-CAR11
DLL3-CAR12

FIG. 8A

DLL3 CAR-T surface marker
% positive
100
80
60
40
20
0
Parental
DLL3-CAR02-1
DLL3-CAR09
DLL3-CAR10
DLL3-CAR11
DLL3-CAR12
CD8+
CD4+

FIG. 8B

DLL3 CAR-T surface marker
% positive
100
80
60
40
20
0
Parental
DLL3-CAR02-1
DLL3-CAR09
DLL3-CAR10
DLL3-CAR11
DLL3-CAR12
Tim3-LAG3-
Tim3+LAG3-
Tim3+LAG3+
Tim3-LAG3+

FIG. 8C

DLL3 CAR-T surface marker
% positive
100
80
60
40
20
0
Parental
DLL3-CAR02-1
DLL3-CAR09
DLL3-CAR10
DLL3-CAR11
DLL3-CAR12
TE
TEM
TCM
SCM

FIG. 8D

DLL3 CAR-T 72h killing
(Target cell: SHP-77)
killing %
0
20
40
60
80
100
1:2
1:10
1:20
E:T ratio
Parental
DLL3-CAR02-1
DLL3-CAR09
DLL3-CAR10
DLL3-CAR11
DLL3-CAR12

FIG. 9A

DLL3 CAR-T 72h killing
(Target cell: NCI-H2171)
killing %
0
20
40
60
80
100
1:2
1:10
1:20
E:T ratio
Parental
DLL3-CAR02-1
DLL3-CAR09
DLL3-CAR10
DLL3-CAR11
DLL3-CAR12

FIG. 9B

DLL3 CAR-T 72h killing
(Target cell: SHP-77)
killing %
100
80
60
40
20
0
-20
1:2
1:10
1:20
E:T ratio
Parental
DLL3-CAR01
DLL3-CAR02-1
DLL3-CAR09
DLL3-CAR11

FIG. 10A

DLL3 CAR-T 72h killing
(Target cell: NCI-H82)
killing %
100
80
60
40
20
0
1:2
1:10
1:20
E:T ratio
Parental
DLL3-CAR01
DLL3-CAR02-1
DLL3-CAR09
DLL3-CAR11

FIG. 10B

DLL3 CAR-T 72h killing
(Target cell: NCI-H2171)
killing %
100
80
60
40
20
0
-20
1:2
1:10
1:20
E:T ratio
Parental
DLL3-CAR01
DLL3-CAR02-1
DLL3-CAR09
DLL3-CAR11

FIG. 10C

DLL3 CAR-T IFN-γ
pg/mL
1000
100
10
1
SHP-77
H82
H2171
E:T ratio= 1:2
Target cell only
Parental
DLL3-CAR01
DLL3-CAR02-1
DLL3-CAR09
DLL3-CAR11

FIG. 11

DLL3 CAR-T proliferation
400
300
200
100
0
Fold
Day3
Day7
Day11
Day post activation
Parental
DLL3-CAR02-1
DLL3-CAR09
DLL3-CAR11
DLL3-CAR13
DLL3-CAR14

FIG. 12

DLL3 CAR-T surface marker
100
80
60
40
20
0
% positive
Parental
DLL3-CAR02-1
DLL3-CAR09
DLL3-CAR11
DLL3-CAR13
DLL3-CAR14
CD8+
CD4+

FIG. 13A

DLL3 CAR-T surface marker
% positive
100
80
60
40
20
0
Parental
DLL3-CAR02-1
DLL3-CAR09
DLL3-CAR11
DLL3-CAR13
DLL3-CAR14
Tim3-LAG3-
Tim3+LAG3-
Tim3+LAG3+
Tim3-LAG3+

FIG. 13B

DLL3 CAR-T surface marker
% positive
100
80
60
40
20
0
Parental
DLL3-CAR02-1
DLL3-CAR09
DLL3-CAR11
DLL3-CAR13
DLL3-CAR14
TE
TEM
TCM
SCM

FIG. 13C

DLL3 CAR-T 72h killing
(Target cell: NCI-H2171)
killing %
100
80
60
40
20
0
-20
1:2
1:10
1:20
E:T ratio
Parental
DLL3-CAR02-1
DLL3-CAR09
DLL3-CAR11
DLL3-CAR13
DLL3-CAR14

FIG. 14

Tumor Volume Progression
Tumor V[mm3],Mean with SEM
4000
3000
2000
1000
300
200
100
0
SHP-77
injection
-4 0 3 6 9 14 17 20 23 27 37 40
Days after the treatment
***
***
***
Group1 Control-PBS , n=5
Group2 DLL3-CAR02-1 8e6 , n=5
Group3 DLL3-CAR09 8e6, n=5
Group4 DLL3-CAR11 8e6, n=5

FIG. 15A

BW Change%
% Change,Mean with SEM
40
20
0
0 3 6 9 14 17 20 23 27 37 40
Days after the treatment
PBS , n=5
DLL3-CAR02-1 8e6 , n=5
DLL3-CAR09 8e6, n=5
DLL3-CAR11 8e6, n=5

FIG. 15B

Survival curve
Probability of Survival
100
50
0
0 10 15 20 25 30 35 40 45 50
Days after treatment
PBS , n=5
DLL3-CAR02-1 8e6 , n=5
DLL3-CAR09 8e6, n=5
DLL3-CAR11 8e6, n=5

FIG. 15C

Proliferation of T cells in PB
%,T cell in lymphocytes
PBS
DLL3-CAR02-1
DLL3-CAR09
DLL3-CAR11
D6 D9 D14 D21 D40
Days after the treatment

FIG. 15D

Tumor Volume Progression

Tumor V[mm3],Mean with SEM
3000
2000
1000
0
NCI-H2171
injection
-3
0
4
7
11
14
18
21
27
33
Days after treatment
PBS , n=5
DLL3-CAR02-1 1.5e7 , n=5
DLL3-CAR09 1.5e7, n=5
DLL3-CAR11 1.5e7, n=5
DLL3-CAR13 1.5e7, n=5
DLL3-CAR14 1.5e7, n=5

FIG. 15E

BW Change%

% Change,Mean with SEM
50
30
10
-10
0
4
7
11
14
18
21
27
33
Days after treatment
PBS
DLL3-CAR02-1
DLL3-CAR09
DLL3-CAR11
DLL3-CAR13
DLL3-CAR14

FIG. 15F

Survival curve
Probability of Survival
100
50
0
0
10
20
40
Days after treatment
PBS
DLL3-CAR02-1
DLL3-CAR09
DLL3-CAR11
DLL3-CAR13
DLL3-CAR14

FIG. 15G

Proliferation of T cells in PB
%,T cell in lymphocytes
40
30
20
10
0
0.09 0.04 0.01
6.99
3.78
0.24
26.42
16.63
0.72
12.07
3.24
0.36
8.85 8.08
0.16
12.81
9.90
0.57
D5 D11 D19
Days after treatment
PBS
DLL3-CAR02-1
DLL3-CAR09
DLL3-CAR11
DLL3-CAR13
DLL3-CAR14

FIG. 15H

DLL3-CAR15
5` LTR
CD8α SP
VL-(G4S)3-VH
CD8 Hinge
CD8 TM
4-1BB
CD3ζ
P2A
sCD70
3` LTR

FIG. 16

DLL3 CAR expression
Transfection %
100
80
60
40
20
0
parental T
DLL3-CAR09
DLL3-CAR15
parental T
DLL3-CAR09
DLL3-CAR15

FIG. 17A

DLL3 CAR-T CD4/CD8
100
80
60
40
20
0
Parental T
DLL3-CAR09
DLL3-CAR15
CD8+
CD4+

FIG. 17B

DLL3 CAR-T exhaustion marker
100
80
60
40
20
0
Parental T
DLL3-CAR09
DLL3-CAR15
Tim3-LAG3-
Tim3+LAG3-
Tim3+LAG3+
Tim3-LAG3+

FIG. 17C

DLL3 CAR-T memory markers
100
80
60
40
20
0
Parental T
DLL3-CAR09
DLL3-CAR15
TE
TEM
TCM
SCM

FIG. 17D

CD70 secretion in supernatant
ng/mL
4
3
2
1
0
Parental T
DLL3-CAR09
DLL3-CAR15
Parental T
DLL3-CAR09
DLL3-CAR15

FIG. 17E

DLL3 CAR-T 72h killing
(Taregt cell: SHP-77)
killing %
100
50
0
1:2
1:10
1:20
E:T ratio
Parental T
DLL3-CAR09
DLL3-CAR15

FIG. 18A

DLL3 CAR-T 72h killing
(Taregt cell: NCI-H2171)
killing %
100
80
60
40
20
0
1:2
1:10
1:20
E:T ratio
Parental T
DLL3-CAR09
DLL3-CAR15

FIG. 18B

DLL3 CAR-T serial killing
(Target cell: NCI-H2171-Luc)
killing %
100
80
60
40
20
0
Round1
Round2
Round3
Round of stimulation
E:T ratio= 1:2
Parental T
DLL3-CAR09
DLL3-CAR15

FIG. 18C

SHP-77 Tumor volume
Volume (mm3), Mean with SEM
2500
2000
1500
1000
500
0
0 5 8 12 15 19 22 26
Days after the treatment
PBS
DLL3-CAR09
DLL3-CAR15

FIG. 19A

BW Change%
Body Weight Change(%)
5
0
-5
-10
-15
-20
-25
0 5 8 12 15 19 22 26
Days after the treatment
PBS
DLL3-CAR09
DLL3-CAR15

FIG. 19B

Proliferation of T cells（blood）
% of CD45+CD3+ in lymphocytes
6
4
2
0
5
12
15
22
26
5
12
15
22
26
5
12
15
22
26
Days after the treatment
PBS
DLL3-CAR09
DLL3-CAR15

FIG. 19C

NCI-H2171
Volume (mm3), Mean with SEM
3000
2000
1000
0
0
6
10
14
17
Days after CART infusion
*
PBS
DLL3-CAR09
DLL3-CAR15

FIG. 19D

BW Change%
% Change,Mean with SEM
20
15
10
5
0
-5
0
6
10
14
Days after CART infusion
PBS
DLL3-CAR09
DLL3-CAR15

FIG. 19E

Tumor Growth Inhibition
Inhibition %
50
40
30
20
10
0
-10
6
10
14
17
Days after CART infusion
DLL3-CAR09
DLL3-CAR15

FIG. 19F

DLL3-CAR16 | 5` LTR | CD8α SP | VL-(G4S)3-VH | CD8 Hinge | CD8 TM | 4-1BB | CD3ζ | P2A | mCD70 | 3` LTR

FIG. 20

80
60
40
20
0
Transfection %
Parental
DLL3-CAR09
DLL3-CAR15
DLL3-CAR16
Parental
DLL3-CAR09
DLL3-CAR15
DLL3-CAR16

FIG. 21A

100
80
60
40
20
0
CD8+
CD4+
Parental
DLL3-CAR09
DLL3-CAR15
DLL3-CAR16

FIG. 21B

100
80
60
40
20
0
Tim3-LAG3-
Tim3+LAG3-
Tim3+LAG3+
Tim3-LAG3+
Parental
DLL3-CAR09
DLL3-CAR15
DLL3-CAR16

FIG. 21C

100
80
60
40
20
0
TE
TEM
TCM
SCM
Parental
DLL3-CAR09
DLL3-CAR15
DLL3-CAR16

FIG. 21D

25
20
15
10
5
0
ng/mL
Parental
DLL3-CAR09
DLL3-CAR15
DLL3-CAR16


FIG. 21E

100
80
60
40
20
0
%
CD27
CD70
Parental
DLL3-CAR09
DLL3-CAR15
DLL3-CAR16


FIG. 21F

DLL3 CAR-T 72h killing
(Target cell :SHP-77)
killing %
100
80
60
40
20
0
1:2
1:10
1:20
1:40
E:T ratio
Parental
DLL3-CAR09
DLL3-CAR15
DLL3-CAR16


FIG. 21G

DLL3 CAR-T 72h killing
(Target cell :NCI-H2171)
killing %
100
80
60
40
20
0
1:2
1:10
1:20
1:40
E:T ratio
Parental
DLL3-CAR09
DLL3-CAR15
DLL3-CAR16


FIG. 21H

NCI-H2171 Tumor volume
Tumor V[mm3],Mean with SEM
2500
2000
1500
1000
500
0
0
4
8
11
15
18
22
Days after the treatment
PBS
DLL3-CAR09
DLL3-CAR15
DLL3-CAR16

FIG. 22A

BW Change
Body Weight Change(%)
20
15
10
5
0
-5
-10
0
4
8
11
15
Days after the treatment
PBS
DLL3-CAR09
DLL3-CAR15
DLL3-CAR16

FIG. 22B

T cell in PB
% of CD45⁺CD3⁺ in lymphocytes
100
80
60
40
20
0
4
8
11
Days after the treatment
PBS
DLL3-CAR09
DLL3-CAR15
DLL3-CAR16

FIG. 22C

CD70⁺ in CD3⁺
%
80
60
40
20
0
4
8
11
Days after the treatment
PBS
DLL3-CAR09
DLL3-CAR15
DLL3-CAR16

FIG. 22D

CD27+ in CD3+
100
50
0
%
4
8
11
Days after the treatment
PBS
DLL3-CAR09
DLL3-CAR15
DLL3-CAR16

FIG. 22E

**INTERNATIONAL SEARCH REPORT**

International application No.
**PCT/CN2024/124869**

**A. CLASSIFICATION OF SUBJECT MATTER**

C07K16/28(2006.01)i; C07K19/00(2006.01)i; C12N15/13(2006.01)i; C12N15/62(2006.01)i; C12N5/10(2006.01)i; C12N15/85(2006.01)i; A61K39/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

Databases: CNTXT, DWPI, ENTXT, ENTXTC, WOTXT, WOTXTC, VEN, WPABS, WPABSC, CJFD, CNKI, 万方, WANFANG, 百度学术, Baidu Scholar, PubMed, ISI_Web of Science, STN, GenBank, EMBL-EBI, 中国专利生物序列检索系统, China Patent Biological Sequence Search System; search terms: 嵌合抗原受体, 抗原结合片段, 抗体, δ样配体3, delta样配体3, 胞外结合区, 铰链区, 跨膜区, 共刺激, 信号传导结构域, 信号肽, 连接子, 肿瘤, 癌症, CAR, CAR-T, chimeric antigen receptor, delta-like ligand3, DLL3, antibody, signal peptide, CD8, CD28, CD8α, linker, CD4, PD1, 4-1BB, CD70, CD3, P2A, tumor, cancer, 对SEQ ID NOs: 1-60, 81-91, 101进行序列检索, search for SEQ ID NOs: 1-60, 81-91 and 101.

**C. DOCUMENTS CONSIDERED TO BE RELEVANT**

| Category* | Citation of document, with indication, where appropriate, of the relevant passages | Relevant to claim No. |
|---|---|---|
| PX | WO 2024099265 A1 (SHANGHAI SIMNOVA BIOTECHNOLOGY CO., LTD.) 16 May 2024 (2024-05-16)<br>embodiment 5, and tables 5-7 | 1-19 |
| A | CN 116874599 A (SICHUAN UNIVERSITY) 13 October 2023 (2023-10-13)<br>see abstract, claims 1-10, and description, paragraphs 23-25 | 1-19 |
| A | CN 116003628 A (NANJING BOAN BIOTECHNOLOGY CO., LTD. et al.) 25 April 2023 (2023-04-25)<br>see abstract, and claims 1-13 | 1-19 |
| A | CN 113543799 A (ALLOGENE THERAPEUTICS, INC. et al.) 22 October 2021 (2021-10-22)<br>see abstract, and claims 1-45 | 1-19 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/124869**

**C. DOCUMENTS CONSIDERED TO BE RELEVANT**

| Category* | Citation of document, with indication, where appropriate, of the relevant passages | Relevant to claim No. |
|---|---|---|
| A | CN 112334193 A (AMGEN INC. et al.) 05 February 2021 (2021-02-05)<br>see abstract, and claims 1-80 | 1-19 |
| A | CN 116731182 A (SHANDONG BOAN BIOTECHNOLOGY CO., LTD.) 12 September 2023 (2023-09-12)<br>see abstract, and claims 1-10 | 1-19 |

Form PCT/ISA/210 (second sheet) (July 2022)

**INTERNATIONAL SEARCH REPORT**

International application No.
**PCT/CN2024/124869**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/124869**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20**
because they relate to subject matter not required to be searched by this Authority, namely:
Said claim relates to a method for treating a disease in a human or animal.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/124869**

| Patent document cited in search report | Publication date (day/month/year) | Patent family member(s) | Publication date (day/month/year) |
|---|---|---|---|
| WO 2024099265 A1 | 16 May 2024 | None | |
| CN 116874599 A | 13 October 2023 | None | |
| CN 116003628 A | 25 April 2023 | WO 2024037288 A1 | 22 February 2024 |
| CN 113543799 A | 22 October 2021 | EP 3930744 A1 | 05 January 2022 |
| | | TW 202045547 A | 16 December 2020 |
| | | SG 11202108011 UA | 30 August 2021 |
| | | CO 2021011326 A2 | 09 September 2021 |
| | | KR 20210138574 A | 19 November 2021 |
| | | WO 2020180591 A1 | 10 September 2020 |
| | | PE 20220495 A1 | 07 April 2022 |
| | | JP 2022523781 A | 26 April 2022 |
| | | US 2021107979 A1 | 15 April 2021 |
| | | US 11673953 B2 | 13 June 2023 |
| | | BR 112021016984 A2 | 30 November 2021 |
| | | US 2023287120 A1 | 14 September 2023 |
| | | CA 3131908 A1 | 10 September 2020 |
| | | AU 2020231308 A1 | 19 August 2021 |
| | | IL 285943 A | 31 October 2021 |
| | | MX 2021010441 A | 21 September 2021 |
| CN 112334193 A | 05 February 2021 | CA 3095920 A1 | 17 October 2019 |
| | | CL 2023000896 A1 | 03 November 2023 |
| | | CO 2020013392 A2 | 10 November 2020 |
| | | UY 38182 A | 31 October 2019 |
| | | EA 202092417 A1 | 26 January 2021 |
| | | US 2021163621 A1 | 03 June 2021 |
| | | US 12012462 B2 | 18 June 2024 |
| | | BR 112020020857 A2 | 19 January 2021 |
| | | TW 202011999 A | 01 April 2020 |
| | | TWI 842703 B | 21 May 2024 |
| | | SA 520420301 B1 | 05 August 2024 |
| | | WO 2019200007 A1 | 17 October 2019 |
| | | SG 11202009923 TA | 27 November 2020 |
| | | IL 277735 A | 30 November 2020 |
| | | MA 52203 A | 17 February 2021 |
| | | CL 2023000895 A1 | 03 November 2023 |
| | | CR 20200539 A | 01 July 2021 |
| | | AU 2019251473 A1 | 22 October 2020 |
| | | JOP 20200259 A1 | 11 October 2020 |
| | | KR 20210018797 A | 18 February 2021 |
| | | MX 2020010753 A | 29 January 2021 |
| | | EP 3773912 A1 | 17 February 2021 |
| | | JP 2021520788 A | 26 August 2021 |
| | | JP 7479290 B2 | 08 May 2024 |
| | | AR 114283 A1 | 12 August 2020 |
| | | PH 12020551671 A1 | 07 June 2021 |
| | | CL 2020002613 A1 | 28 December 2020 |
| | | PE 20211396 A1 | 27 July 2021 |
| CN 116731182 A | 12 September 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311344635
- EP 2101823 B1
- US 20210107979 A1
- WO 2021008610 A1


### Non-patent literature cited in the description


- **KORNDORFER, I. P.** ; **BESTE, G.** ; **SKERRA, A.** *Proteins*, 2003, vol. 53, 121-129
- **ROQUE, A. C. A.** ; **LOWE, C. R.** ; **TAIPA, M. A**. Antibodies and genetically engineered related molecules: production and purification. *Biotechnol. Prog.*, 2004, vol. 20, 639-654
- **LEFRANC, M. P** ; **POMMIÉ, C** ; **RUIZ, M** ; **GIUDICELLI, V** ; **FOULQUIER, E** ; **TRUONG, L** ; **LEFRANC, G.** IMGT unique numbering for immunoglobulin and T cell receptor variable domains and Ig superfamily V-like domains.. *Developmental & Comparative Immunology*, 2003, vol. 27 (1), 55-77
- **KABAT et al.** Sequences of Proteins of Immunological Interest. Public Health Service, 1991
- **CHOTHIA, C.** ; **LESK, A. M.** Canonical structures for the hypervariable regions of immunoglobulins.. *Journal of molecular biology*, 1987, vol. 196 (4), 901-917
- **STADLER, C. R** ; **BÄHR-MAHMUD, H.** ; **CELIK, L** ; **HEBICH, B** ; **ROTH, A. S** ; **ROTH, R. P.** ; **SAHIN, U**. Elimination of large tumors in mice by mRNA-encoded bispecific antibodies. *Nature medicine*, 2017, vol. 23 (7), 815-817